# EUROPEAN PATENT APPLICATION

(11) **EP 2 772 545 A1**
(43) Date of publication of application: **03.09.2014**
(21) Application number: 13157503.7
(22) Date of filing: 01.03.2013
(51) Int. Cl.: C12P 19/00, C12P 19/20, C12N 9/34

(54) **Starch active proteins**

(71) Applicant: Nederlandse Organisatie voor Toegepast -Natuurwetenschappelijk Onderzoek TNO, 2628 VK Delft (NL)
(72) Inventor: Punt, Peter Jan, 2628 VK Delft (NL); Overkamp, Karin Martine, 2628 VK Delft (NL)
(74) Representative: Jansen, Cornelis Marinus

(57) **Abstract**

1. The invention compirises the use of a starch active protein comprising a domain comprising the nucleotide sequence [AS] GXD [ST] CPECXI [LR] EPVX [AS] WP [DNST] [LV] [TED]XAXVGR [ST] GPCGYNAR [DV] [SGN] [ITV] DYNXPXXXWGXXX[VI] [IVA][TS]Y[TKS]XG [DEQ] X [AIV] [DET]V[EQ]WC [VL] D [HAN] NGDHGGM, more preferably HGY [LM] X₆₋₁₀ T [RGK] [VIL] [GN] X [EQN] [AS] GXD [ST] CPECXI [LR] EPVX [AS] WP [DNST] [LV] [TED]XAXVGR [ST] GPCGYNAR [DV] [SGN] [ITV] DYNXPXXXWGXXX[VI] [IVA] [TS]Y[TKS]XG [DEQ] X [AIV] [DET] V [EQ] WC [VL] D [HAN] NGDHGGM[FY] [SAT] [YW] [GR] ICQ [DN] QX [IL] V [DNS] [KL] [FL] [LIT] [DTQ] [AP] X [HY] XP [ST] XXEKXAAEXCFXXGXLXC [TK] [GDE] VXG [AQ] XCX [FY] [NS] [PSG] DCXXXXPAC [WYH] R [NK] DWF [STA] CXX [WF] XXXXXXXCXXVD [NG] [AS] XX [NG] SCXT [TS] I [AS] GG [YF] [TPK] V [STP] X [KR] [VI] K [LI] P or a sequence that has an identity of more than 70%, thereto for modifying starch. Also comprised are proteins that contain said domain and a CBM20 domain for the modification of starch.

## Description

### FIELD OF THE INVENTION

The invention invention is related to the field of enzymatic carbohydrate formation and conversion, more specifically to the control of starch formation and conversion.

### BACKGROUND ART

A large number of processes have been described for converting starch to starch hydrolysates, such as maltose, glucose or specialty syrups, either for use as sweeteners or as precursors for other saccharides such as fructose. Glucose may also be fermented to ethanol or other fermentation products. Starch is a high molecular-weight polymer consisting of chains of glucose units. It usually consists of about 80% amylopectin and 20% amylose. Amylopectin is a branched polysaccharide in which linear chains of alpha-1,4 D-glucose residues are joined by alpha-1,6 glucosidic linkages. Amylose is a linear polysaccharide built up of D-glucopyranose units linked together by alpha-1,4 glucosidic linkages. In the case of converting starch into a soluble starch hydrolysate, the starch is depolymerized. The conventional depolymerization process consists of a gelatinization step and two consecutive process steps, namely a liquefaction process and a saccharification process.

Granular starch consists of microscopic granules, which are insoluble in water at room temperature. When an aqueous starch slurry is heated, the granules swell and eventually burst, dispersing the starch molecules into the solution, During this "gelatinzation" process there is a dramatic increase in viscosity. As the solids level is 30-40% in a typical industrial process, the starch has to be thinned or "liquefied" so that it can be handled. This reduction in viscosity is today mostly obtained by enzymatic degradation. During the liquefaction step, the long-chained starch is degraded into smaller branched and linear units (maltodextrins) by an alpha-amylase. The liquefaction process is typically carried out at about 105-110°C for about 5 to 10 minutes followed by about 1-2 hours at about 95°C. The temperature is then lowered to 60°C, a glucoamylase or a beta-amylase and optionally a debranching enzyme, such as an isoamylase or a pullulanase, are added and the saccharification process proceeds for about 24 to 72 hours.

Numerous starch converting enzymes have been described, especially enzymes from bacteria and filamentous fungi. There is, however still need for further novel enzymes.

Most of these enzymes are characterized by having a starch binding domain, denominated as CBM20 (se also http://www.cazy.org/fam/acc_CBM.html). However, very limited catalytic activities have been shown to be linked to CBM20 domains. In fungi this is limited to amylases GH13 and glucoamylases GH15.

### SUMMARY OF THE INVENTION

The inventors have now discovered a novel group of starch active enzymes, sharing a hitherto unknown domain (sometimes in addition to a CBM20 domain). Therefore the invention comprises]a starch active protein comprising a domain comprising the amino acid sequence
[AS] GXD [ST] CPECXI [LR] EPVX [AS] WP [DNST] [LV] [TED]XAXVGR [ST] GPCGYNAR [DV] [SGN] [ITV] DYNXPXXXWGXXX[VI] [IVA] [TS]Y[TKS]XG [DEQ] X [AIV] [DET]V[EQ]WC [VL] D [HAN] NGDHGGM,
or a sequence that has an identity of more than 70%, preferably more than 75%, more preferably more than 80%, more preferably more than 85%, more preferably more than 90%, more preferably more than 95%, more preferably more than 98% with said amino acid sequence,
more preferably comprising the sequence
HGY [LM] X₆₋₁₀ T [RGK] [VIL] [GN] X [EQN] [AS] GXD [ST] CPECXI [LR] EPVX [AS] WP [DNST] [LV] [TED]XAXVGR [ST] GPCGYNAR [DV] [SGN] [ITV] DYNXPXXXWGXXX[VI] [IVA] [TS]Y[TKS]XG [DEQ] X [AIV] [DET]V[EQ]WC [VL] D [HAN] NGDHGGM[FY] [SAT] [YW] [GR] ICQ [DN] QX [IL] V [DNS] [KL] [FL] [LIT] [DTQ] [AP] X [HY] XP [ST] XXEKXAAEXCFXXGXLXC [TK] [GDE] VXG [AQ] XCX [FY] [NS] [PSG] DCXXXXPAC [WYH] R [NK] DWF [STA] CXX [WF] XXXXXXXCXXVD [NG] [AS] XX [NG] SCXT [TS] I [AS] GG [YF] [TPK]V [STP] X [KR] [VI]K [LI] P
or a sequence that has an identity of more than 70%, preferably more than 75%, more preferably more than 80%, more preferably more than 85%, more preferably more than 90%, more preferably more than 95%, more preferably more than 98% with said amino acid sequence. Even more preferably, the starch active protein comprises the sequence: 
hg yltvpasrtr lgfeagidtc pecsilepvs awpdltaaqvgrsgpcgyna rvsvdynqpg
dywgnepvvs ytagdvvevq wcvdhngdhg gmftygicqn qtlvdlfltp gylptneekq
aaedcflege lsclhvpgqt cnynpdcsag epcyqndwft cnafqadnnr acqgvdgaal
nscmttiagg ytvtkkikip dyssshtllr frwnsfqtaq vylhcadiai
or MKVFAPLLSL SLATSVAGHG YMYIPSSRTR LGHEAGIDSC PECAILEPVS
SWPDLDAAPV GRSGPCGYNA RDSIDYNQPT TNWGSDAVQS YSPGEEIEVQ
WCVDHNGDHG GMFTYRICQD QSIVDKFLDP SYLPTNDEKQ AAEDCFDAGL
LPCTDVSGQE CGYSADCTEG EACWRNDWFT CNGFEASDRP KCQGVDNAEL
NSCYTSIAGG YTVTKKVKLP EYTSNHTLIS FKWNSFQTGQ IYLSCADIAI Q
 or a sequence that has an identity of more than 70%, preferably more than 75%, more preferably more than 80%, more preferably more than 85%, more preferably more than 90%, more preferably more than 95%, more preferably more than 98% with said amino acid sequences. Even more preferably said starch active protein is selected from the group consisting of the proteins with the NCBI accession no.
XP_663707.1, XP_003667407.1, XP_001224179.1, XP_002563839.1, XP_001823115.2, XP_002378449.1, BAE61982.1, EIT80577.1, XP_001273485.1 ,EGU89432.1 ,XP_001261597.1 ,XP_001216118.1 ,XP_384880.1 and ELR05959.1.
and the proteins encoded by the genes comprising the ESTs with the NCBI accession no. JK051852, JK048632, JK058718 , FE630975.1, FE630976.1, AM283774.1, and CX651361.1. In another embodiment, the invention comprises a starch active protein as defined above, that additionally comprises a CBM20 domain. Preferably, said starch active protein is selected from the group consisting of the proteins with NCBI accession no.
XP_663067.1 ,XP_001209972.1 ,XP_001264149.1 ,XP_002559611.1 ,XP_001270422.1 ,CAE75704.1 ,XP_003345013.1 ,EG056169.1 ,CCF35072.1 ,XP_963607.1 ,ELA34007.1 ,XP_003004371.1 ,EGX44447.1 ,EMD93787.1 ,XP_003302617.1 ,EMD62518.1 ,EGY15971.1 ,XP_003046877.1 ,XP_001935374.1 ,EFQ31521.1 ,XP_003842509.1 ,XP_001903564.1 ,XP_001559169.1 ,XP_001589670.1 ,EAQ71571.1 ,XP_001228636.1 and XP_001803614.1.

A preferred starch active protein according to the invention is from fungal origin, specifically, wherein said fungus is chosen from the group consisting of Aspergillus, more particularly, A. niger, A. nidulans, A. terreus, A. clavatus, A. oryzae or A. flavus" Neurospora, more particularly N. crassa or N. tetrasperma, Sclerotina, Gibberella, Coniothyrium, Psiticum, Magnaporthe, Podospora, Chaetomium, Phaeosphaeria, Botryotinia, Neosartorya, Pyrenophora, Panicum, Aureococcus, Penicillium, Trichoderma, Sordaria, Colleotrichum, Verticillium, Arthrobotrys, Nectria, Leptosphaeria, Fusarium, Glomerella, Geomyces, and Myceliophthora.

In another embodiment, the invention comprises a nucleic acid encoding a starch active protein according to the invention.

In yet another embodiment, the invention comprises a vector comprising a nucleic acid according to the invention. Furthermore, the invention pertains to a host cell capable of expressing a starch active protein comprising a nucleic acid or a vector as defined above.

Further, the invention comprises the use of a starch active protein according to the invention in the modification of raw starch, specifically wherein said modification comprises hydrolysis and/or oxidation, wherein the starch active protein increases the accessibility of the starch for starch degrading enzymes. Also part of the invention is a method for enhanced degradation of starch by treating said starch with amylase and a starch active protein according to the invention.

### LEGEND TO THE FIGURES

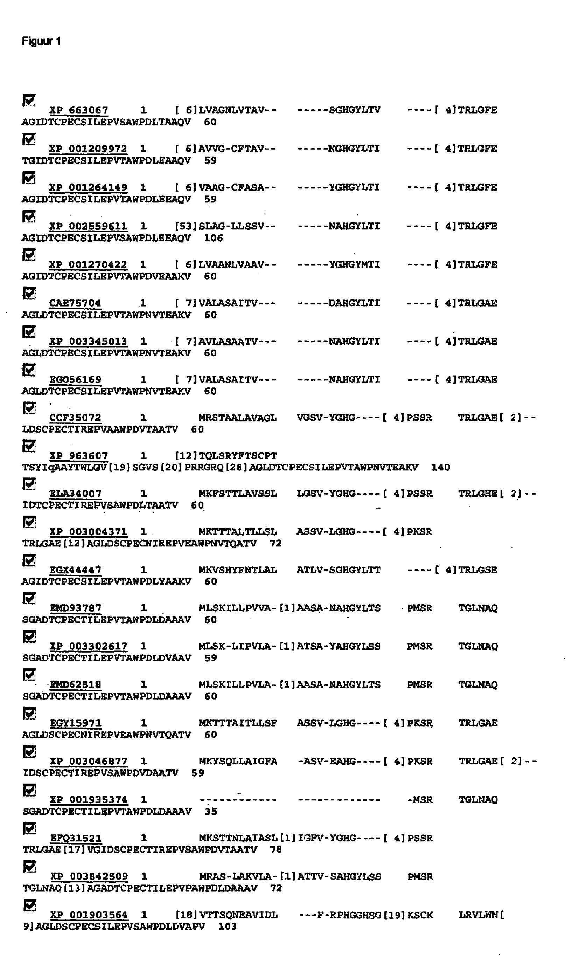

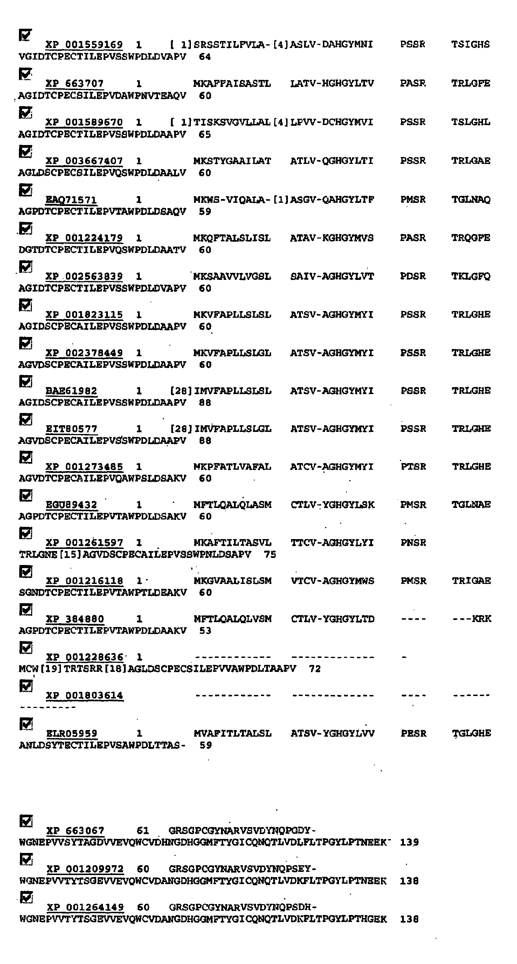

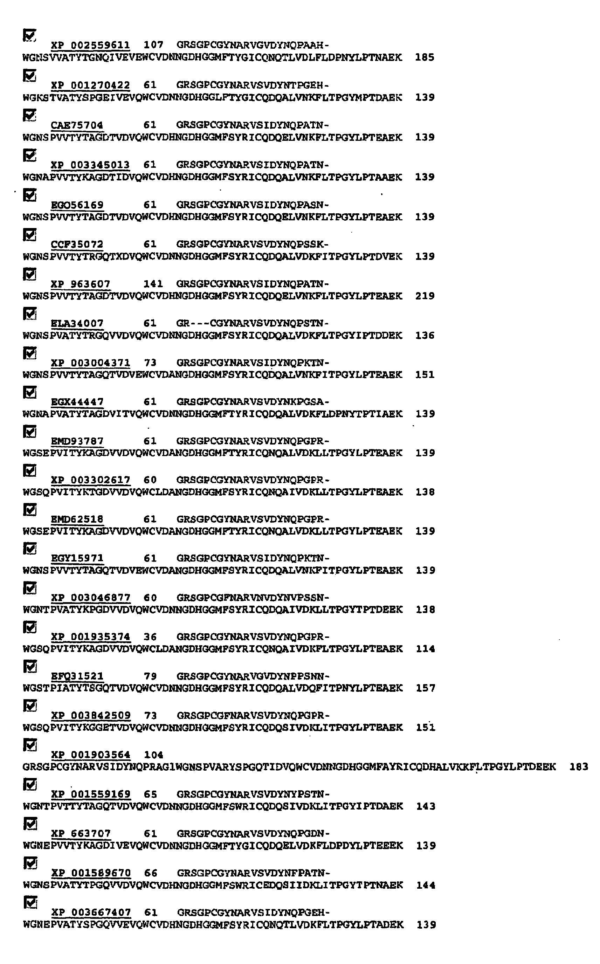

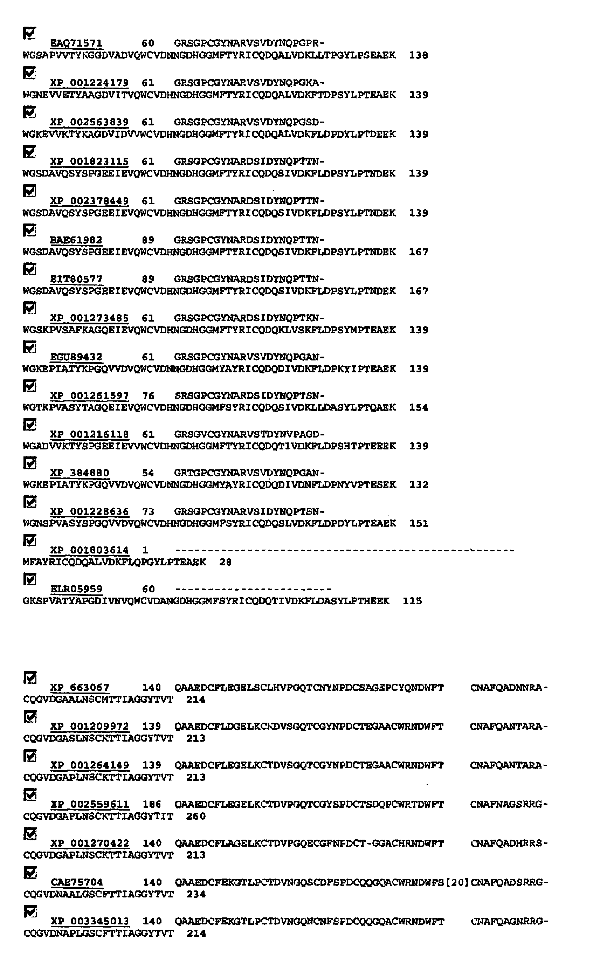

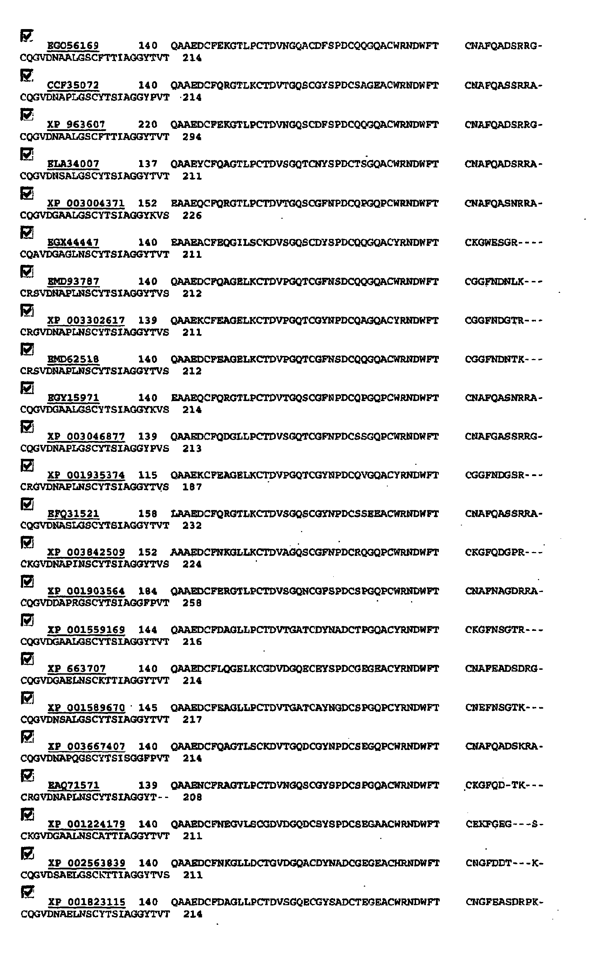

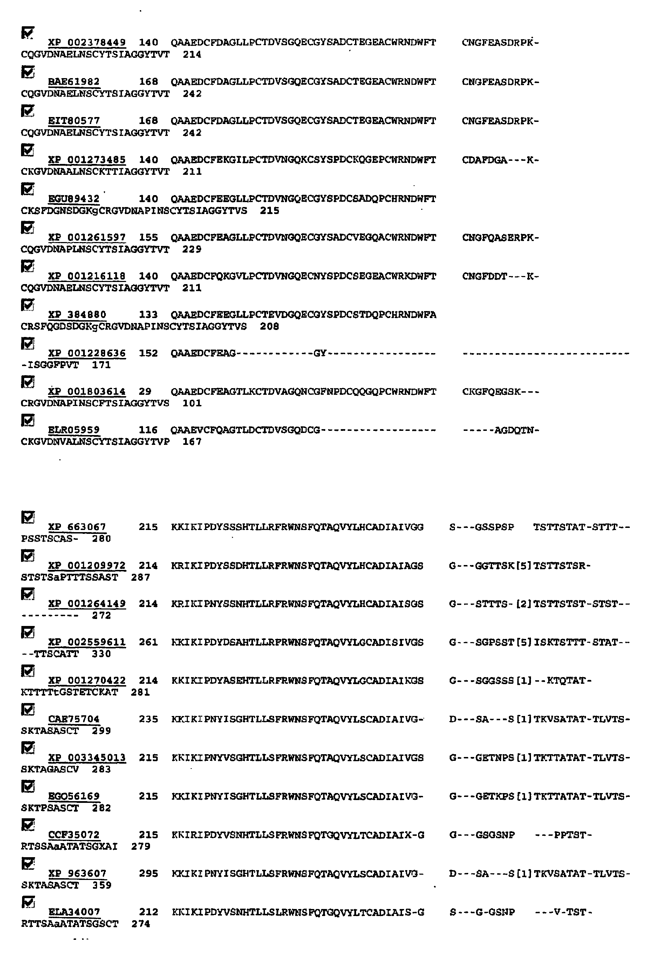

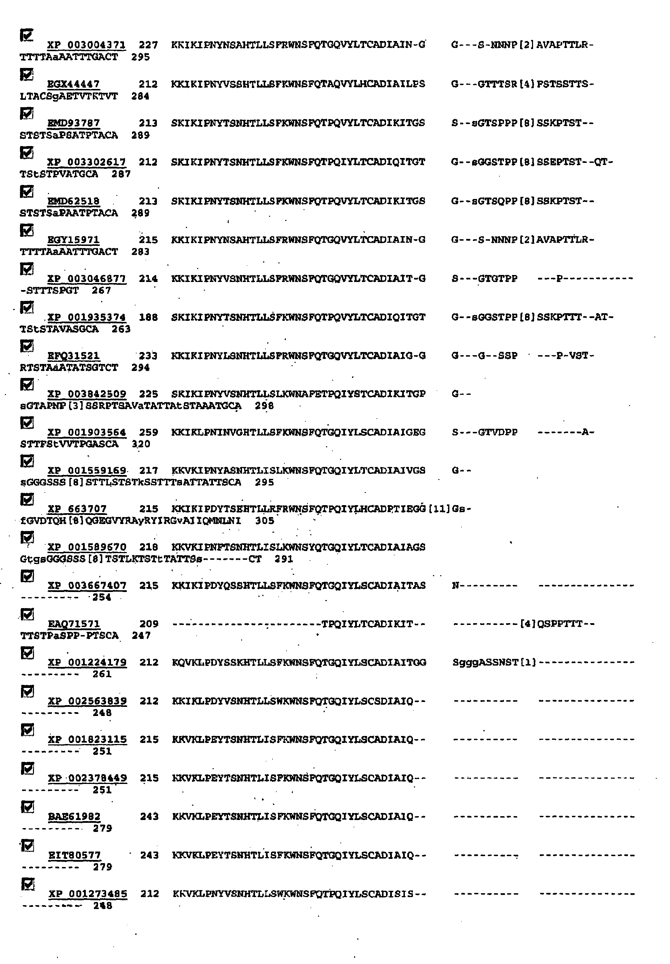

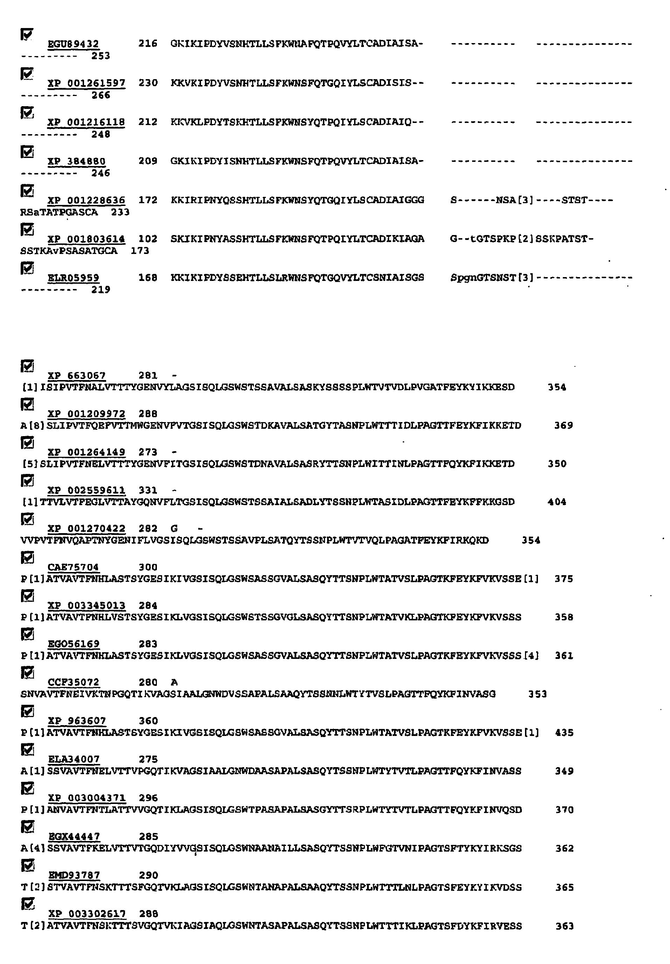

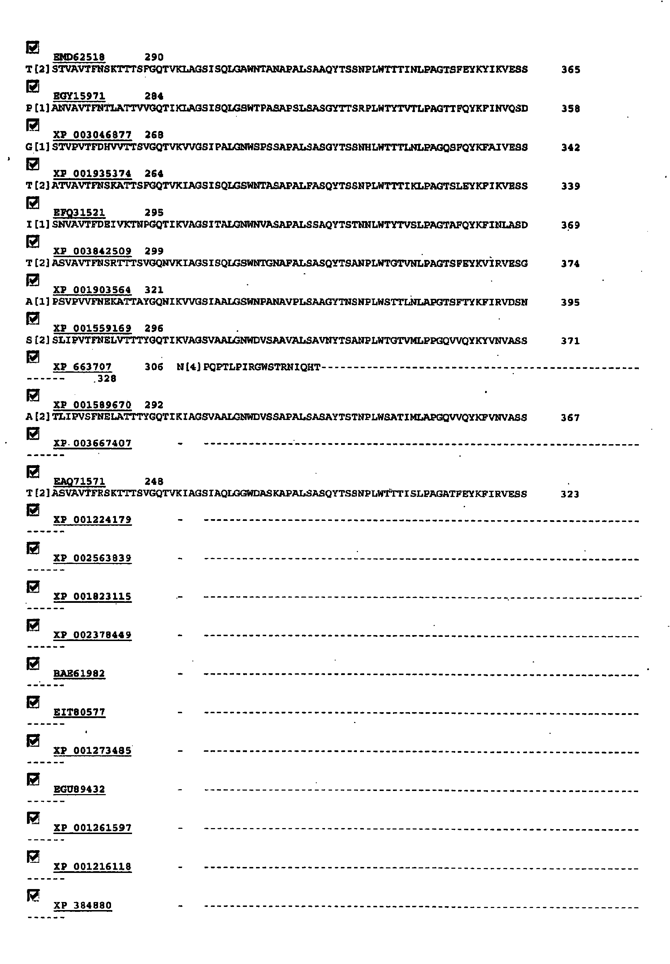

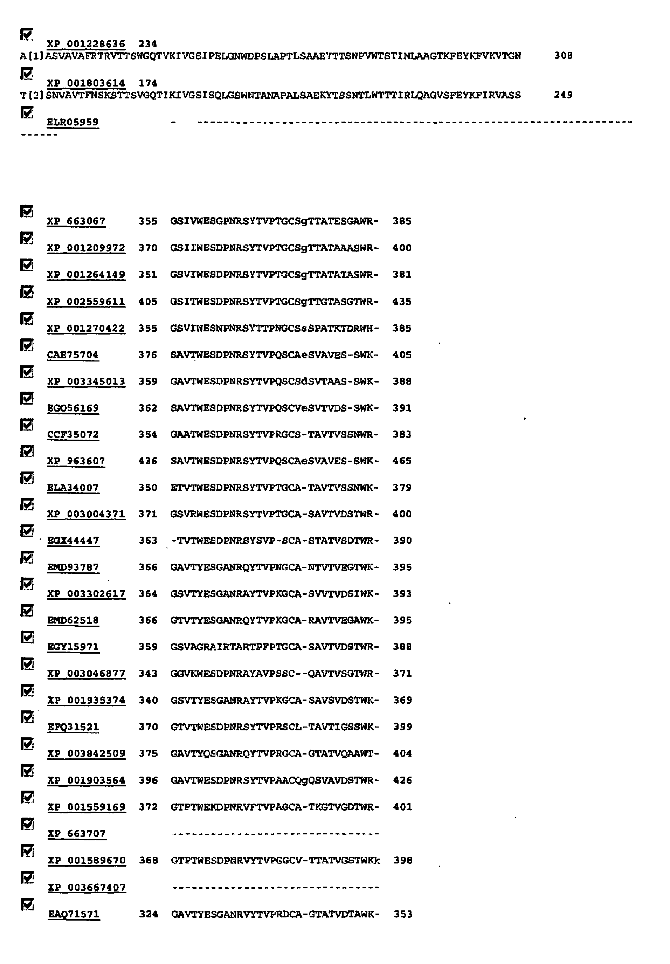

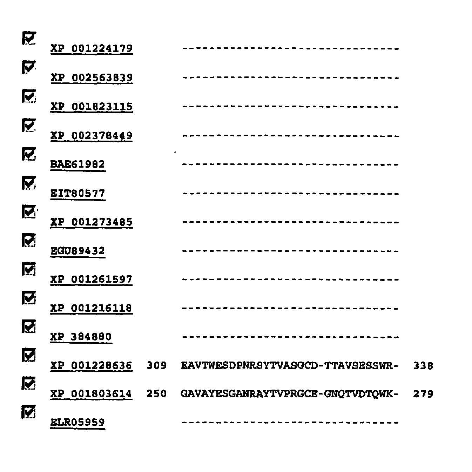

Fig. 1 gives an alignment of homologues of the Aspergillus nidulans CBM20 containing AN5463 protein in other fungal species.
Fig. 2 .BLAST homology of family members to polysaccharide monooxygenase CBP21 (active site histidine indicated).
Fig. 3. PAGE analysis of the proteins precipitated by the starch. Coomassie brilliant blue staining of the protein gels revealed bands of the expected size for the *A. nidulans* AN5463.2 protein which contains an intrinsic CBM20 domain. Also the ***A. oryzae*** BAE61982cur protein (ZY029436) which does not contain a CBM20 domain, was detected after starch binding.

### DETAILED DESCRIPTION

The term "sequence identity," as used herein, is generally expressed as a percentage and refers to the percent of amino acid residues or nucleotides, as appropriate, that are identical as between two sequences when optimally aligned. For the purposes of this invention, sequence identity means the sequence identity determined using the well-known Basic Local Alignment Search Tool (BLAST), which is publicly available through the National Cancer Institute/National Institutes of Health (Bethesda, Maryland) and has been described in printed publications (see, e.g., Altschul et al., J. Mol. Biol, 215(3), 403-10 (1990)). Preferred parameters for amino acid sequences comparison using BLASTP are gap open 11.0, gap extend 1, Blosum 62 matrix. Protein analysis software matches similar sequences using measures of homology assigned to various substitutions, deletions and other modifications. Conservative substitutions typically include substitutions within the following groups: glycine, alanine; valine, isoleucine, leucine; aspartic acid, glutamic acid; asparagine, glutamine; serine, threonine; lysine, arginine; and phenylalanine, tyrosine.

The term "signal peptide" or "signal sequence" as used herein, refers to an amino acid sequence, typically located at the amino terminus of an immature protein or polypeptide (e.g., prior to secretion from a cell and associated processing and cleavage), which directs the secretion of the protein or polypeptide from the cell in which it is produced. The signal peptide typically is removed from an immature protein or polypeptide prior to or during secretion and, thus, is not present in the mature, secreted polypeptide.

As used herein, the term "recombinant nucleic acid molecule" refers to a recombinant DNA molecule or a recombinant RNA molecule. A recombinant nucleic acid molecule is any synthetic nucleic acid construct or nucleic acid molecule containing joined nucleic acid molecules from different original sources or and not naturally occurring or attached together and prepared by using recombinant DNA techniques.

The term "recombinant host cell" as used herein, refers to a host cell strain containing nucleic acid not naturally occurring in that strain and which has been introduced into that strain using recombinant DNA techniques.

The term "nucleic acid" as used herein, includes reference to a deoxyribonucleotide or ribonucleotide polymer, i.e. a polynucleotide, in either single-or double-stranded form, and unless otherwise limited, encompasses known analogues having the essential nature of natural nucleotides in that they hybridize to single-stranded nucleic acids in a manner similar to naturally occurring nucleotides (e. g., peptide nucleic acids). A polynucleotide can be full-length or a subsequence of a native or heterologous structural or regulatory gene. Unless otherwise indicated, the term includes reference to the specified sequence as well as the complementary sequence thereof. Thus, DNAs or RNAs with backbones modified for stability or for other reasons are "polynucleotides" (a polymeric form of nucleotides, either ribonucleotides or deoxyribonucleotides, double-or single-stranded of any length) as that term is intended herein. Moreover, DNAs or RNAs comprising unusual bases, such as inosine, or modified bases, such as tritylated bases, to name just two examples, are polynucleotides as the term is used herein. It will be appreciated that a great variety of modifications have been made to DNA and RNA that serve many useful purposes known to those of skill in the art. The term polynucleotide as it is employed herein embraces such chemically, enzymatically or metabolically modified forms of polynucleotides, as well as the chemical forms of DNA and RNA characteristic of viruses and cells, including among other things, simple and complex cells.

Every nucleic acid sequence herein that encodes a polypeptide also, by reference to the genetic code, describes every possible silent variation of the nucleic acid. The term "conservatively modified variants" applies to both amino acid and nucleic acid sequences. With respect to particular nucleic acid sequences, conservatively modified variants refer to those nucleic acids which encode identical or conservatively modified variants of the amino acid sequences due to the degeneracy of the genetic code.

The term "degeneracy of the genetic code" refers to the fact that a large number of functionally identical nucleic acids encode any given protein. For instance, the codons GCA, GCC, GCG and GCU all encode the amino acid alanine. Thus, at every position where an alanine is specified by a codon, the codon can be altered to any of the corresponding codons described without altering the encoded polypeptide. Such nucleic acid variations are "silent variations" and represent one species of conservatively modified variation.

The term "gene", as used herein, refers to a nucleic acid sequence containing a template for a nucleic acid polymerase, in eukaryotes, RNA polymerase II. Genes are transcribed into mRNAs that are then translated into protein.

"Expression" refers to the transcription of a gene into structural RNA (rRNA, tRNA) or messenger RNA (mRNA) with subsequent translation into a protein.

The term "complementary", as used herein, refers to a sequence of nucleotides which forms a hydrogen-bonded duplex with another sequence of nucleotides according to Watson-Crick base-paring rules. For example, the complementary base sequence for 5'-AAGGCT-3' is 3'-TTCCGA-5'. As used herein, "substantially complementary" means that two nucleic acid sequences have at least about 40, preferably about 50% more preferably at least 55%, more preferably about 60%, more preferably about 70%, more preferably about 80%, even more preferably 90%, and most preferably about 98%, sequence complementarity to each other.

The term "hybridise" refers to the process by which single strands of nucleic acid sequences form double-helical segments through hydrogen bonding between complementary nucleotides.

As used herein, the term "expression control sequence" refers to a nucleic acid sequence that regulates the transcription and translation of a gene to which it is operatively linked. An expression control sequence is "operatively linked" to a gene when the expression control sequence controls and regulates the transcription and, where appropriate, translation of the gene. The term "operatively linked" includes the provision of an appropriate start codon (e.g. ATG), in front of a polypeptide-encoding gene and maintaining the correct reading frame of that gene to permit proper translation of the mRNA.

As used herein, the term "operably linked" refers to a juxtaposition wherein the components so described are in a relationship permitting them to function in their intended manner. A control sequence "operably linked" to another control sequence and/or to a coding sequence is ligated in such a way that transcription and/or expression of the coding sequence is achieved under conditions compatible with the control sequence. Generally, operably linked means that the nucleic acid sequences being linked are contiguous and, where necessary to join two protein coding regions, contiguous and in the same reading frame.

The term "vector" as used herein, includes reference to an autosomal expression vector and to an integration vector used for integration into the chromosome.

The term "expression vector" refers to a DNA molecule, linear or circular, that comprises a segment encoding a polypeptide of interest under the control of (i.e., operably linked to) additional nucleic acid segments that provide for its transcription. Such additional segments may include promoter and terminator sequences, and may optionally include one or more origins of replication, one or more selectable markers, an enhancer, a polyadenylation signal, and the like. Expression vectors are generally derived from plasmid or viral DNA, or may contain elements of both. In particular an expression vector comprises a nucleotide sequence that comprises in the 5' to 3' direction and operably linked: (a) a fungal-recognized transcription and translation initiation region, (b) a coding sequence for a polypeptide of interest, and (c) a fungal-recognized transcription and translation termination region. "Plasmid" refers to autonomously replicating extrachromosomal DNA which is not integrated into a microorganism's genome and is usually circular in nature.

An "integration vector" refers to a DNA molecule, linear or circular, that can be incorporated in a microorganism's genome and provides for stable inheritance of a gene encoding a polypeptide of interest. The integration vector generally comprises one or more segments comprising a gene sequence encoding a polypeptide of interest under the control of (i.e., operably linked to) additional nucleic acid segments that provide for its transcription. Such additional segments may include promoter and terminator sequences, and one or more segments that drive the incorporation of the gene of interest into the genome of the target cell, usually by the process of homologous recombination. Typically, the integration vector will be one which can be transferred into the host cell, but which has a replicon, which is non-functional in that organism. Integration of the segment comprising the gene of interest may be selected if an appropriate marker is included within that segment.

"Transformation" and "transforming", as used herein, refers to the insertion of an exogenous polynucleotide into a host cell, irrespective of the method used for the insertion, for example, direct uptake, transduction, f-mating or electroporation. The exogenous polynucleotide may be maintained as a non-integrated vector, for example, a plasmid, or alternatively, may be integrated into the host cell genome.

By "host cell" is meant a cell which contains a vector or recombinant nucleic acid molecule and supports the replication and/or expression of the vector or recombinant nucleic acid molecule. Host cells may be prokaryotic cells such as E. coli, or eukaryotic cells such as yeast, fungus, plant, insect, amphibian, or mammalian cells. Preferably, host cells are fungal cells.

The term "fungus" or "fungi" includes a wide variety of nucleated, spore-bearing organisms which are devoid of chlorophyll. Examples of fungi include yeasts, mildews, molds, rusts and mushrooms. Preferred fungi in aspects of the present invention are organisms of the genera Aspergillus, more particularly, A. niger, A. nidulans, A. terreus, A. clavatus, A. oryzae or A. flavus" Neurospora, more particularly N. crassa or N. tetrasperma, Sclerotina, Gibberella, Coniothyrium, Psiticum, Magnaporthe, Podospora, Chaetomium, Phaeosphaeria, Botryotinia, Neosartorya, Pyrenophora, Panicum, Aureococcus, Penicillium, Trichoderma, Sordaria, Colleotrichum, Verticillium, Arthrobotrys, Nectria, Leptosphaeria, Fusarium, Glomerella, Geomyces, and Myceliophthora.

The terms "isolated" or "purified" as used herein refer to a nucleic acid or protein or peptide that is removed from at least one component with which it is naturally associated. In the present invention, an isolated nucleic acid can include a vector comprising the nucleic acid. Purified as used herein to describe a polypeptide produced by cultivation of a recombinant host cell refers to removing that polypeptide from at least one component with which it is naturally associated in the host cell or culture medium.

The CBM20 domain that is present in many of the proteins that are held to have activity on starch has a consensus amino acid sequence that comprises the following sequence: [TL]-X₂-G-X₂-[IVLF]-X-[ILV]-X-G-[NSD]-X₃-L-G-X-W-X₅₋₇-[ML]-X₅₋₁₁--W-X₃-[ILV]
in which the amino acids between square brackets are alternatives on that position, and Xₙ denotes a series of n freely chosen amino acids. Alternatively, the CBM20 domain is a sequence that has a high (i.e. more than 70%) identity with the above consensus sequence.. However, no or only very limited catalytic activity has been shown to reside in or be linked to said CBM20 domain.
Now, the inventors have discovered a novel class of starch active proteins that share a common domain, represented by the consensus sequence
[AS] GXD [ST] CPECXI [LR] EPVX [AS] WP [DNST] [LV] [TED]XAXVGR [ST] GPCGYNAR [DV] [SGN] [ITV] DYNXPXXXWGXXX[VI] [IVA] [TS]Y[TKS]XG [DEQ] X [AIV] [DET]V[EQ]WC [VL] D [HAN] NGDHGGM,
More preferably the consensus sequence
HGY [LM] X₆₋₁₀ T [RGK] [VIL] [GN] X [EQN] [AS] GXD [ST] CPECXI [LR] EPVX [AS] WP [DNST] [LV] [TED]XAXVGR [ST] GPCGYNAR [DV] [SGN] [ITV] DYNXPXXXWGXXX[VI] [IVA] [TS]Y[TKS]XG [DEQ] X [AIV] [DET] V [EQ] WC [VL] D [HAN] NGDHGGM[FY] [SAT] [YW] [GR] ICQ [DN] QX [IL] V [DNS] [KL] [FL] [LIT] [DTQ] [AP] X [HY] XP [ST] XXEKXAAEXCFXXGXLXC [TK] [GDE] VXG [AQ] XCX [FY] [NS] [PSG] DCXXXXPAC [WYH] R [NK] DWF [STA] CXX [WF] XXXXXXXCXXVD [NG] [AS] XX [NG] SCXT [TS] I [AS] GG [YF] [TPK]V [STP] X [KR] [VI]K [LI] P
or an amino acid sequence that has a high degree of identity with said consensus sequence. Specifically, the starch active proteins of the invention comprise the following sequence:
hg yltvpasrtr Igfeagidtc pecsilepvs awpdltaaqvgrsgpcgyna rvsvdynqpg dywgnepvvs ytagdvvevq wcvdhngdhg gmftygicqn qtlvdlfltp gylptneekq aaedcflege lsclhvpgqt cnynpdcsag epcyqndwft cnafqadnnr acqgvdgaal nscmttiagg ytvtkkikip dyssshtllr frwnsfqtaq vylhcadiai or an amino acid sequence that has a high degree of identity with this sequence. Alternatively, the starch active proteins of the invention comprise the following sequence: MKVFAPLLSL SLATSVAGHG YMYIPSSRTR LGHEAGIDSC PECAILEPVS 
SWPDLDAAPV GRSGPCGYNA RDSIDYNQPT TNWGSDAVQS YSPGEEIEVQ
WCVDHNGDHG GMFTYRICQD QSIVDKFLDP SYLPTNDEKQ AAEDCFDAGL
LPCTDVSGQE CGYSADCTEG EACWRNDWFT CNGFEASDRP KCQGVDNAEL
NSCYTSIAGG YTVTKKVKLP EYTSNHTLIS FKWNSFQTGQ IYLSCADIAI Q
 or an amino acid sequence that has a high degree of identity with this sequence.

A high degree of identity is herein defined as an identity of more than 70%, preferably more than 75%, more preferably more than 80%, more preferably more than 85%, more preferably more than 90%, more preferably more than 95%, more preferably more than 98%.

Further, orthologs or homologs of this sequences can be found with the following accession numbers:
**Without CBM20**
   XP_663707.1
   XP_003667407.1
   XP_001224179.1
   XP_002563839.1
   XP_001823115.2
   XP_002378449.1
   BAE61982.1
   EIT80577.1
   XP_001273485.1
   EGU89432.1
   XP_001261597.1
   XP_001216118.1
   XP_384880.1
   ELR05959.1
**With CBM20**
   XP_663067.1
   XP_001209972.1
   XP_001264149.1
   XP_002559611.1
   XP_001270422.1
   CAE75704.1
   XP_003345013.1
   EGO56169.1
   CCF35072.1
   XP_963607.1
   ELA34007.1
   XP_003004371.1
   EGX44447.1
   EMD93787.1
   XP_003302617.1
   EMD62518.1
   EGY15971.1
   XP_003046877.1
   XP_001935374.1
   EFQ31521.1
   XP_003842509.1
   XP_001903564.1
   XP_001559169.1
   XP_001589670.1
   EAQ71571.1
   XP_001228636.1
   XP_001803614.1

This new class of proteins has been discovered in the search of proteins with CBM20 domains. It appeared that several of the proteins, especially those from fungal origin contained a conserved domain next to the CBM20 domain. Further search for more proteins that also comprised the conserved new domain has led to the proteins of the present invention, which pertains to proteins that contain both the CBM20 and the new domain and also proteins that only contain the new domain. It is submitted that all currently known proteins with said domain as listed above or a domain which is highly identical thereto are listed in the experimental part and that no clear function was hitherto known from any of these proteins.

The proteins of the invention are generally derived from fungi, although several of them are reported to be associated to plants or algae. For the former two sequences there is no confirmation whether they are actually from plant origin or from an organism, such as a fungus, which was present in or on the plants when sampled. In this category also the following EST sequences can be mentioned: JK051852, JK048632, JK058718 EY432787.1, FE630975.1, FE630976.1, EY428237.1, AM283774.1, CX651361.1 and EH117524.1.

Also part of the invention is a nucleotide sequence encoding one or more of the starch active proteins described above. Such a nucleotide sequence can be any nucleotide sequence that encodes said protein(s), but preferably it is the natural coding sequence found in the organisms from which the starch active proteins are derived. However, if these nucleotide sequences are meant for expression in a different host organism, the nucleotide sequence(s) may be adapted to optimize expression is said host organism (codon optimization). For expression purposes, the nucleotide sequence is included in an expression vector that also provides for regulatory sequences, operably linked with the coding nucleotide sequence.

The proteins of the invention can be used in isolated form for addition to a raw starch substrate, alone or together with other starch degrading enzymes, such as amylase and/or glucoamylase. The proteins of the invention may yield a starch hydrolytic activity per se, or they increase the accessibility of the starch by other starch degrading enzymes.

In another embodiment, the proteins of the invention can be (over)expressed in a host cell. Overexpression can be effected in several ways. It can be caused by transforming a host cell with a gene coding for a protein of the invention. Alternatively, another method for effecting overexpression is to provide a stronger promoter in front of and regulating the expression of said gene. This can be achieved by use of a strong heterologous promoter or by providing mutations in the endogenous promoter. An increased expression of the protein can also be caused by removing possible inhibiting regulatory proteins, e.g. that inhibit the expression of such proteins. The person skilled in the art will know other ways of increasing the activity of the above mentioned starch active proteins.

In another aspect of the invention, host cells overexpressing at least one of the above mentioned nucleotide sequences, encoding at least one starch active protein of the invention, are produced and used, for production of said protein(s).

Host cells used in the invention are preferably cells of filamentous fungi, yeasts and/or bacteria, such as, but not limited to, *Aspergillus* sp., such as the fungi *A. terreus, A. itaconicus* and *A. niger, Aspergillus nidulans, Aspergillus oryzae or Aspergillus fuminagates, Ustilago zeae, Ustilago maydis, Ustilago sp., Candida sp., Yarrowia lipolytica, Rhodotorula sp.* and *Pseudozyma antarctica,* the bacterium *E. coli* and the yeast *Saccharomyces cerevisiae.* Especially preferred are host cells that also produce one or more starch degrading enzymes, such as amylase or glucoamylase.

Recombinant host cells described above can be obtained using methods known in the art for providing cells with recombinant nucleic acids. These include transformation, transconjugation, transfection or electroporation of a host cell with a suitable plasmid (also referred to as vector) comprising the nucleic acid construct of interest operationally coupled to a promoter sequence to drive expression. Host cells of the invention are preferably transformed with a nucleic acid construct as further defined below and may comprise a single but preferably comprises multiple copies of the nucleic acid construct. The nucleic acid construct may be maintained episomally and thus comprise a sequence for autonomous replication, such as an ARS sequence. Suitable episomal nucleic acid constructs may e.g. be based on the yeast 2µ or pKD1 (Fleer et al., 1991, Biotechnology 9: 968-975) plasmids. Preferably, however, the nucleic acid construct is integrated in one or more copies into the genome of the host cell. Integration into the host cell's genome may occur at random by illegitimate recombination but preferably the nucleic acid construct is integrated into the host cell's genome by homologous recombination as is well known in the art of fungal molecular genetics (see e.g. WO 90/14423, EP-A-0 481 008, EP-A-0 635 574 and US 6,265,186). Most preferably for homologous recombination the ku70Δ/ku80Δ technique is used as described for instance in WO 02/052026.

Transformation of host cells with the nucleic acid constructs of the invention and additional genetic modification of the fungal host cells of the invention as described above may be carried out by methods well known in the art. Such methods are e.g. known from standard handbooks, such as Sambrook and Russel (2001) "Molecular Cloning: A Laboratory Manual (3rd edition), Cold Spring Harbor Laboratory, Cold Spring Harbor Laboratory Press, or F. Ausubel et al, eds., "Current protocols in molecular biology", Green Publishing and Wiley Interscience, New York (1987). Methods for transformation and genetic modification of fungal host cells are known from e.g. EP-A-0 635 574, WO 98/46772, WO 99/60102 and WO 00/37671.

In another aspect the invention relates to a vector comprising a nucleotide sequence encoding a starch active protein as defined above and usable for transformation of a host cell as defined above. In the nucleic acid construct, the coding nucleotide sequences preferably is/are operably linked to a promoter for control and initiation of transcription of the nucleotide sequence in a host cell as defined below. The promoter preferably is capable of causing sufficient expression of the starch active protein described above, in the host cell. Promoters useful in the nucleic acid constructs of the invention include the promoter that in nature provides for expression of the coding genes. Further, both constitutive and inducible natural promoters as well as engineered promoters can be used. Promotors suitable to drive expression of the genes in the hosts of the invention include e.g. promoters from glycolytic genes (e.g. from a glyceraldehyde-3-phosphate dehydrogenase gene), ribosomal protein encoding gene promoters, alcohol dehydrogenase promoters (ADH1, ADH4, and the like), promoters from genes encoding amylo- or cellulolytic enzymes (glucoamylase, TAKA-amylase and cellobiohydrolase). Other promoters, both constitutive and inducible and enhancers or upstream activating sequences will be known to those of skill in the art. The promoters used in the nucleic acid constructs of the present invention may be modified, if desired, to affect their control characteristics. Preferably, the promoter used in the nucleic acid construct for expression of the genes is homologous to the host cell in which genes are expressed.

In the nucleic acid construct, the 3'-end of the coding nucleotide acid sequence(s) preferably is/are operably linked to a transcription terminator sequence. Preferably the terminator sequence is operable in a host cell of choice. In any case the choice of the terminator is not critical; it may e.g. be from any fungal gene, although terminators may sometimes work if from a non-fungal, eukaryotic, gene. The transcription termination sequence further preferably comprises a polyadenylation signal.

Optionally, a selectable marker may be present in the nucleic acid construct. As used herein, the term "marker" refers to a gene encoding a trait or a phenotype which permits the selection of, or the screening for, a host cell containing the marker. A variety of selectable marker genes are available for use in the transformation of fungi. Suitable markers include auxotrophic marker genes involved in amino acid or nucleotide metabolism, such as e.g. genes encoding ornithine-transcarbamylases (argB), orotidine-5'-decaboxylases (pyrG, URA3) or glutamine-amido-transferase indoleglycerol-phosphate-synthase phosphoribosyl-anthranilate isomerases (trpC), or involved in carbon or nitrogen metabolism, such e.g. niaD or facA, and antibiotic resistance markers such as genes providing resistance against phleomycin, bleomycin or neomycin (G418). Preferably, bidirectional selection markers are used for which both a positive and a negative genetic selection is possible. Examples of such bidirectional markers are the pyrG (URA3), facA and amdS genes. Due to their bidirectionality these markers can be deleted from transformed filamentous fungus while leaving the introduced recombinant DNA molecule in place, in order to obtain fungi that do not contain selectable markers. This essence of this MARKER GENE FREE™ transformation technology is disclosed in EP-A-0 635 574, which is herein incorporated by reference. Of these selectable markers the use of dominant and bidirectional selectable markers such as acetamidase genes like the amdS genes of *A. nidulans, A. niger* and *P. chrysogenum* is most preferred. In addition to their bidirectionality these markers provide the advantage that they are dominant selectable markers that, the use of which does not require mutant (auxotrophic) strains, but which can be used directly in wild type strains.

Optional further elements that may be present in the nucleic acid constructs of the invention include, but are not limited to, one or more leader sequences, enhancers, integration factors, and/or reporter genes, intron sequences, centromers, telomers and/or matrix attachment (MAR) sequences. The nucleic acid constructs of the invention may further comprise a sequence for autonomous replication, such as an ARS sequence. Suitable episomal nucleic acid constructs may e.g. be based on the yeast 2µ or pKD1 (Fleer et al., 1991, Biotechnology 9: 968-975) plasmids. Alternatively the nucleic acid construct may comprise sequences for integration, preferably by homologous recombination (see e.g. WO98/46772). Such sequences may thus be sequences homologous to the target site for integration in the host cell's genome. The nucleic acid constructs of the invention can be provided in a manner known per se, which generally involves techniques such as restricting and linking nucleic acids/nucleic acid sequences, for which reference is made to the standard handbooks, such as Sambrook and Russel (2001) "Molecular Cloning: A Laboratory Manual (3rd edition), Cold Spring Harbor Laboratory, Cold Spring Harbor Laboratory Press, or F. Ausubel et al, eds., "Current protocols in molecular biology", Green Publishing and Wiley Interscience, New York (1987).

In a further aspect the invention relates to fermentation processes in which the transformed host cells of the invention are used for the conversion of a starch substrate. A preferred fermentation process is an aerobic fermentation process. The fermentation process may either be a submerged or a solid state fermentation process.

In a solid state fermentation process (sometimes referred to as semi-solid state fermentation) the transformed host cells are fermenting on a solid medium that provides anchorage points for the fungus in the absence of any freely flowing substance. The amount of water in the solid medium can be any amount of water. For example, the solid medium could be almost dry, or it could be slushy. A person skilled in the art knows that the terms "solid state fermentation" and "semi-solid state fermentation" are interchangeable. A wide variety of solid state fermentation devices have previously been described (for review see, Larroche et al., "Special Transformation Processes Using Fungal Spores and Immobilized Cells", Adv. Biochem. Eng. Biotech., (1997), Vol 55, pp. 179; Roussos et al., "Zymotis: A large Scale Solid State Fermenter", Applied Biochemistry and Biotechnology, (1993), Vol. 42, pp. 37-52; Smits et al., "Solid-State Fermentation-A Mini Review, 1998), Agro-Food-Industry Hi-Tech, March/April, pp. 29-36). These devices fall within two categories, those categories being static systems and agitated systems. In static systems, the solid media is stationary throughout the fermentation process. Examples of static systems used for solid state fermentation include flasks, petri dishes, trays, fixed bed columns, and ovens. Agitated systems provide a means for mixing the solid media during the fermentation process. One example of an agitated system is a rotating drum (Larroche et al., *supra*). In a submerged fermentation process on the other hand, the transformed fungal host cells are fermenting while being submerged in a liquid medium, usually in a stirred tank fermenter as are well known in the art, although also other types of fermenters such as e.g. airlift-type fermenters may also be applied (see e.g. US 6,746,862).

Preferred in the invention is a submerged fermentation process, which is performed fed-batch. This means that there is a continuous input of feed containing a carbon source and/or other relevant nutrients in order to improve protein yields. The input of the feed can, for example, be at a constant rate or when the concentration of a specific substrate or fermentation parameter falls below some set point.

Further comprised in the invention is the use of a starch active protein according to the invention for modification of raw starch substrate. Preferably said use encompasses e reformation of the starch by the starch active protein, thereby allowing other starch degrading enzymes to approach the starch and exert their function.

### EXAMPLES

### Example 1: Genome mining for Starch active protein like proteins

Genome mining lead to the identification of a novel class of fungal proteins with activity towards insoluble starch.

This class is characterised by the following criteria:
- No significant homology to catalytic core of any known fungal starch active GH family members;
- No homology to GH13 / GH15
- Three different classes of protein could be identified
- No homologues for the two major subfamilies in *Aspergillus niger* and *Trichoderma reesei*

The *Aspergillus nidulans* CBM20 containing AN5463 has limited similarity to polysaccharide monooxygenases CBM33/GH61, making this a novel class of enzymes active towards starch: The protein has a very limited number of orthologues in Aspergillus species (and also in some other species; see homology tree).

**Homologues in Aspergillus species**

| | | |
|---|---|---|
| A. oryzae | AOR_1_454114 (no CBM20) | - |
| A. nidulans | AN6103 (no CBM20) | Clustered with fungal TFX domain gene |

| **A.nidulans** | **AN5463** | **Clustered with fungal TFX domain gene** |
|---|---|---|
| | | |
| A. nidulans | AN0778 | Clustered with PL3 (pectate lyase) / VHS-ENTH-ANTH |
| A. oryzae | AOR_1_614054 | Clustered with PL3 (pectate lyase) / VHS-ENTH-ANTH |
| A. niger | An01g12440 | Clustered with PL3 (pectate lyase) / VHS-ENTH-ANTH |

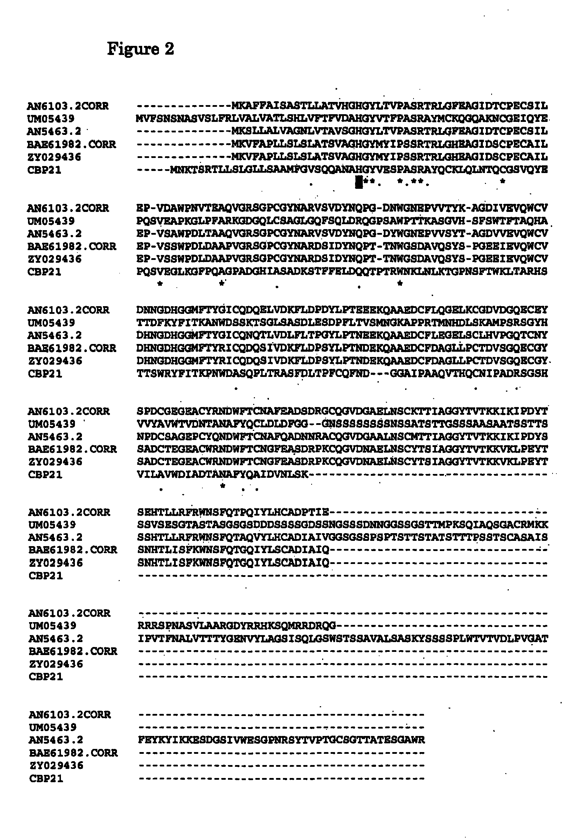

The sequence of these proteins and a homology blast is provided in Fig. 2.

Below is a further list of homologues in other fungal species (see also the alignment in the Figures)

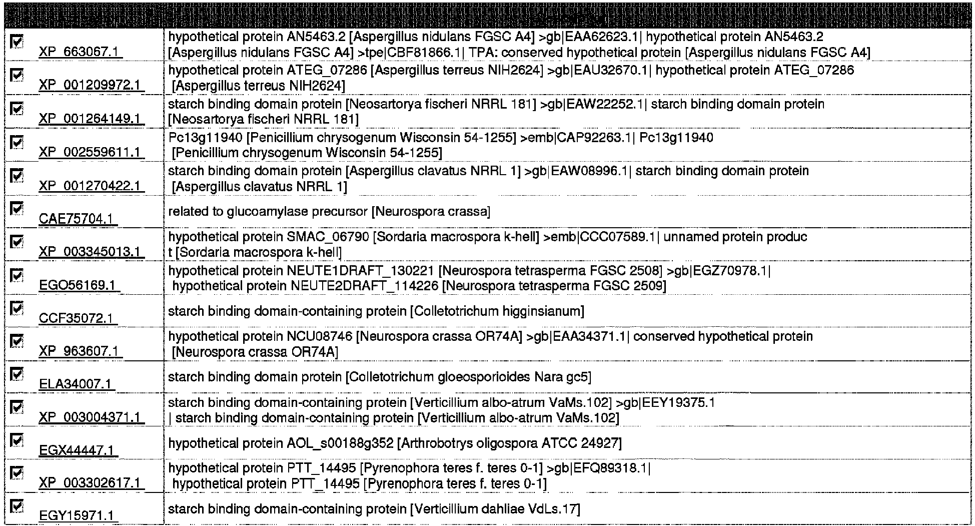

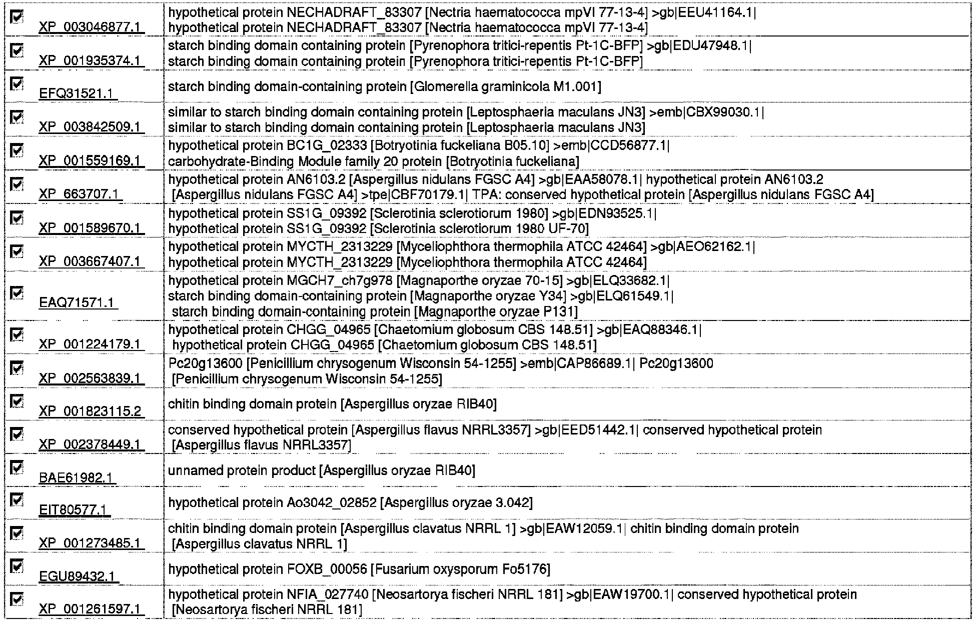

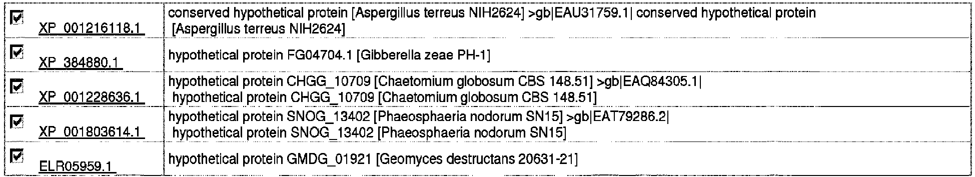

### Example 2. Expression of genes encoding novel starch active proteins in Aspergillus niger

In order to unambiguously establish that the discovered proteins aid to the increased saccharification of alpha-glucan containing plant derived substrates, a host naturally not expressing these genes was (co-)transformed with the respective genes under control of a suitable promoter

### (i) Gene design

Synthetic (codon-optimized) full length gene copies from the A. nidulans AN5463.2, AN6103.2 (the latter curated based on genome mining) and two versions (one with and one without CBM20 domain) of the *A. oryzae* BAE61982 gene (curated based on EST sequence ZY029436) were generated based on the manually curated *A. nidulans*/*oryzae* deposited Genbank sequences. For the construction of the hybrid (CBM20 containing) version of the *A. oryzae* BAE61982 gene the well characterized GlaA CBM20 domain was used.

CLUSTAL multiple sequence alignment

<<<------------------------------------------
ZY029436                MKVFAPLLSLSLATSVAGHGYMYIPSSRTRLGHEAGIDSCPECAILEPVSSWPDLDAAPV
AN5463.2                MKSLLALVAGNLVTAVSGHGYLTVPASRTRLGFEAGIDTCPECSILEPVSAWPDLTAAQV
ZY029436 + CBM20       MKVFAPLLSLSLATSVAGHGYMYIPSSRTRLGHEAGIDSCPECAILEPVSSWPDLDAAPV
An6103.2                MKAFFAISASTLLATVHGHGYLTVPASRTRLGFEAGIDTCPECSILEPVDAWPNVTEAQV
                        **    . .  * ..* ****. .*.****** *****.****.***** .** .  * *

                        ------------------------------------------------------------
ZY029436                GRSGPCGYNARDSIDYNQPTTNWGSDAVQSYSPGEEIEVQWCVDHNGDHGGMFTYRICQD
AN5463.2                GRSGPCGYNARVSVDYNQPGDYWGNEPVVSYTAGDVVEVQWCVDHNGDHGGMFTYGICQN
A.oryzaee BAE61982hybrGRSGPCGYNARDSIDYNQPTTNWGSDAVQSYSPGEEIEVQWCVDHNGDHGGMFTYRICQD
An6103.2                GRSGPCGYNARVSVDYNQPGDNWGNEPVVTYKAGDIVEVQWCVDNNGDHGGMFTYGICQD
                        *********** *.*****   ** . * .*  *. .*******.********** ***

                                    Conserved domain SAP
ZY029436                QSIVDKFLDPSYLPTNDEKQAAEDCFDAGLLPCTDVSGQECGYSADCTEGEACWRNDWFT
AN5463.2                QTLVDLFLTPGYLPTNEEKQAAEDCFLEGELSCLHVPGQTCNYNPDCSAGEPCYQNDWFT
ZY029436 + CBM20       QSIVDKFLDPSYLPTNDEKQAAEDCFDAGLLPCTDVSGQECGYSADCTEGEACWRNDWFT
An6103.2                QELVDKFLDPDYLPTEEEKQAAEDCFLQGELKCGDVDGQECEYSPDCGEGEACYRNDWFT
                        * .** ** * **** .*********  * * *  * ** * *  **  ** * .*****

------------------------------------------------------------
ZY029436 CNGFEASDRPKCQGVDNAELNSCYTSIAGGYTVTKKVKLPEYTSNHTLISFKWNSFQTGQ
AN5463.2 CNAFQADNNRACQGVDGAALNSCMTTIAGGYTVTKKIKIPDYSSSHTLLRFRWNSFQTAQ
ZY029436 + CBM20 CNGFEASDRPKCQGVDNAELNSCYTSIAGGYTVTKKVKLPEYTSNHTLISFKWNSFQTGQ
A. nidulans An6103.2cuCNAFEADSDRGCQGVDGAELNSCKTTIAGGYTVTKKIKIPDYTSEHTLLRFRWNSFQTPQ
** *.* *****.* **** *.**********.*.*.*.* ***. *.****** *

-----→>>>>><<<<< S/T riche hinge region >>>>><<<<<---
ZY029436 IYLSCADIAIQ-------------------------------------------------
AN5463.2 VYLHCADIAIVG--------------GSGSSPSPTSTTSTATSTTTP-SSTSCASAISIP
ZY029436 + CBM20 IYLSCADIAIPSIVATGGTTTTATPTGSGSVTSTSKTTATASKTSTSTSSTSCTTPTAVA
An6103.2 IYLHCADPTIE-------------------------------------------------
.** *** .* **** * ** ** * * *****

------------------------CBM20 domain------------------------
AN5463.2 VTFNALVTTTYGENVYLAGSISQLGSWSTSSAVALSASKYSSSSPLWTVTVDLPVGATFE
ZY029436 + CBM20 VTFDLTATTTYGENIYLVGSISQLGDWETSDGIALSADKYTSSDPLWYVTVTLPAGESFE
*** ******* ** ******* **** **** ** ** *** *** * * **

------------------------------------->>>>>
AN5463.2 YKYIKKESDGSIVWESGPNRSYTVPTGCSGTTATESGAWR
ZY029436 + CBM20 YKFIRIESDDSVEWESDPNREYTVPQACGISTATVTDTWR
** * *** * *** *** **** * *** **

### (ii) Overexpresssion of synthetic genes copies

The synthetic gene copies were inserted in an expression vector based on the *A. nidulans* gpdA promoter, carrying also the amdS selection marker. This Aspergillus expression vector pAN52-4amdSdoubleNotI was derived by cloning the Aspergillus selection marker amdS and an additional NotI cloning site into the Aspergillus expression vector pAN52-4 (EMBL accession #Z32699).

The resulting expression vectors were introduced in a protease deficient A. *niger* host strain devoid of any CBM20 containing proteins (MGG029ΔamaA: see Van Der Kaaij et al., 2007;Weenink et al., 2006). ΔmdS+ transformants were selected using acrylamide selection. Microplate cultures of a collection of transformants per gene version were cultivated and screened for the presence of starch binding proteins using a small scale starch based binding assay and SDS PAGE.

### (iii) Fermentation and protein purification

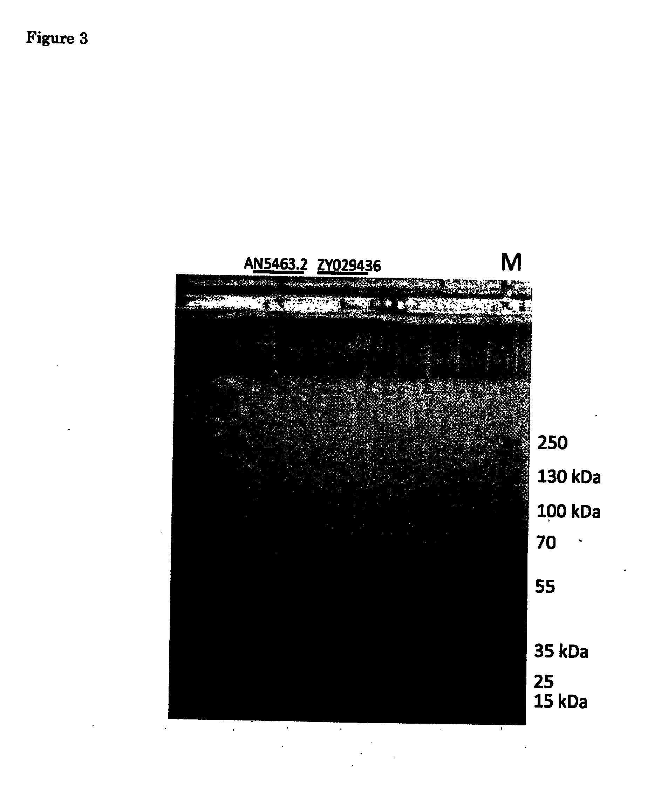

Transformants selected from 3(ii) were cultivated in standard fedbatch fermentation and the starch active proteins produced were purified from the culture supernatant using selective starch binding (Van der Kaaij et al., 2007), followed by PAGE analysis of the proteins precipitated by the starch. Coomassie brilliant blue staining of the protein gels revealed bands of the expected size for the *A. nidulans* AN5463.2 protein which contains an intrinsic CBM20 domain. Also the *A. oryzae* BAE61982cur protein (ZY029436) which does not contain a CBM20 domain, was detected after starch binding, suggesting that the conserved SAP domain contains intrinsic starch binding activity (see Fig. 3).

### (iv) Standard saccharification analysis

The purified Sap proteins were subsequently tested for their effects on starch hydrolysis using a standard α-amylase based starch saccharification assay. Saccharification (60 min 90°C) in the presence of SAP proteins resulted in increased release of glucose compared to saccharification (60 min 90°C) in the absence of SAP proteins.

| | **Treatment** | **Glucose (g/l)** |
|---|---|---|
| α-amylase | 60 min 90C | 5.599 |
| α-amylase + AN5463.2 | 60 min 90C | 7.248 |
| α-amylase + ZY029436 | 60 min 90C | 6.101 |

The positive effect of the SAP proteins on the release of glucose when incubated at 90°C in the presence of α-amylase was confirmed by testing whether the SAP proteins alone would have a positive effect on the release of glucose when incubated o/n at 37°C with starch prior to the addition of α-amylase and the 60 min 90°C heat treatment. The results were similar to the saccharification results without pre-incubation, confirming the positive effect of the SAP proteins on glucose release in the presence of α-amylase at 90°C and indicating that the SAP proteins alone did not have a strong effect on saccharification efficiency.

| | **Treatment 1** | **Treatment 2** | **Treatment 3** | **Glucose (g/l)** |
|---|---|---|---|---|
| α-amylase | | | 60 min 90C | 5.599 |
| AN5463.2 | o/n 37C | α-amylase added | 60 min 90C | 7.551 |
| ZY029436 | o/n 37C | α-amylase added | 60 min 90C | 6.094 |

### (v) Analysis of oxidative enzyme activities

The HPAEC-MS analysis method (Coulier et al., *2013*)) can be used to distinguish in short sugar-polymers of up to 6 units, whether deoxy-sugars or sugar-acids are present in the molecule. To assess whether the starch active proteins produced in fermentation supernatants from (iii) had any sugar-oxidation activities, these supernatants were incubated with starch prior to addition of amylase. After the starch had been saccharified by amylase to monomers and short sugar polymers, the samples were analysed by HPAEC-MS. The results showed that the starch active proteins produced an additional deoxy-sugar (dC6-C6).

### (vi) Summary of the SAP protein analysis

| | AN5463.2 | ZY029436 |
|---|---|---|
| Starch binding domain (CBM20) | yes | no |
| Protein band after starch binding | yes | yes |
| Increased glucose release when SAP and a-amylase are present | yes | yes |
| Additional deoxy sugar after saccharification | yes: dC6-C6 | yes: dC6-C6 |

van der Kaaij, R. M., Yuan, X. L., Franken, A., Ram, A. F. J., Punt, P. J., Maarel, M. J. E. C. & Dijkhuizen, L. (2007). Characterization of two novel, putatively cell wall associated and GPI-anchored, a-glucanotransferase enzymes of Aspergillus niger. Eukaryot Cell 6, 1178-1188.
Weenink, X.O., Punt, P.J., Van Den Hondel, C.A.M.J.J., Ram, A.F.J. A new method for screening and isolation of hypersecretion mutants in Aspergillus niger (2006) Applied Microbiology and Biotechnology, 69 (6), pp. 711-717
Leon Coulier, Ying Zha, Richard Bas, Peter J. Punt Analysis of oligosaccharides in lignocellulosic biomass hydrolysates by high-performance anion-exchange chromatography coupled with mass spectrometry (HPAEC-MS) Bioresource Technology, Volume 133, April 2013, Pages 221-231

## Claims

1. Use of a starch active protein comprising a domain comprising the amino acid sequence
[AS] GXD [ST] CPECXI [LR] EPVX [AS] WP [DNST] [LV] [TED]XAXVGR [ST] GPCGYNAR [DV] [SGN] [ITV] DYNXPXXXWGXXX[VI] [IVA][TS]Y[TKS]XG [DEQ] X [AIV] [DET]V[EQ]WC [VL] D [HAN] NGDHGGM,
or a sequence that has an identity of more than 70%, preferably more than 75%, more preferably more than 80%, more preferably more than 85%, more preferably more than 90%, more preferably more than 95%, more preferably more than 98% with said amino acid sequence in the modification of raw starch

2. Use according to claim 1 wherein said starch ctive protein has a domain comprising the amino acid sequence
HGY [LM] X₆₋₁₀ T [RGK] [VIL] [GN] X [EQN] [AS] GXD [ST] CPECXI [LR] EPVX [AS] WP [DNST] [LV] [TED]XAXVGR [ST] GPCGYNAR [DV] [SGN] [ITV] DYNXPXXXWGXXX[VI] [IVA] [TS]Y[TKS]XG [DEQ] X [AIV] [DET] V [EQ] WC [VL] D [HAN] NGDHGGM[FY] [SAT] [YW] [GR] ICQ [DN] QX [IL] V [DNS] [KL] [FL] [LIT] [DTQ] [AP] X [HY] XP [ST] XXEKXAAEXCFXXGXLXC [TK] [GDE] VXG [AQ] XCX [FY] [NS] [PSG] DCXXXXPAC [WYH] R [NK] DWF [STA] CXX [WF] XXXXXXXCXXVD [NG] [AS] XX [NG] SCXT [TS] I [AS] GG [YF] [TPK]V [STP] X [KR] [VI]K [LI] P
or a sequence that has an identity of more than 70%, preferably more than 75%, more preferably more than 80%, more preferably more than 85%, more preferably more than 90%, more preferably more than 95%, more preferably more than 98% with said amino acid sequence in the modification of raw starch.

3. Use according to claim 1 or 2, wherein the starch active protein comprises the sequence:
hg yltvpasrtr Igfeagidtc pecsilepvs awpdltaaqvgrsgpcgyna rvsvdynqpg dywgnepvvs ytagdvvevq wcvdhngdhg gmftygicqn qtlvdlfltp gylptneekq aaedcflege lsclhvpgqt cnynpdcsag epcyqndwft cnafqadnnr acqgvdgaal nscmttiagg ytvtkkikip dyssshtllr frwnsfqtaq vylhcadiai
or a sequence that has an identity of more than 70%, preferably more than 75%, more preferably more than 80%, more preferably more than 85%, more preferably more than 90%, more preferably more than 95%, more preferably more than 98% with said amino acid sequence.

4. Use according to claim 1 or 2, wherein the starch active protein comprises the sequence: 
MKVFAPLLSL SLATSVAGHG YMYIPSSRTR LGHEAGIDSC PECAILEPVS
SWPDLDAAPV GRSGPCGYNA RDSIDYNQPT TNWGSDAVQS YSPGEEIEVQ
WCVDHNGDHG GMFTYRICQD QSIVDKFLDP SYLPTNDEKQ AAEDCFDAGL
LPCTDVSGQE CGYSADCTEG EACWRNDWFT CNGFEASDRP KCQGVDNAEL
NSCYTSIAGG YTVTKKVKLP EYTSNHTLIS FKWNSFQTGQ IYLSCADIAI Q
 or a sequence that has an identity of more than 70%, preferably more than 75%, more preferably more than 80%, more preferably more than 85%, more preferably more than 90%, more preferably more than 95%, more preferably more than 98% with said amino acid sequence.

5. Use according to claim 1 or 2, wherein the starch active protein is selected from the group consisting of the proteins with the NCBI accession no.
XP_663707.1, XP_003667407.1, XP_001224179.1, XP_002563839.1, XP_001823115.2, XP_002378449.1, BAE61982.1, EIT80577.1, XP_001273485.1 ,EGU89432.1 ,XP_001261597.1 ,XP_001216118.1 ,XP_384880.1, ELR05959.1, JK051852, JK048632, JK058718 EY432787.1, FE630975.1, FE630976.1, EY428237.1, AM283774.1, CX651361.1 and EH117524.1,
and the proteins encoded by the genes comprising the ESTs with the NCBI accession no. JK051852, JK048632, JK058718 FE630975.1, FE630976.1, AM283774.1 and, CX651361.1.

6. Use according to claim 1 or 2, wherein the starch active protein additionally comprises a CBM20 domain.

7. Use according to claim 6, wherein the starch active protein is selected from the group consisting of the proteins with NCBI accession no.
XP_663067.1 ,XP_001209972.1 ,XP_001264149.1 ,XP_002559611.1 ,XP_001270422.1 ,CAE75704.1 ,XP_003345013.1 ,EG056169.1 ,CCF35072.1 ,XP_963607.1 ,ELA34007.1 ,XP_003004371.1 ,EGX44447.1 ,EMD93787.1 ,XP_003302617.1 ,EMD62518.1 ,EGY15971.1 ,XP_003046877.1 ,XP_001935374.1 ,EFQ31521.1 ,XP_003842509.1 ,XP_001903564.1 ,XP_001559169.1 ,XP_001589670.1 .EAQ71571.1 ,XP_001228636.1 and XP_001803614.1.

8. Use according to any of claims 1-6, wherein the starch active protein is from fungal origin, preferably wherein the fungus is chosen from the group consisting of Aspergillus, more particularly, A. niger, A. nidulans, A. terreus, A. clavatus, A. oryzae or A. flavus" Neurospora, more particularly N. crassa or N. tetrasperma, Sclerotina, Gibberella, Coniothyrium, Psiticum, Magnaporthe, Podospora, Chaetomium, Phaeosphaeria, Botryotinia, Neosartorya, Pyrenophora, Panicum, Aureococcus, Penicillium, Trichoderma, Sordaria, Colleotrichum, Verticillium, Arthrobotrys, Nectria, Leptosphaeria, Fusarium, Glomerella, Geomyces, and Myceliophthora,.

9. A vector comprising a nucleic acid encoding a starch active protein as defined in any of claims 1-8..

10. A host cell capable of overexpressing a starch active protein as defined in any of claims 1-8.

11. A host cell according to claim 10 comprising a vector according to claim 9.

12. Use according to any of claims 1-8, wherein the starch active protein increases the accessibility of the starch for starch degrading enzymes.

13. Method for the modification of raw starch comprising bringing starch into contact with one or more of the starch active proteins as defined in any of claim 1-8, preferably wherein said modification is starch hydrolysis or starch oxidation..

14. Method for the production of an oligomeric deoxy sugar comprising bringing starch into contact with one or more of the starch active proteins as defined in any of claims 1-8.

15. Method for the enhanced degradation of starch wherein the starch is treated with a mixture of amylase and a starch active protein as defined in any of claims 1-8.


SEQUENCE LISTING

<110> Nederlandse Organisatie voor toegepast-
natuurwetenschappelijk onderzoek TNO

<120> Novel starch active proteins

<130> P100745EP00

<140> EP 13157503.7
<141> 2013-03-01

<160> 56

<170> PatentIn version 3.5

<210> 1
<211> 78
<212> PRT
<213> Artificial Sequence

<220>
<223> consensus CBM20 domain


<220>
<221> VARIANT
<222> (1)..(1)
<223> May be S

<220>
<221> misc_feature
<222> (3)..(3)
<223> Xaa can be any naturally occurring amino acid

<220>
<221> VARIANT
<222> (5)..(5)
<223> May be T

<220>
<221> misc_feature
<222> (10)..(10)
<223> Xaa can be any naturally occurring amino acid

<220>
<221> VARIANT
<222> (12)..(12)
<223> May be R

<220>
<221> misc_feature
<222> (16)..(16)
<223> Xaa can be any naturally occurring amino acid

<220>
<221> VARIANT
<222> (17)..(17)
<223> May be S

<220>
<221> VARIANT
<222> (20)..(20)

<223>  May be N, S or T

<220>
<221>  VARIANT
<222>  (21)..(21)
<223>  May be V

<220>
<221>  VARIANT
<222>  (22)..(22)
<223>  May be E or D

<220>
<221>  misc_feature
<222>  (23)..(23)
<223>  Xaa can be any naturally occurring amino acid

<220>
<221>  misc_feature
<222>  (25)..(25)
<223>  Xaa can be any naturally occurring amino acid

<220>
<221>  VARIANT
<222>  (29)..(29)
<223>  May be S

<220>
<221>  VARIANT
<222>  (38)..(38)
<223>  May be V

<220>
<221>  VARIANT
<222>  (39)..(39)
<223>  May be G or N

<220>
<221>  VARIANT
<222>  (40)..(40)
<223>  May be T or V

<220>
<221>  misc_feature
<222>  (44)..(44)
<223>  Xaa can be any naturally occurring amino acid

<220>
<221>  misc_feature
<222>  (46)..(48)
<223>  Xaa can be any naturally occurring amino acid

<220>
<221>  misc_feature
<222>  (51)..(53)
<223>  Xaa can be any naturally occurring amino acid

<220>
<221>  VARIANT
<222>  (54)..(54)
<223>  May be I

<220>

<221>  VARIANT
<222>  (55)..(55)
<223>  May be V or A

<220>
<221>  VARIANT
<222>  (56)..(56)
<223>  May be S

<220>
<221>  VARIANT
<222>  (58)..(58)
<223>  May be K or S

<220>
<221>  misc_feature
<222>  (59)..(59)
<223>  Xaa can be any naturally occurring amino acid

<220>
<221>  VARIANT
<222>  (61)..(61)
<223>  May be E or T

<220>
<221>  misc_feature
<222>  (62)..(62)
<223>  Xaa can be any naturally occurring amino acid

<220>
<221>  VARIANT
<222>  (63)..(63)
<223>  May be I or V

<220>
<221>  VARIANT
<222>  (64)..(64)
<223>  May be E or T

<220>
<221>  VARIANT
<222>  (66)..(66)
<223>  May be q

<220>
<221>  VARIANT
<222>  (69)..(69)
<223>  May be L

<220>
<221>  VARIANT
<222>  (71)..(71)
<223>  May be A or N

<400>  1

Ala Gly Xaa Asp Ser Cys Pro Glu Cys Xaa Ile Leu Glu Pro Val Xaa
1               5                   10                  15

Ala Trp Pro Asp Leu Thr Xaa Ala Xaa Val Gly Arg Ser Gly Pro Cys
            20                  25                  30

Gly Tyr Asn Ala Arg Asp Ser Ile Asp Tyr Asn Xaa Pro Xaa Xaa Xaa
        35                  40                  45

Trp Gly Xaa Xaa Xaa Val Ile Thr Tyr Thr Xaa Gly Asp Xaa Ala Asp
    50                  55                  60

Val Glu Trp Cys Val Asp His Asn Gly Asp His Gly Gly Met
65                  70                  75

<210>  2
<211>  203
<212>  PRT
<213>  Artificial Sequence

<220>
<223>  consensus starch active domain

<220>
<221>  VARIANT
<222>  (4)..(4)
<223>  May be M

<220>
<221>  misc_feature
<222>  (5)..(10)
<223>  Xaa can be any naturally occurring amino acid

<220>
<221>  VARIANT
<222>  (12)..(12)
<223>  May be G or K

<220>
<221>  VARIANT
<222>  (13)..(13)
<223>  May be I or L

<220>
<221>  VARIANT
<222>  (14)..(14)
<223>  May be N

<220>
<221>  misc_feature
<222>  (15)..(15)
<223>  Xaa can be any naturally occurring amino acid

<220>
<221>  VARIANT
<222>  (16)..(16)
<223>  May be Q or N

<220>
<221>  VARIANT
<222>  (17)..(17)
<223>  May be S

<220>

<221>  misc_feature
<222>  (19)..(19)
<223>  Xaa can be any naturally occurring amino acid

<220>
<221>  VARIANT
<222>  (21)..(21)
<223>  May be T

<220>
<221>  misc_feature
<222>  (26)..(26)
<223>  Xaa can be any naturally occurring amino acid

<220>
<221>  VARIANT
<222>  (28)..(28)
<223>  May be R

<220>
<221>  misc_feature
<222>  (32)..(32)
<223>  Xaa can be any naturally occurring amino acid

<220>
<221>  VARIANT
<222>  (33)..(33)
<223>  May be S

<220>
<221>  VARIANT
<222>  (36)..(36)
<223>  May be N, S or T

<220>
<221>  VARIANT
<222>  (37)..(37)
<223>  May be V

<220>
<221>  VARIANT
<222>  (38)..(38)
<223>  May be E or D

<220>
<221>  misc_feature
<222>  (39)..(39)
<223>  Xaa can be any naturally occurring amino acid

<220>
<221>  misc_feature
<222>  (41)..(41)
<223>  Xaa can be any naturally occurring amino acid

<220>
<221>  VARIANT
<222>  (45)..(45)
<223>  May be T

<220>
<221>  VARIANT
<222>  (54)..(54)
<223>  May be V

<220>
<221>  VARIANT
<222>  (55)..(55)
<223>  May be G or N

<220>
<221>  VARIANT
<222>  (56)..(56)
<223>  May be T or V

<220>
<221>  misc_feature
<222>  (60)..(60)
<223>  Xaa can be any naturally occurring amino acid

<220>
<221>  misc_feature
<222>  (62)..(64)
<223>  Xaa can be any naturally occurring amino acid

<220>
<221>  misc_feature
<222>  (67)..(69)
<223>  Xaa can be any naturally occurring amino acid

<220>
<221>  VARIANT
<222>  (70)..(70)
<223>  May be I

<220>
<221>  VARIANT
<222>  (71)..(71)
<223>  May be V or A

<220>
<221>  VARIANT
<222>  (72)..(72)
<223>  May be S

<220>
<221>  VARIANT
<222>  (74)..(74)
<223>  May be K or S

<220>
<221>  misc_feature
<222>  (75)..(75)
<223>  Xaa can be any naturally occurring amino acid

<220>
<221>  VARIANT
<222>  (77)..(77)
<223>  May be E or Q

<220>
<221>  misc_feature
<222>  (78)..(78)
<223>  Xaa can be any naturally occurring amino acid

<220>
<221>  VARIANT

<222>  (79)..(79)
<223>  May be I or V

<220>
<221>  VARIANT
<222>  (80)..(80)
<223>  May be E or T

<220>
<221>  VARIANT
<222>  (82)..(82)
<223>  May be Q

<220>
<221>  VARIANT
<222>  (85)..(85)
<223>  May be L

<220>
<221>  VARIANT
<222>  (87)..(87)
<223>  May be A or N

<220>
<221>  VARIANT
<222>  (95)..(95)
<223>  May be Y

<220>
<221>  VARIANT
<222>  (96)..(96)
<223>  May be A or T

<220>
<221>  VARIANT
<222>  (97)..(97)
<223>  May be W

<220>
<221>  VARIANT
<222>  (98)..(98)
<223>  May be R

<220>
<221>  VARIANT
<222>  (102)..(102)
<223>  May be N

<220>
<221>  misc_feature
<222>  (104)..(104)
<223>  Xaa can be any naturally occurring amino acid

<220>
<221>  VARIANT
<222>  (105)..(105)
<223>  May be L

<220>
<221>  VARIANT
<222>  (107)..(107)
<223>  May be N or S

<220>
<221>  VARIANT
<222>  (108)..(108)
<223>  May be L

<220>
<221>  VARIANT
<222>  (109)..(109)
<223>  May be L

<220>
<221>  VARIANT
<222>  (110)..(110)
<223>  May be I or T

<220>
<221>  VARIANT
<222>  (111)..(111)
<223>  May be T or Q

<220>
<221>  VARIANT
<222>  (112)..(112)
<223>  May be P

<220>
<221>  misc_feature
<222>  (113)..(113)
<223>  Xaa can be any naturally occurring amino acid

<220>
<221>  VARIANT
<222>  (114)..(114)
<223>  May be Y

<220>
<221>  misc_feature
<222>  (115)..(115)
<223>  Xaa can be any naturally occurring amino acid

<220>
<221>  VARIANT
<222>  (117)..(117)
<223>  May be T

<220>
<221>  misc_feature
<222>  (118)..(119)
<223>  Xaa can be any naturally occurring amino acid

<220>
<221>  misc_feature
<222>  (122)..(122)
<223>  Xaa can be any naturally occurring amino acid

<220>
<221>  misc_feature
<222>  (126)..(126)
<223>  Xaa can be any naturally occurring amino acid

<220>
<221>  misc_feature
<222>  (129)..(130)

<223>  Xaa can be any naturally occurring amino acid

<220>
<221>  misc_feature
<222>  (132)..(132)
<223>  Xaa can be any naturally occurring amino acid

<220>
<221>  misc_feature
<222>  (134)..(134)
<223>  Xaa can be any naturally occurring amino acid

<220>
<221>  VARIANT
<222>  (136)..(136)
<223>  May be K

<220>
<221>  VARIANT
<222>  (137)..(137)
<223>  May be D or E

<220>
<221>  misc_feature
<222>  (139)..(139)
<223>  Xaa can be any naturally occurring amino acid

<220>
<221>  VARIANT
<222>  (141)..(141)
<223>  May be Q

<220>
<221>  misc_feature
<222>  (142)..(142)
<223>  Xaa can be any naturally occurring amino acid

<220>
<221>  misc_feature
<222>  (144)..(144)
<223>  Xaa can be any naturally occurring amino acid

<220>
<221>  VARIANT
<222>  (145)..(145)
<223>  May be Y

<220>
<221>  VARIANT
<222>  (146)..(146)
<223>  May be S

<220>
<221>  VARIANT
<222>  (147)..(147)
<223>  May be S or G

<220>
<221>  misc_feature
<222>  (150)..(153)
<223>  Xaa can be any naturally occurring amino acid

<220>

<221>  VARIANT
<222>  (157)..(157)
<223>  May be Y or H

<220>
<221>  VARIANT
<222>  (159)..(159)
<223>  May be K

<220>
<221>  VARIANT
<222>  (163)..(163)
<223>  May be T or A

<220>
<221>  misc_feature
<222>  (165)..(166)
<223>  Xaa can be any naturally occurring amino acid

<220>
<221>  VARIANT
<222>  (167)..(167)
<223>  May be F

<220>
<221>  misc_feature
<222>  (168)..(174)
<223>  Xaa can be any naturally occurring amino acid

<220>
<221>  misc_feature
<222>  (176)..(177)
<223>  Xaa can be any naturally occurring amino acid

<220>
<221>  VARIANT
<222>  (180)..(180)
<223>  May be G

<220>
<221>  VARIANT
<222>  (181)..(181)
<223>  May be S

<220>
<221>  misc_feature
<222>  (182)..(183)
<223>  Xaa can be any naturally occurring amino acid

<220>
<221>  VARIANT
<222>  (184)..(184)
<223>  May be G

<220>
<221>  misc_feature
<222>  (187)..(187)
<223>  Xaa can be any naturally occurring amino acid

<220>
<221>  VARIANT
<222>  (189)..(189)
<223>  May be S

<220>
<221>  VARIANT
<222>  (191)..(191)
<223>  May be S

<220>
<221>  VARIANT
<222>  (194)..(194)
<223>  May be F

<220>
<221>  VARIANT
<222>  (195)..(195)
<223>  May be P or K

<220>
<221>  VARIANT
<222>  (197)..(197)
<223>  May be T or P

<220>
<221>  misc_feature
<222>  (198)..(198)
<223>  Xaa can be any naturally occurring amino acid

<220>
<221>  VARIANT
<222>  (199)..(199)
<223>  May be R

<220>
<221>  VARIANT
<222>  (200)..(200)
<223>  May be I

<220>
<221>  VARIANT
<222>  (202)..(202)
<223>  May be I

<400>  2

His Gly Tyr Leu Xaa Xaa Xaa Xaa Xaa Xaa Thr Arg Val Gly Xaa Glu
1               5                   10                  15

Ala Gly Xaa Asp Ser Cys Pro Glu Cys Xaa Ile Leu Glu Pro Val Xaa
            20                  25                  30

Ala Trp Pro Asp Leu Thr Xaa Ala Xaa Val Gly Arg Ser Gly Pro Cys
        35                  40                  45

Gly Tyr Asn Ala Arg Asp Ser Ile Asp Tyr Asn Xaa Pro Xaa Xaa Xaa
    50                  55                  60

Trp Gly Xaa Xaa Xaa Val Ile Thr Tyr Thr Xaa Gly Asp Xaa Ala Asp
65                  70                  75                  80

Val Glu Trp Cys Val Asp His Asn Gly Asp His Gly Gly Met Phe Ser
                85                  90                  95

Tyr Gly Ile Cys Gln Asp Gln Xaa Ile Val Asp Lys Phe Leu Asp Ala
            100                 105                 110

Xaa His Xaa Pro Ser Xaa Xaa Glu Lys Xaa Ala Ala Glu Xaa Cys Phe
        115                 120                 125

Xaa Xaa Gly Xaa Leu Xaa Cys Thr Gly Val Xaa Gly Ala Xaa Cys Xaa
    130                 135                 140

Phe Asn Pro Asp Cys Xaa Xaa Xaa Xaa Pro Ala Cys Trp Arg Asn Asp
145                 150                 155                 160

Trp Phe Ser Cys Xaa Xaa Trp Xaa Xaa Xaa Xaa Xaa Xaa Xaa Cys Xaa
                165                 170                 175

Xaa Val Asp Asn Ala Xaa Xaa Asn Ser Cys Xaa Thr Thr Ile Ala Gly
            180                 185                 190

Gly Tyr Thr Val Ser Xaa Lys Val Lys Leu Pro
        195                 200

<210>  3
<211>  207
<212>  PRT
<213>  Artificial Sequence

<220>
<223>  consensus starch active protein long

<220>
<221>  VARIANT
<222>  (4)..(4)
<223>  May be M

<220>
<221>  misc_feature
<222>  (5)..(14)
<223>  Xaa can be any naturally occurring amino acid

<220>
<221>  VARIANT
<222>  (16)..(16)
<223>  May be G or K

<220>
<221>  VARIANT
<222>  (17)..(17)
<223>  May be I or L

<220>
<221>  VARIANT

<222>  (18)..(18)
<223>  May be N

<220>
<221>  misc_feature
<222>  (19)..(19)
<223>  Xaa can be any naturally occurring amino acid

<220>
<221>  VARIANT
<222>  (20)..(20)
<223>  May be Q or N

<220>
<221>  VARIANT
<222>  (21)..(21)
<223>  May be S

<220>
<221>  misc_feature
<222>  (23)..(23)
<223>  Xaa can be any naturally occurring amino acid

<220>
<221>  VARIANT
<222>  (25)..(25)
<223>  May be T

<220>
<221>  misc_feature
<222>  (30)..(30)
<223>  Xaa can be any naturally occurring amino acid

<220>
<221>  VARIANT
<222>  (32)..(32)
<223>  May be R

<220>
<221>  misc_feature
<222>  (36)..(36)
<223>  Xaa can be any naturally occurring amino acid

<220>
<221>  VARIANT
<222>  (37)..(37)
<223>  May be S

<220>
<221>  VARIANT
<222>  (38)..(38)
<223>  May be N, S or T

<220>
<221>  VARIANT
<222>  (41)..(41)
<223>  May be V

<220>
<221>  VARIANT
<222>  (42)..(42)
<223>  May be E or D

<220>
<221> misc_feature
<222> (43)..(43)
<223> Xaa can be any naturally occurring amino acid

<220>
<221> misc_feature
<222> (45)..(45)
<223> Xaa can be any naturally occurring amino acid

<220>
<221> VARIANT
<222> (49)..(49)
<223> May be T

<220>
<221> VARIANT
<222> (58)..(58)
<223> May be V

<220>
<221> VARIANT
<222> (59)..(59)
<223> May be G or N

<220>
<221> VARIANT
<222> (60)..(60)
<223> May be T or V

<220>
<221> misc_feature
<222> (64)..(64)
<223> Xaa can be any naturally occurring amino acid

<220>
<221> misc_feature
<222> (66)..(68)
<223> Xaa can be any naturally occurring amino acid

<220>
<221> misc_feature
<222> (71)..(73)
<223> Xaa can be any naturally occurring amino acid

<220>
<221> VARIANT
<222> (74)..(74)
<223> May be I

<220>
<221> VARIANT
<222> (75)..(75)
<223> May be V or A

<220>
<221> VARIANT
<222> (76)..(76)
<223> May be S

<220>
<221> VARIANT
<222> (78)..(78)

<223>  May be K or S

<220>
<221>  misc_feature
<222>  (79)..(79)
<223>  Xaa can be any naturally occurring amino acid

<220>
<221>  VARIANT
<222>  (81)..(81)
<223>  May be E or Q

<220>
<221>  misc_feature
<222>  (82)..(82)
<223>  Xaa can be any naturally occurring amino acid

<220>
<221>  VARIANT
<222>  (83)..(83)
<223>  May be I or V

<220>
<221>  VARIANT
<222>  (84)..(84)
<223>  May be E or Q

<220>
<221>  VARIANT
<222>  (86)..(86)
<223>  May be Q

<220>
<221>  VARIANT
<222>  (89)..(89)
<223>  May be L

<220>
<221>  VARIANT
<222>  (91)..(91)
<223>  May be A or N

<220>
<221>  VARIANT
<222>  (99)..(99)
<223>  May be Y

<220>
<221>  VARIANT
<222>  (100)..(100)
<223>  May be A or T

<220>
<221>  VARIANT
<222>  (101)..(101)
<223>  May be W

<220>
<221>  VARIANT
<222>  (102)..(102)
<223>  May be R

<220>

<221>  VARIANT
<222>  (106)..(106)
<223>  May be N

<220>
<221>  misc_feature
<222>  (108)..(108)
<223>  Xaa can be any naturally occurring amino acid

<220>
<221>  VARIANT
<222>  (109)..(109)
<223>  May be L

<220>
<221>  VARIANT
<222>  (111)..(111)
<223>  May be N or S

<220>
<221>  VARIANT
<222>  (112)..(112)
<223>  May be L

<220>
<221>  VARIANT
<222>  (113)..(113)
<223>  May be L

<220>
<221>  VARIANT
<222>  (114)..(114)
<223>  May be I or T

<220>
<221>  VARIANT
<222>  (115)..(115)
<223>  May be T or Q

<220>
<221>  VARIANT
<222>  (116)..(116)
<223>  May be P

<220>
<221>  misc_feature
<222>  (117)..(117)
<223>  Xaa can be any naturally occurring amino acid

<220>
<221>  VARIANT
<222>  (118)..(118)
<223>  May be Y

<220>
<221>  misc_feature
<222>  (119)..(119)
<223>  Xaa can be any naturally occurring amino acid

<220>
<221>  VARIANT
<222>  (121)..(121)
<223>  May be T

<220>
<221>  misc_feature
<222>  (122)..(123)
<223>  Xaa can be any naturally occurring amino acid

<220>
<221>  misc_feature
<222>  (126)..(126)
<223>  Xaa can be any naturally occurring amino acid

<220>
<221>  misc_feature
<222>  (130)..(130)
<223>  Xaa can be any naturally occurring amino acid

<220>
<221>  misc_feature
<222>  (133)..(134)
<223>  Xaa can be any naturally occurring amino acid

<220>
<221>  misc_feature
<222>  (136)..(136)
<223>  Xaa can be any naturally occurring amino acid

<220>
<221>  misc_feature
<222>  (138)..(138)
<223>  Xaa can be any naturally occurring amino acid

<220>
<221>  VARIANT
<222>  (140)..(140)
<223>  May be K

<220>
<221>  VARIANT
<222>  (141)..(141)
<223>  May be D or E

<220>
<221>  misc_feature
<222>  (143)..(143)
<223>  Xaa can be any naturally occurring amino acid

<220>
<221>  VARIANT
<222>  (145)..(145)
<223>  May be Q

<220>
<221>  misc_feature
<222>  (146)..(146)
<223>  Xaa can be any naturally occurring amino acid

<220>
<221>  misc_feature
<222>  (148)..(148)
<223>  Xaa can be any naturally occurring amino acid

<220>
<221>  VARIANT

<222>  (149)..(149)
<223>  May be Y

<220>
<221>  VARIANT
<222>  (150)..(150)
<223>  May be S

<220>
<221>  VARIANT
<222>  (151)..(151)
<223>  May be S or G

<220>
<221>  misc_feature
<222>  (154)..(157)
<223>  Xaa can be any naturally occurring amino acid

<220>
<221>  VARIANT
<222>  (161)..(161)
<223>  May be Y or H

<220>
<221>  VARIANT
<222>  (163)..(163)
<223>  May be K

<220>
<221>  VARIANT
<222>  (167)..(167)
<223>  May be T or A

<220>
<221>  misc_feature
<222>  (169)..(170)
<223>  Xaa can be any naturally occurring amino acid

<220>
<221>  VARIANT
<222>  (171)..(171)
<223>  May be F

<220>
<221>  misc_feature
<222>  (172)..(178)
<223>  Xaa can be any naturally occurring amino acid

<220>
<221>  misc_feature
<222>  (180)..(181)
<223>  Xaa can be any naturally occurring amino acid

<220>
<221>  VARIANT
<222>  (184)..(184)
<223>  May be G

<220>
<221>  VARIANT
<222>  (185)..(185)
<223>  May be S

<220>
<221>  misc_feature
<222>  (186)..(187)
<223>  Xaa can be any naturally occurring amino acid

<220>
<221>  VARIANT
<222>  (188)..(188)
<223>  May be G

<220>
<221>  misc_feature
<222>  (191)..(191)
<223>  Xaa can be any naturally occurring amino acid

<220>
<221>  VARIANT
<222>  (193)..(193)
<223>  May be S

<220>
<221>  VARIANT
<222>  (195)..(195)
<223>  May be S

<220>
<221>  VARIANT
<222>  (198)..(198)
<223>  May be F

<220>
<221>  VARIANT
<222>  (199)..(199)
<223>  May be P or K

<220>
<221>  VARIANT
<222>  (201)..(201)
<223>  May be T or P

<220>
<221>  misc_feature
<222>  (202)..(202)
<223>  Xaa can be any naturally occurring amino acid

<220>
<221>  VARIANT
<222>  (203)..(203)
<223>  May be R

<220>
<221>  VARIANT
<222>  (204)..(204)
<223>  May be R

<220>
<221>  VARIANT
<222>  (206)..(206)
<223>  May be R

<400>  3

His Gly Tyr Leu Xaa Xaa Xaa Xaa Xaa Xaa Xaa Xaa Xaa Xaa Thr Arg

1               5                   10                  15

Val Gly Xaa Glu Ala Gly Xaa Asp Ser Cys Pro Glu Cys Xaa Ile Leu
            20                  25                  30

Glu Pro Val Xaa Ala Trp Pro Asp Leu Thr Xaa Ala Xaa Val Gly Arg
        35                  40                  45

Ser Gly Pro Cys Gly Tyr Asn Ala Arg Asp Ser Ile Asp Tyr Asn Xaa
    50                  55                  60

Pro Xaa Xaa Xaa Trp Gly Xaa Xaa Xaa Val Ile Thr Tyr Thr Xaa Gly
65                  70                  75                  80

Asp Xaa Ala Asp Val Glu Trp Cys Val Asp His Asn Gly Asp His Gly
                85                  90                  95

Gly Met Phe Ser Tyr Gly Ile Cys Gln Asp Gln Xaa Ile Val Asp Lys
            100                 105                 110

Phe Leu Asp Ala Xaa His Xaa Pro Ser Xaa Xaa Glu Lys Xaa Ala Ala
        115                 120                 125

Glu Xaa Cys Phe Xaa Xaa Gly Xaa Leu Xaa Cys Thr Gly Val Xaa Gly
    130                 135                 140

Ala Xaa Cys Xaa Phe Asn Pro Asp Cys Xaa Xaa Xaa Xaa Pro Ala Cys
145                 150                 155                 160

Trp Arg Asn Asp Trp Phe Ser Cys Xaa Xaa Trp Xaa Xaa Xaa Xaa Xaa
                165                 170                 175

Xaa Xaa Cys Xaa Xaa Val Asp Asn Ala Xaa Xaa Asn Ser Cys Xaa Thr
            180                 185                 190

Thr Ile Ala Gly Gly Tyr Thr Val Ser Xaa Lys Val Lys Leu Pro
        195                 200                 205

<210>  4
<211>  232
<212>  PRT
<213>  Aspergillus terreus

<400>  4

His Gly Tyr Leu Thr Val Pro Ala Ser Arg Thr Arg Leu Gly Phe Glu
1               5                   10                  15

Ala Gly Ile Asp Thr Cys Pro Glu Cys Ser Ile Leu Glu Pro Val Ser

20                  25                  30

Ala Trp Pro Asp Leu Thr Ala Ala Gln Val Gly Arg Ser Gly Pro Cys
        35                  40                  45

Gly Tyr Asn Ala Arg Val Ser Val Asp Tyr Asn Gln Pro Gly Asp Tyr
    50                  55                  60

Trp Gly Asn Glu Pro Val Val Ser Tyr Thr Ala Gly Asp Val Val Glu
65                  70                  75                  80

Val Gln Trp Cys Val Asp His Asn Gly Asp His Gly Gly Met Phe Thr
                85                  90                  95

Tyr Gly Ile Cys Gln Asn Gln Thr Leu Val Asp Leu Phe Leu Thr Pro
            100                 105                 110

Gly Tyr Leu Pro Thr Asn Glu Glu Lys Gln Ala Ala Glu Asp Cys Phe
        115                 120                 125

Leu Glu Gly Glu Leu Ser Cys Leu His Val Pro Gly Gln Thr Cys Asn
    130                 135                 140

Tyr Asn Pro Asp Cys Ser Ala Gly Glu Pro Cys Tyr Gln Asn Asp Trp
145                 150                 155                 160

Phe Thr Cys Asn Ala Phe Gln Ala Asp Asn Asn Arg Ala Cys Gln Gly
                165                 170                 175

Val Asp Gly Ala Ala Leu Asn Ser Cys Met Thr Thr Ile Ala Gly Gly
            180                 185                 190

Tyr Thr Val Thr Lys Lys Ile Lys Ile Pro Asp Tyr Ser Ser Ser His
        195                 200                 205

Thr Leu Leu Arg Phe Arg Trp Asn Ser Phe Gln Thr Ala Gln Val Tyr
    210                 215                 220

Leu His Cys Ala Asp Ile Ala Ile
225                 230

<210>  5
<211>  251
<212>  PRT
<213>  Aspergillus terreus

<400>  5

Met Lys Val Phe Ala Pro Leu Leu Ser Leu Ser Leu Ala Thr Ser Val

1               5                   10                  15

Ala Gly His Gly Tyr Met Tyr Ile Pro Ser Ser Arg Thr Arg Leu Gly
            20                  25                  30

His Glu Ala Gly Ile Asp Ser Cys Pro Glu Cys Ala Ile Leu Glu Pro
        35                  40                  45

Val Ser Ser Trp Pro Asp Leu Asp Ala Ala Pro Val Gly Arg Ser Gly
    50                  55                  60

Pro Cys Gly Tyr Asn Ala Arg Asp Ser Ile Asp Tyr Asn Gln Pro Thr
65                  70                  75                  80

Thr Asn Trp Gly Ser Asp Ala Val Gln Ser Tyr Ser Pro Gly Glu Glu
                85                  90                  95

Ile Glu Val Gln Trp Cys Val Asp His Asn Gly Asp His Gly Gly Met
            100                 105                 110

Phe Thr Tyr Arg Ile Cys Gln Asp Gln Ser Ile Val Asp Lys Phe Leu
        115                 120                 125

Asp Pro Ser Tyr Leu Pro Thr Asn Asp Glu Lys Gln Ala Ala Glu Asp
    130                 135                 140

Cys Phe Asp Ala Gly Leu Leu Pro Cys Thr Asp Val Ser Gly Gln Glu
145                 150                 155                 160

Cys Gly Tyr Ser Ala Asp Cys Thr Glu Gly Glu Ala Cys Trp Arg Asn
                165                 170                 175

Asp Trp Phe Thr Cys Asn Gly Phe Glu Ala Ser Asp Arg Pro Lys Cys
            180                 185                 190

Gln Gly Val Asp Asn Ala Glu Leu Asn Ser Cys Tyr Thr Ser Ile Ala
        195                 200                 205

Gly Gly Tyr Thr Val Thr Lys Lys Val Lys Leu Pro Glu Tyr Thr Ser
    210                 215                 220

Asn His Thr Leu Ile Ser Phe Lys Trp Asn Ser Phe Gln Thr Gly Gln
225                 230                 235                 240

Ile Tyr Leu Ser Cys Ala Asp Ile Ala Ile Gln
                245                 250

<210>  6
<211>  251
<212>  PRT
<213>  Artificial Sequence

<220>
<223>  curated protein based on ZY029436 - no CBM20

<400>  6

Met Lys Val Phe Ala Pro Leu Leu Ser Leu Ser Leu Ala Thr Ser Val
1               5                   10                  15

Ala Gly His Gly Tyr Met Tyr Ile Pro Ser Ser Arg Thr Arg Leu Gly
            20                  25                  30

His Glu Ala Gly Ile Asp Ser Cys Pro Glu Cys Ala Ile Leu Glu Pro
        35                  40                  45

Val Ser Ser Trp Pro Asp Leu Asp Ala Ala Pro Val Gly Arg Ser Gly
    50                  55                  60

Pro Cys Gly Tyr Asn Ala Arg Asp Ser Ile Asp Tyr Asn Gln Pro Thr
65                  70                  75                  80

Thr Asn Trp Gly Ser Asp Ala Val Gln Ser Tyr Ser Pro Gly Glu Glu
                85                  90                  95

Ile Glu Val Gln Trp Cys Val Asp His Asn Gly Asp His Gly Gly Met
            100                 105                 110

Phe Thr Tyr Arg Ile Cys Gln Asp Gln Ser Ile Val Asp Lys Phe Leu
        115                 120                 125

Asp Pro Ser Tyr Leu Pro Thr Asn Asp Glu Lys Gln Ala Ala Glu Asp
    130                 135                 140

Cys Phe Asp Ala Gly Leu Leu Pro Cys Thr Asp Val Ser Gly Gln Glu
145                 150                 155                 160

Cys Gly Tyr Ser Ala Asp Cys Thr Glu Gly Glu Ala Cys Trp Arg Asn
                165                 170                 175

Asp Trp Phe Thr Cys Asn Gly Phe Glu Ala Ser Asp Arg Pro Lys Cys
            180                 185                 190

Gln Gly Val Asp Asn Ala Glu Leu Asn Ser Cys Tyr Thr Ser Ile Ala
        195                 200                 205

Gly Gly Tyr Thr Val Thr Lys Lys Val Lys Leu Pro Glu Tyr Thr Ser

210                 215                 220

Asn His Thr Leu Ile Ser Phe Lys Trp Asn Ser Phe Gln Thr Gly Gln
225                 230                 235                 240

Ile Tyr Leu Ser Cys Ala Asp Ile Ala Ile Gln
                245                 250

<210>  7
<211>  400
<212>  PRT
<213>  Artificial Sequence

<220>
<223>  curated protein based on ZY029436 - with CBM20

<400>  7

Met Lys Val Phe Ala Pro Leu Leu Ser Leu Ser Leu Ala Thr Ser Val
1               5                   10                  15

Ala Gly His Gly Tyr Met Tyr Ile Pro Ser Ser Arg Thr Arg Leu Gly
            20                  25                  30

His Glu Ala Gly Ile Asp Ser Cys Pro Glu Cys Ala Ile Leu Glu Pro
        35                  40                  45

Val Ser Ser Trp Pro Asp Leu Asp Ala Ala Pro Val Gly Arg Ser Gly
    50                  55                  60

Pro Cys Gly Tyr Asn Ala Arg Asp Ser Ile Asp Tyr Asn Gln Pro Thr
65                  70                  75                  80

Thr Asn Trp Gly Ser Asp Ala Val Gln Ser Tyr Ser Pro Gly Glu Glu
                85                  90                  95

Ile Glu Val Gln Trp Cys Val Asp His Asn Gly Asp His Gly Gly Met
            100                 105                 110

Phe Thr Tyr Arg Ile Cys Gln Asp Gln Ser Ile Val Asp Lys Phe Leu
        115                 120                 125

Asp Pro Ser Tyr Leu Pro Thr Asn Asp Glu Lys Gln Ala Ala Glu Asp
    130                 135                 140

Cys Phe Asp Ala Gly Leu Leu Pro Cys Thr Asp Val Ser Gly Gln Glu
145                 150                 155                 160

Cys Gly Tyr Ser Ala Asp Cys Thr Glu Gly Glu Ala Cys Trp Arg Asn
                165                 170                 175

Asp Trp Phe Thr Cys Asn Gly Phe Glu Ala Ser Asp Arg Pro Lys Cys
            180                 185                 190

Gln Gly Val Asp Asn Ala Glu Leu Asn Ser Cys Tyr Thr Ser Ile Ala
        195                 200                 205

Gly Gly Tyr Thr Val Thr Lys Lys Val Lys Leu Pro Glu Tyr Thr Ser
    210                 215                 220

Asn His Thr Leu Ile Ser Phe Lys Trp Asn Ser Phe Gln Thr Gly Gln
225                 230                 235                 240

Ile Tyr Leu Ser Cys Ala Asp Ile Ala Ile Pro Ser Ile Val Ala Thr
                245                 250                 255

Gly Gly Thr Thr Thr Thr Ala Thr Pro Thr Gly Ser Gly Ser Val Thr
            260                 265                 270

Ser Thr Ser Lys Thr Thr Ala Thr Ala Ser Lys Thr Ser Thr Ser Thr
        275                 280                 285

Ser Ser Thr Ser Cys Thr Thr Pro Thr Ala Val Ala Val Thr Phe Asp
    290                 295                 300

Leu Thr Ala Thr Thr Thr Tyr Gly Glu Asn Ile Tyr Leu Val Gly Ser
305                 310                 315                 320

Ile Ser Gln Leu Gly Asp Trp Glu Thr Ser Asp Gly Ile Ala Leu Ser
                325                 330                 335

Ala Asp Lys Tyr Thr Ser Ser Asp Pro Leu Trp Tyr Val Thr Val Thr
            340                 345                 350

Leu Pro Ala Gly Glu Ser Phe Glu Tyr Lys Phe Ile Arg Ile Glu Ser
        355                 360                 365

Asp Asp Ser Val Glu Trp Glu Ser Asp Pro Asn Arg Glu Tyr Thr Val
    370                 375                 380

Pro Gln Ala Cys Gly Thr Ser Thr Ala Thr Val Thr Asp Thr Trp Arg
385                 390                 395                 400

<210> 8
<211> 385
<212> PRT
<213> Emericella nidulans

<400>  8

Met Lys Ser Leu Leu Ala Leu Val Ala Gly Asn Leu Val Thr Ala Val
1               5                   10                  15

Ser Gly His Gly Tyr Leu Thr Val Pro Ala Ser Arg Thr Arg Leu Gly
            20                  25                  30

Phe Glu Ala Gly Ile Asp Thr Cys Pro Glu Cys Ser Ile Leu Glu Pro
        35                  40                  45

Val Ser Ala Trp Pro Asp Leu Thr Ala Ala Gln Val Gly Arg Ser Gly
    50                  55                  60

Pro Cys Gly Tyr Asn Ala Arg Val Ser Val Asp Tyr Asn Gln Pro Gly
65                  70                  75                  80

Asp Tyr Trp Gly Asn Glu Pro Val Val Ser Tyr Thr Ala Gly Asp Val
                85                  90                  95

Val Glu Val Gln Trp Cys Val Asp His Asn Gly Asp His Gly Gly Met
            100                 105                 110

Phe Thr Tyr Gly Ile Cys Gln Asn Gln Thr Leu Val Asp Leu Phe Leu
        115                 120                 125

Thr Pro Gly Tyr Leu Pro Thr Asn Glu Glu Lys Gln Ala Ala Glu Asp
    130                 135                 140

Cys Phe Leu Glu Gly Glu Leu Ser Cys Leu His Val Pro Gly Gln Thr
145                 150                 155                 160

Cys Asn Tyr Asn Pro Asp Cys Ser Ala Gly Glu Pro Cys Tyr Gln Asn
                165                 170                 175

Asp Trp Phe Thr Cys Asn Ala Phe Gln Ala Asp Asn Asn Arg Ala Cys
            180                 185                 190

Gln Gly Val Asp Gly Ala Ala Leu Asn Ser Cys Met Thr Thr Ile Ala
        195                 200                 205

Gly Gly Tyr Thr Val Thr Lys Lys Ile Lys Ile Pro Asp Tyr Ser Ser
    210                 215                 220

Ser His Thr Leu Leu Arg Phe Arg Trp Asn Ser Phe Gln Thr Ala Gln
225                 230                 235                 240

Val Tyr Leu His Cys Ala Asp Ile Ala Ile Val Gly Gly Ser Gly Ser

245                 250                 255

Ser Pro Ser Pro Thr Ser Thr Thr Ser Thr Ala Thr Ser Thr Thr Thr
            260                 265                 270

Pro Ser Ser Thr Ser Cys Ala Ser Ala Ile Ser Ile Pro Val Thr Phe
        275                 280                 285

Asn Ala Leu Val Thr Thr Thr Tyr Gly Glu Asn Val Tyr Leu Ala Gly
    290                 295                 300

Ser Ile Ser Gln Leu Gly Ser Trp Ser Thr Ser Ser Ala Val Ala Leu
305                 310                 315                 320

Ser Ala Ser Lys Tyr Ser Ser Ser Ser Pro Leu Trp Thr Val Thr Val
                325                 330                 335

Asp Leu Pro Val Gly Ala Thr Phe Glu Tyr Lys Tyr Ile Lys Lys Glu
            340                 345                 350

Ser Asp Gly Ser Ile Val Trp Glu Ser Gly Pro Asn Arg Ser Tyr Thr
        355                 360                 365

Val Pro Thr Gly Cys Ser Gly Thr Thr Ala Thr Glu Ser Gly Ala Trp
    370                 375                 380

Arg
385

<210>  9
<211>  251
<212>  PRT
<213>  Artificial Sequence

<220>
<223>  curated protein from Emericella nidulans protein AN6103.2

<400>  9

Met Lys Ala Phe Phe Ala Ile Ser Ala Ser Thr Leu Leu Ala Thr Val
1               5                   10                  15

His Gly His Gly Tyr Leu Thr Val Pro Ala Ser Arg Thr Arg Leu Gly
            20                  25                  30

Phe Glu Ala Gly Ile Asp Thr Cys Pro Glu Cys Ser Ile Leu Glu Pro
        35                  40                  45

Val Asp Ala Trp Pro Asn Val Thr Glu Ala Gln Val Gly Arg Ser Gly
    50                  55                  60

Pro Cys Gly Tyr Asn Ala Arg Val Ser Val Asp Tyr Asn Gln Pro Gly
65                  70                  75                  80

Asp Asn Trp Gly Asn Glu Pro Val Val Thr Tyr Lys Ala Gly Asp Ile
                85                  90                  95

Val Glu Val Gln Trp Cys Val Asp Asn Asn Gly Asp His Gly Gly Met
            100                 105                 110

Phe Thr Tyr Gly Ile Cys Gln Asp Gln Glu Leu Val Asp Lys Phe Leu
        115                 120                 125

Asp Pro Asp Tyr Leu Pro Thr Glu Glu Glu Lys Gln Ala Ala Glu Asp
    130                 135                 140

Cys Phe Leu Gln Gly Glu Leu Lys Cys Gly Asp Val Asp Gly Gln Glu
145                 150                 155                 160

Cys Glu Tyr Ser Pro Asp Cys Gly Glu Gly Glu Ala Cys Tyr Arg Asn
                165                 170                 175

Asp Trp Phe Thr Cys Asn Ala Phe Glu Ala Asp Ser Asp Arg Gly Cys
            180                 185                 190

Gln Gly Val Asp Gly Ala Glu Leu Asn Ser Cys Lys Thr Thr Ile Ala
        195                 200                 205

Gly Gly Tyr Thr Val Thr Lys Lys Ile Lys Ile Pro Asp Tyr Thr Ser
    210                 215                 220

Glu His Thr Leu Leu Arg Phe Arg Trp Asn Ser Phe Gln Thr Pro Gln
225                 230                 235                 240

Ile Tyr Leu His Cys Ala Asp Pro Thr Ile Glu
                245                 250


<210>  10
<211>  385
<212>  PRT
<213>  Emericella nidulans

<400>  10

Met Lys Ser Leu Leu Ala Leu Val Ala Gly Asn Leu Val Thr Ala Val
1               5                   10                  15

Ser Gly His Gly Tyr Leu Thr Val Pro Ala Ser Arg Thr Arg Leu Gly
            20                  25                  30

Phe Glu Ala Gly Ile Asp Thr Cys Pro Glu Cys Ser Ile Leu Glu Pro
        35                  40                  45

Val Ser Ala Trp Pro Asp Leu Thr Ala Ala Gln Val Gly Arg Ser Gly
    50                  55                  60

Pro Cys Gly Tyr Asn Ala Arg Val Ser Val Asp Tyr Asn Gln Pro Gly
65                  70                  75                  80

Asp Tyr Trp Gly Asn Glu Pro Val Val Ser Tyr Thr Ala Gly Asp Val
                85                  90                  95

Val Glu Val Gln Trp Cys Val Asp His Asn Gly Asp His Gly Gly Met
            100                 105                 110

Phe Thr Tyr Gly Ile Cys Gln Asn Gln Thr Leu Val Asp Leu Phe Leu
        115                 120                 125

Thr Pro Gly Tyr Leu Pro Thr Asn Glu Glu Lys Gln Ala Ala Glu Asp
    130                 135                 140

Cys Phe Leu Glu Gly Glu Leu Ser Cys Leu His Val Pro Gly Gln Thr
145                 150                 155                 160

Cys Asn Tyr Asn Pro Asp Cys Ser Ala Gly Glu Pro Cys Tyr Gln Asn
                165                 170                 175

Asp Trp Phe Thr Cys Asn Ala Phe Gln Ala Asp Asn Asn Arg Ala Cys
            180                 185                 190

Gln Gly Val Asp Gly Ala Ala Leu Asn Ser Cys Met Thr Thr Ile Ala
        195                 200                 205

Gly Gly Tyr Thr Val Thr Lys Lys Ile Lys Ile Pro Asp Tyr Ser Ser
    210                 215                 220

Ser His Thr Leu Leu Arg Phe Arg Trp Asn Ser Phe Gln Thr Ala Gln
225                 230                 235                 240

Val Tyr Leu His Cys Ala Asp Ile Ala Ile Val Gly Gly Ser Gly Ser
                245                 250                 255

Ser Pro Ser Pro Thr Ser Thr Thr Ser Thr Ala Thr Ser Thr Thr Thr
            260                 265                 270

Pro Ser Ser Thr Ser Cys Ala Ser Ala Ile Ser Ile Pro Val Thr Phe

275                 280                 285

Asn Ala Leu Val Thr Thr Thr Tyr Gly Glu Asn Val Tyr Leu Ala Gly
    290                 295                 300

Ser Ile Ser Gln Leu Gly Ser Trp Ser Thr Ser Ser Ala Val Ala Leu
305                 310                 315                 320

Ser Ala Ser Lys Tyr Ser Ser Ser Ser Pro Leu Trp Thr Val Thr Val
                325                 330                 335

Asp Leu Pro Val Gly Ala Thr Phe Glu Tyr Lys Tyr Ile Lys Lys Glu
            340                 345                 350

Ser Asp Gly Ser Ile Val Trp Glu Ser Gly Pro Asn Arg Ser Tyr Thr
        355                 360                 365

Val Pro Thr Gly Cys Ser Gly Thr Thr Ala Thr Glu Ser Gly Ala Trp
    370                 375                 380

Arg
385

<210>  11
<211>  400
<212>  PRT
<213>  Aspergillus terreus

<400>  11

Met Leu Leu Thr Val Leu Ala Val Val Gly Cys Phe Thr Ala Val Asn
1               5                   10                  15

Gly His Gly Tyr Leu Thr Ile Pro Ala Ser Arg Thr Arg Leu Gly Phe
            20                  25                  30

Glu Thr Gly Ile Asp Thr Cys Pro Glu Cys Ser Ile Leu Glu Pro Val
        35                  40                  45

Thr Ala Trp Pro Asp Leu Glu Ala Ala Gln Val Gly Arg Ser Gly Pro
    50                  55                  60

Cys Gly Tyr Asn Ala Arg Val Ser Val Asp Tyr Asn Gln Pro Ser Glu
65                  70                  75                  80

Tyr Trp Gly Asn Glu Pro Val Val Thr Tyr Thr Ser Gly Glu Val Val
                85                  90                  95

Glu Val Gln Trp Cys Val Asp Ala Asn Gly Asp His Gly Gly Met Phe

            100                 105                 110

Thr Tyr Gly Ile Cys Gln Asn Gln Thr Leu Val Asp Lys Phe Leu Thr
        115                 120                 125

Pro Gly Tyr Leu Pro Thr Asn Glu Glu Lys Gln Ala Ala Glu Asp Cys
    130                 135                 140

Phe Leu Asp Gly Glu Leu Lys Cys Lys Asp Val Ser Gly Gln Thr Cys
145                 150                 155                 160

Gly Tyr Asn Pro Asp Cys Thr Glu Gly Ala Ala Cys Trp Arg Asn Asp
                165                 170                 175

Trp Phe Thr Cys Asn Ala Phe Gln Ala Asn Thr Ala Arg Ala Cys Gln
            180                 185                 190

Gly Val Asp Gly Ala Ser Leu Asn Ser Cys Lys Thr Thr Ile Ala Gly
        195                 200                 205

Gly Tyr Thr Val Thr Lys Arg Ile Lys Ile Pro Asp Tyr Ser Ser Asp
    210                 215                 220

His Thr Leu Leu Arg Phe Arg Trp Asn Ser Phe Gln Thr Ala Gln Val
225                 230                 235                 240

Tyr Leu His Cys Ala Asp Ile Ala Ile Ala Gly Ser Gly Gly Gly Thr
                245                 250                 255

Thr Ser Lys Ser Thr Thr Ser Thr Thr Ser Thr Thr Ser Thr Ser Arg
            260                 265                 270

Ser Thr Ser Thr Ser Ala Pro Thr Thr Thr Ser Ser Ala Ser Thr Ala
        275                 280                 285

Thr Pro Ile Cys Thr Thr Gln Ala Ser Leu Ile Pro Val Thr Phe Gln
    290                 295                 300

Glu Phe Val Thr Thr Met Trp Gly Glu Asn Val Phe Val Thr Gly Ser
305                 310                 315                 320

Ile Ser Gln Leu Gly Ser Trp Ser Thr Asp Lys Ala Val Ala Leu Ser
                325                 330                 335

Ala Thr Gly Tyr Thr Ala Ser Asn Pro Leu Trp Thr Thr Thr Ile Asp
            340                 345                 350

Leu Pro Ala Gly Thr Thr Phe Glu Tyr Lys Phe Ile Lys Lys Glu Thr
        355                 360                 365

Asp Gly Ser Ile Ile Trp Glu Ser Asp Pro Asn Arg Ser Tyr Thr Val
    370                 375                 380

Pro Thr Gly Cys Ser Gly Thr Thr Ala Thr Ala Ala Ala Ser Trp Arg
385                 390                 395                 400

<210>  12
<211>  381
<212>  PRT
<213>  Neosartorya fischeri

<400>  12

Met Ile Phe Ser Ile Leu Val Ala Ala Gly Cys Phe Ala Ser Ala Tyr
1               5                   10                  15

Gly His Gly Tyr Leu Thr Ile Pro Ala Ser Arg Thr Arg Leu Gly Phe
            20                  25                  30

Glu Ala Gly Ile Asp Thr Cys Pro Glu Cys Ser Ile Leu Glu Pro Val
        35                  40                  45

Thr Ala Trp Pro Asp Leu Glu Glu Ala Gln Val Gly Arg Ser Gly Pro
    50                  55                  60

Cys Gly Tyr Asn Ala Arg Val Ser Val Asp Tyr Asn Gln Pro Ser Asp
65                  70                  75                  80

His Trp Gly Asn Glu Pro Val Val Thr Tyr Thr Ser Gly Glu Val Val
                85                  90                  95

Glu Val Gln Trp Cys Val Asp Ala Asn Gly Asp His Gly Gly Met Phe
            100                 105                 110

Thr Tyr Gly Ile Cys Gln Asn Gln Thr Leu Val Asp Lys Phe Leu Thr
        115                 120                 125

Pro Gly Tyr Leu Pro Thr His Gly Glu Lys Gln Ala Ala Glu Asp Cys
    130                 135                 140

Phe Leu Glu Gly Glu Leu Lys Cys Thr Asp Val Ser Gly Gln Thr Cys
145                 150                 155                 160

Gly Tyr Asn Pro Asp Cys Thr Glu Gly Ala Ala Cys Trp Arg Asn Asp
                165                 170                 175

Trp Phe Thr Cys Asn Ala Phe Gln Ala Asn Thr Ala Arg Ala Cys Gln
            180                 185                 190

Gly Val Asp Gly Ala Pro Leu Asn Ser Cys Lys Thr Thr Ile Ala Gly
        195                 200                 205

Gly Tyr Thr Val Thr Lys Arg Ile Lys Ile Pro Asn Tyr Ser Ser Asn
    210                 215                 220

His Thr Leu Leu Arg Phe Arg Trp Asn Ser Phe Gln Thr Ala Gln Val
225                 230                 235                 240

Tyr Leu His Cys Ala Asp Ile Ala Ile Ser Gly Ser Gly Ser Thr Thr
                245                 250                 255

Thr Ser Thr Thr Thr Ser Thr Thr Ser Thr Ser Thr Ser Thr Ser Thr
            260                 265                 270

Cys Thr Ala Thr Ala Ser Leu Ile Pro Val Thr Phe Asn Glu Leu Val
        275                 280                 285

Thr Thr Thr Tyr Gly Glu Asn Val Phe Ile Thr Gly Ser Ile Ser Gln
    290                 295                 300

Leu Gly Ser Trp Ser Thr Asp Asn Ala Val Ala Leu Ser Ala Ser Arg
305                 310                 315                 320

Tyr Thr Thr Ser Asn Pro Leu Trp Ile Thr Thr Ile Asn Leu Pro Ala
                325                 330                 335

Gly Thr Thr Phe Gln Tyr Lys Phe Ile Lys Lys Glu Thr Asp Gly Ser
            340                 345                 350

Val Ile Trp Glu Ser Asp Pro Asn Arg Ser Tyr Thr Val Pro Thr Gly
        355                 360                 365

Cys Ser Gly Thr Thr Ala Thr Ala Thr Ala Ser Trp Arg
    370                 375                 380

<210> 13
<211> 435
<212> PRT
<213> Penicillium chrysogenum

<400> 13

Met Gly Pro Trp Thr Lys Val Ser Asn Ala Ile Ile Arg Ser Asp Asn
1               5                   10                  15

Gln Ala Asp Asp Asn Ala Ala Met Asn Gly Cys Ala Glu Gln Val Thr
            20                  25                  30

Leu Ile Ser Gly Arg Leu Arg Glu Asn Lys Thr Thr Ile Arg Met Lys
        35                  40                  45

Ala Ser Ser Ile Leu Ser Leu Ala Gly Leu Leu Ser Ser Val Asn Ala
    50                  55                  60

His Gly Tyr Leu Thr Ile Pro Ser Ser Arg Thr Arg Leu Gly Phe Glu
65                  70                  75                  80

Ala Gly Ile Asp Thr Cys Pro Glu Cys Ser Ile Leu Glu Pro Val Ser
                85                  90                  95

Ala Trp Pro Asp Leu Glu Glu Ala Gln Val Gly Arg Ser Gly Pro Cys
            100                 105                 110

Gly Tyr Asn Ala Arg Val Gly Val Asp Tyr Asn Gln Pro Ala Ala His
        115                 120                 125

Trp Gly Asn Ser Val Val Ala Thr Tyr Thr Gly Asn Gln Ile Val Glu
    130                 135                 140

Val Glu Trp Cys Val Asp Asn Asn Gly Asp His Gly Gly Met Phe Thr
145                 150                 155                 160

Tyr Gly Ile Cys Gln Asn Gln Thr Leu Val Asp Leu Phe Leu Asp Pro
                165                 170                 175

Asn Tyr Leu Pro Thr Asn Ala Glu Lys Gln Ala Ala Glu Asp Cys Phe
            180                 185                 190

Leu Glu Gly Glu Leu Lys Cys Thr Asp Val Pro Gly Gln Thr Cys Gly
        195                 200                 205

Tyr Ser Pro Asp Cys Thr Ser Asp Gln Pro Cys Trp Arg Thr Asp Trp
    210                 215                 220

Phe Thr Cys Asn Ala Phe Asn Ala Gly Ser Arg Arg Gly Cys Gln Gly
225                 230                 235                 240

Val Asp Gly Ala Pro Leu Asn Ser Cys Lys Thr Thr Ile Ala Gly Gly
                245                 250                 255

Tyr Thr Ile Thr Lys Lys Ile Lys Ile Pro Asp Tyr Asp Ser Ala His
            260                 265                 270

Thr Leu Leu Arg Phe Arg Trp Asn Ser Phe Gln Thr Ala Gln Val Tyr
        275                 280                 285

Leu Gly Cys Ala Asp Ile Ser Ile Val Gly Ser Gly Ser Gly Pro Ser
    290                 295                 300

Ser Thr Thr Ser Met Thr Ser Ile Ser Lys Thr Ser Thr Thr Thr Ser
305                 310                 315                 320

Thr Ala Thr Thr Thr Ser Cys Ala Thr Thr Ala Thr Thr Val Leu Val
                325                 330                 335

Thr Phe Glu Gly Leu Val Thr Thr Ala Tyr Gly Gln Asn Val Phe Leu
            340                 345                 350

Thr Gly Ser Ile Ser Gln Leu Gly Ser Trp Ser Thr Ser Ser Ala Ile
        355                 360                 365

Ala Leu Ser Ala Asp Leu Tyr Thr Ser Ser Asn Pro Leu Trp Thr Ala
    370                 375                 380

Ser Ile Asp Leu Pro Ala Gly Thr Thr Phe Glu Tyr Lys Phe Phe Lys
385                 390                 395                 400

Lys Gly Ser Asp Gly Ser Ile Thr Trp Glu Ser Asp Pro Asn Arg Ser
                405                 410                 415

Tyr Thr Val Pro Thr Gly Cys Ser Gly Thr Thr Gly Thr Ala Ser Gly
            420                 425                 430

Thr Trp Arg
        435

<210>  14
<211>  385
<212>  PRT
<213>  Aspergillus clavatus

<400>  14

Met Lys Asn Gln Ile Ala Leu Val Ala Ala Asn Leu Val Ala Ala Val
1               5                   10                  15

Tyr Gly His Gly Tyr Met Thr Ile Pro Phe Ser Arg Thr Arg Leu Gly
            20                  25                  30

Phe Glu Ala Gly Ile Asp Thr Cys Pro Glu Cys Ser Ile Leu Glu Pro
        35                  40                  45

Val Thr Ala Trp Pro Asp Val Glu Ala Ala Lys Val Gly Arg Ser Gly
    50                  55                  60

Pro Cys Gly Tyr Asn Ala Arg Val Ser Val Asp Tyr Asn Thr Pro Gly
65                  70                  75                  80

Glu His Trp Gly Lys Ser Thr Val Ala Thr Tyr Ser Pro Gly Glu Ile
                85                  90                  95

Val Glu Val Gln Trp Cys Val Asp Asn Asn Gly Asp His Gly Gly Leu
            100                 105                 110

Phe Thr Tyr Gly Ile Cys Gln Asp Gln Ala Leu Val Asn Lys Phe Leu
        115                 120                 125

Thr Pro Gly Tyr Met Pro Thr Asp Ala Glu Lys Gln Ala Ala Glu Asp
    130                 135                 140

Cys Phe Leu Ala Gly Glu Leu Lys Cys Thr Asp Val Pro Gly Gln Glu
145                 150                 155                 160

Cys Gly Phe Asn Pro Asp Cys Thr Gly Gly Ala Cys His Arg Asn Asp
                165                 170                 175

Trp Phe Thr Cys Asn Ala Phe Gln Ala Asp His Arg Arg Ser Cys Gln
            180                 185                 190

Gly Val Asp Gly Ala Pro Leu Asn Ser Cys Lys Thr Thr Ile Ala Gly
        195                 200                 205

Gly Tyr Thr Val Thr Lys Lys Ile Lys Ile Pro Asp Tyr Ala Ser Glu
    210                 215                 220

His Thr Leu Leu Arg Phe Arg Trp Asn Ser Phe Gln Thr Ala Gln Val
225                 230                 235                 240

Tyr Leu Gly Cys Ala Asp Ile Ala Ile Lys Gly Ser Gly Ser Gly Gly
                245                 250                 255

Ser Ser Ser Thr Lys Thr Gln Thr Ala Thr Lys Thr Thr Thr Thr Thr
            260                 265                 270

Gly Ser Thr Glu Thr Cys Lys Ala Thr Gly Val Val Pro Val Thr Phe
        275                 280                 285

Asn Val Gln Ala Pro Thr Asn Tyr Gly Glu Asn Ile Phe Leu Val Gly
    290                 295                 300

Ser Ile Ser Gln Leu Gly Ser Trp Ser Thr Ser Ser Ala Val Pro Leu
305                 310                 315                 320

Ser Ala Thr Gln Tyr Thr Ser Ser Asn Pro Leu Trp Thr Val Thr Val
                325                 330                 335

Gln Leu Pro Ala Gly Ala Thr Phe Glu Tyr Lys Phe Ile Arg Lys Gln
            340                 345                 350

Lys Asp Gly Ser Val Ile Trp Glu Ser Asn Pro Asn Arg Ser Tyr Thr
        355                 360                 365

Thr Pro Asn Gly Cys Ser Ser Ser Pro Ala Thr Lys Thr Asp Arg Trp
    370                 375                 380

His
385

<210>  15
<211>  405
<212>  PRT
<213>  Neurospora crassa

<400>  15

Met Lys Phe Ser Ile Ile Ser Val Ala Leu Ala Ser Ala Ile Thr Val
1               5                   10                  15

Asp Ala His Gly Tyr Leu Thr Ile Pro Phe Ser Arg Thr Arg Leu Gly
            20                  25                  30

Ala Glu Ala Gly Leu Asp Thr Cys Pro Glu Cys Ser Ile Leu Glu Pro
        35                  40                  45

Val Thr Ala Trp Pro Asn Val Thr Glu Ala Lys Val Gly Arg Ser Gly
    50                  55                  60

Pro Cys Gly Tyr Asn Ala Arg Val Ser Ile Asp Tyr Asn Gln Pro Ala
65                  70                  75                  80

Thr Asn Trp Gly Asn Ser Pro Val Val Thr Tyr Thr Ala Gly Asp Thr
                85                  90                  95

Val Asp Val Gln Trp Cys Val Asp His Asn Gly Asp His Gly Gly Met
            100                 105                 110

Phe Ser Tyr Arg Ile Cys Gln Asp Gln Glu Leu Val Asn Lys Phe Leu
        115                 120                 125

Thr Pro Gly Tyr Leu Pro Thr Glu Ala Glu Lys Gln Ala Ala Glu Asp
    130                 135                 140

Cys Phe Glu Lys Gly Thr Leu Pro Cys Thr Asp Val Asn Gly Gln Ser
145                 150                 155                 160

Cys Asp Phe Ser Pro Asp Cys Gln Gln Gly Gln Ala Cys Trp Arg Asn
                165                 170                 175

Asp Trp Phe Ser Lys Leu Ala Cys Ile Ser Arg Lys Lys His Pro Ala
            180                 185                 190

Asn Arg Arg Val Thr Tyr Leu Ala Cys Asn Ala Phe Gln Ala Asp Ser
        195                 200                 205

Arg Arg Gly Cys Gln Gly Val Asp Asn Ala Ala Leu Gly Ser Cys Phe
    210                 215                 220

Thr Thr Ile Ala Gly Gly Tyr Thr Val Thr Lys Lys Ile Lys Ile Pro
225                 230                 235                 240

Asn Tyr Ile Ser Gly His Thr Leu Leu Ser Phe Arg Trp Asn Ser Phe
                245                 250                 255

Gln Thr Ala Gln Val Tyr Leu Ser Cys Ala Asp Ile Ala Ile Val Gly
            260                 265                 270

Asp Ser Ala Ser Thr Thr Lys Val Ser Ala Thr Ala Thr Thr Leu Val
        275                 280                 285

Thr Ser Ser Lys Thr Ala Ser Ala Ser Cys Thr Pro Ala Ala Thr Val
    290                 295                 300

Ala Val Thr Phe Asn His Leu Ala Ser Thr Ser Tyr Gly Glu Ser Ile
305                 310                 315                 320

Lys Ile Val Gly Ser Ile Ser Gln Leu Gly Ser Trp Ser Ala Ser Ser
                325                 330                 335

Gly Val Ala Leu Ser Ala Ser Gln Tyr Thr Thr Ser Asn Pro Leu Trp
            340                 345                 350

Thr Ala Thr Val Ser Leu Pro Ala Gly Thr Lys Phe Glu Tyr Lys Phe
        355                 360                 365

Val Lys Val Ser Ser Glu Gly Ser Ala Val Thr Trp Glu Ser Asp Pro

370                 375                 380

Asn Arg Ser Tyr Thr Val Pro Gln Ser Cys Ala Glu Ser Val Ala Val
385                 390                 395                 400

Glu Ser Ser Trp Lys
                405

<210>  16
<211>  388
<212>  PRT
<213>  Sordaria macrospora

<400>  16

Met Lys Phe Ser Ile Val Ser Ala Val Leu Ala Ser Ala Ala Thr Val
1               5                   10                  15

Asn Ala His Gly Tyr Leu Thr Ile Pro Ala Ser Arg Thr Arg Leu Gly
            20                  25                  30

Ala Glu Ala Gly Leu Asp Thr Cys Pro Glu Cys Ser Ile Leu Glu Pro
        35                  40                  45

Val Thr Ala Trp Pro Asn Val Thr Glu Ala Lys Val Gly Arg Ser Gly
    50                  55                  60

Pro Cys Gly Tyr Asn Ala Arg Val Ser Ile Asp Tyr Asn Gln Pro Ala
65                  70                  75                  80

Thr Asn Trp Gly Asn Ala Pro Val Val Thr Tyr Lys Ala Gly Asp Thr
                85                  90                  95

Ile Asp Val Gln Trp Cys Val Asp His Asn Gly Asp His Gly Gly Met
            100                 105                 110

Phe Ser Tyr Arg Ile Cys Gln Asp Gln Ala Leu Val Asn Lys Phe Leu
        115                 120                 125

Thr Pro Gly Tyr Leu Pro Thr Ala Ala Glu Lys Gln Ala Ala Glu Asp
    130                 135                 140

Cys Phe Glu Lys Gly Thr Leu Pro Cys Thr Asp Val Asn Gly Gln Asn
145                 150                 155                 160

Cys Asn Phe Ser Pro Asp Cys Gln Gln Gly Gln Ala Cys Trp Arg Asn
                165                 170                 175

Asp Trp Phe Thr Cys Asn Ala Phe Gln Ala Gly Asn Arg Arg Gly Cys

180                 185                 190

Gln Gly Val Asp Asn Ala Pro Leu Gly Ser Cys Phe Thr Thr Ile Ala
        195                 200                 205

Gly Gly Tyr Thr Val Thr Lys Lys Ile Lys Ile Pro Asn Tyr Val Ser
    210                 215                 220

Gly His Thr Leu Leu Ser Phe Arg Trp Asn Ser Phe Gln Thr Ala Gln
225                 230                 235                 240

Val Tyr Leu Ser Cys Ala Asp Ile Ala Ile Val Gly Ser Gly Gly Glu
                245                 250                 255

Thr Asn Pro Ser Ser Thr Lys Thr Thr Ala Thr Ala Thr Thr Leu Val
            260                 265                 270

Thr Ser Ser Lys Thr Ala Gly Ala Ser Cys Val Pro Val Ala Thr Val
        275                 280                 285

Ala Val Thr Phe Asn His Leu Val Ser Thr Ser Tyr Gly Glu Ser Ile
    290                 295                 300

Lys Leu Val Gly Ser Ile Ser Gln Leu Gly Ser Trp Ser Thr Ser Ser
305                 310                 315                 320

Gly Val Gly Leu Ser Ala Ser Gln Tyr Thr Thr Ser Asn Pro Leu Trp
                325                 330                 335

Thr Ala Thr Val Lys Leu Pro Ala Gly Thr Lys Phe Glu Tyr Lys Phe
            340                 345                 350

Val Lys Val Ser Ser Ser Gly Ala Val Thr Trp Glu Ser Asp Pro Asn
        355                 360                 365

Arg Ser Tyr Thr Val Pro Gln Ser Cys Ser Asp Ser Val Thr Ala Ala
    370                 375                 380

Ser Ser Trp Lys
385

<210>  17
<211>  391
<212>  PRT
<213>  Neurospora tetrasperma

<400>  17

Met Lys Phe Ser Ile Ile Ser Val Ala Leu Ala Ser Ala Ile Thr Val

1               5                   10                  15

Asn Ala His Gly Tyr Leu Thr Ile Pro Phe Ser Arg Thr Arg Leu Gly
            20                  25                  30

Ala Glu Ala Gly Leu Asp Thr Cys Pro Glu Cys Ser Ile Leu Glu Pro
        35                  40                  45

Val Thr Ala Trp Pro Asn Val Thr Glu Ala Lys Val Gly Arg Ser Gly
    50                  55                  60

Pro Cys Gly Tyr Asn Ala Arg Val Ser Ile Asp Tyr Asn Gln Pro Ala
65                  70                  75                  80

Ser Asn Trp Gly Asn Ser Pro Val Val Thr Tyr Thr Ala Gly Asp Thr
                85                  90                  95

Val Asp Val Gln Trp Cys Val Asp His Asn Gly Asp His Gly Gly Met
            100                 105                 110

Phe Ser Tyr Arg Ile Cys Gln Asp Gln Glu Leu Val Asn Lys Phe Leu
        115                 120                 125

Thr Pro Gly Tyr Leu Pro Thr Glu Ala Glu Lys Gln Ala Ala Glu Asp
    130                 135                 140

Cys Phe Glu Lys Gly Thr Leu Pro Cys Thr Asp Val Asn Gly Gln Ala
145                 150                 155                 160

Cys Asp Phe Ser Pro Asp Cys Gln Gln Gly Gln Ala Cys Trp Arg Asn
                165                 170                 175

Asp Trp Phe Thr Cys Asn Ala Phe Gln Ala Asp Ser Arg Arg Gly Cys
            180                 185                 190

Gln Gly Val Asp Asn Ala Ala Leu Gly Ser Cys Phe Thr Thr Ile Ala
        195                 200                 205

Gly Gly Tyr Thr Val Thr Lys Lys Ile Lys Ile Pro Asn Tyr Ile Ser
    210                 215                 220

Gly His Thr Leu Leu Ser Phe Arg Trp Asn Ser Phe Gln Thr Ala Gln
225                 230                 235                 240

Val Tyr Leu Ser Cys Ala Asp Ile Ala Ile Val Gly Gly Gly Glu Thr
                245                 250                 255

Lys Pro Ser Thr Thr Lys Thr Thr Ala Thr Ala Thr Thr Leu Val Thr
            260                 265                 270

Ser Ser Lys Thr Pro Ser Ala Ser Cys Thr Pro Ala Ala Thr Val Ala
        275                 280                 285

Val Thr Phe Asn His Leu Ala Ser Thr Ser Tyr Gly Glu Ser Ile Lys
    290                 295                 300

Leu Val Gly Ser Ile Ser Gln Leu Gly Ser Trp Ser Ala Ser Ser Gly
305                 310                 315                 320

Val Ala Leu Ser Ala Ser Gln Tyr Thr Thr Ser Asn Pro Leu Trp Thr
                325                 330                 335

Ala Thr Val Ser Leu Pro Ala Gly Thr Lys Phe Glu Tyr Lys Phe Val
            340                 345                 350

Lys Val Ser Ser Ser Asn Gly Gly Gly Ser Ala Val Thr Trp Glu Ser
        355                 360                 365

Asp Pro Asn Arg Ser Tyr Thr Val Pro Gln Ser Cys Val Glu Ser Val
    370                 375                 380

Thr Val Asp Ser Ser Trp Lys
385                 390

<210> 18
<211> 383
<212> PRT
<213> Colletotrichum higginsianum

<220>
<221> misc_feature
<222> (97)..(97)
<223> Xaa can be any naturally occurring amino acid

<220>
<221> misc_feature
<222> (251)..(251)
<223> Xaa can be any naturally occurring amino acid

<220>
<221> misc_feature
<222> (277)..(277)
<223> Xaa can be any naturally occurring amino acid

<400> 18

Met Arg Ser Thr Ala Ala Leu Ala Val Ala Gly Leu Val Gly Ser Val
1               5                   10                  15

Tyr Gly His Gly Tyr Leu Thr Ile Pro Ser Ser Arg Thr Arg Leu Gly
            20                  25                  30

Ala Glu Ala Gly Leu Asp Ser Cys Pro Glu Cys Thr Ile Arg Glu Pro
        35                  40                  45

Val Ala Ala Trp Pro Asp Val Thr Ala Ala Thr Val Gly Arg Ser Gly
    50                  55                  60

Pro Cys Gly Tyr Asn Ala Arg Val Ser Val Asp Tyr Asn Gln Pro Ser
65                  70                  75                  80

Ser Lys Trp Gly Asn Ser Pro Val Val Thr Tyr Thr Arg Gly Gln Thr
                85                  90                  95

Xaa Asp Val Gln Trp Cys Val Asp Asn Asn Gly Asp His Gly Gly Met
            100                 105                 110

Phe Ser Tyr Arg Ile Cys Gln Asp Gln Ala Leu Val Asp Lys Phe Ile
        115                 120                 125

Thr Pro Gly Tyr Leu Pro Thr Asp Val Glu Lys Gln Ala Ala Glu Asp
    130                 135                 140

Cys Phe Gln Arg Gly Thr Leu Lys Cys Thr Asp Val Thr Gly Gln Ser
145                 150                 155                 160

Cys Gly Tyr Ser Pro Asp Cys Ser Ala Gly Glu Ala Cys Trp Arg Asn
                165                 170                 175

Asp Trp Phe Thr Cys Asn Ala Phe Gln Ala Ser Ser Arg Arg Ala Cys
            180                 185                 190

Gln Gly Val Asp Asn Ala Pro Leu Gly Ser Cys Tyr Thr Ser Ile Ala
        195                 200                 205

Gly Gly Tyr Pro Val Thr Lys Lys Ile Arg Ile Pro Asp Tyr Val Ser
    210                 215                 220

Asn His Thr Leu Leu Ser Phe Arg Trp Asn Ser Phe Gln Thr Gly Gln
225                 230                 235                 240

Val Tyr Leu Thr Cys Ala Asp Ile Ala Ile Xaa Gly Gly Gly Ser Gly
                245                 250                 255

Ser Asn Pro Pro Pro Thr Ser Thr Arg Thr Ser Ser Ala Ala Ala Thr
            260                 265                 270

Ala Thr Ser Gly Xaa Ala Ile Ala Ser Asn Val Ala Val Thr Phe Asn
        275                 280                 285

Glu Ile Val Lys Thr Asn Pro Gly Gln Thr Ile Lys Val Ala Gly Ser
    290                 295                 300

Ile Ala Ala Leu Gly Asn Trp Asp Val Ser Ser Ala Pro Ala Leu Ser
305                 310                 315                 320

Ala Ala Gln Tyr Thr Ser Ser Asn Asn Leu Trp Thr Tyr Thr Val Ser
                325                 330                 335

Leu Pro Ala Gly Thr Thr Phe Gln Tyr Lys Phe Ile Asn Val Ala Ser
            340                 345                 350

Gly Gly Ala Ala Thr Trp Glu Ser Asp Pro Asn Arg Ser Tyr Thr Val
        355                 360                 365

Pro Arg Gly Cys Ser Thr Ala Val Thr Val Ser Ser Asn Trp Arg
    370                 375                 380

<210>  19
<211>  465
<212>  PRT
<213>  Neurospora crassa

<400>  19

Met Ser Ser Pro Gln Pro Glu Ile Gln Val Leu Thr Thr Gln Leu Ser
1               5                   10                  15

Arg Tyr Phe Thr Ser Cys Pro Thr Thr Ser Tyr Ile Gln Ala Ala Tyr
            20                  25                  30

Thr Trp Leu Gly Val Ile Trp Val Ala Thr Glu Ser Glu Asn Val Gly
        35                  40                  45

Phe Trp Gly Ile Gly Asp Asp Lys Ser Gly Val Ser Glu Ala Arg Ile
    50                  55                  60

Arg Ala Val Cys Asn Ala Met Lys Ala Gly Cys Tyr Pro Glu Gly Arg
65                  70                  75                  80

Pro Arg Arg Gly Arg Gln Ala Pro Val Arg Leu Thr Glu Thr Thr Lys
                85                  90                  95

Thr Gln Arg Phe Ala Trp Leu His Pro Arg Asn Leu Gln Arg Leu Ala
            100                 105                 110

Pro Trp Ala Gly Leu Asp Thr Cys Pro Glu Cys Ser Ile Leu Glu Pro
        115                 120                 125

Val Thr Ala Trp Pro Asn Val Thr Glu Ala Lys Val Gly Arg Ser Gly
    130                 135                 140

Pro Cys Gly Tyr Asn Ala Arg Val Ser Ile Asp Tyr Asn Gln Pro Ala
145                 150                 155                 160

Thr Asn Trp Gly Asn Ser Pro Val Val Thr Tyr Thr Ala Gly Asp Thr
                165                 170                 175

Val Asp Val Gln Trp Cys Val Asp His Asn Gly Asp His Gly Gly Met
            180                 185                 190

Phe Ser Tyr Arg Ile Cys Gln Asp Gln Glu Leu Val Asn Lys Phe Leu
        195                 200                 205

Thr Pro Gly Tyr Leu Pro Thr Glu Ala Glu Lys Gln Ala Ala Glu Asp
    210                 215                 220

Cys Phe Glu Lys Gly Thr Leu Pro Cys Thr Asp Val Asn Gly Gln Ser
225                 230                 235                 240

Cys Asp Phe Ser Pro Asp Cys Gln Gln Gly Gln Ala Cys Trp Arg Asn
                245                 250                 255

Asp Trp Phe Thr Cys Asn Ala Phe Gln Ala Asp Ser Arg Arg Gly Cys
            260                 265                 270

Gln Gly Val Asp Asn Ala Ala Leu Gly Ser Cys Phe Thr Thr Ile Ala
        275                 280                 285

Gly Gly Tyr Thr Val Thr Lys Lys Ile Lys Ile Pro Asn Tyr Ile Ser
    290                 295                 300

Gly His Thr Leu Leu Ser Phe Arg Trp Asn Ser Phe Gln Thr Ala Gln
305                 310                 315                 320

Val Tyr Leu Ser Cys Ala Asp Ile Ala Ile Val Gly Asp Ser Ala Ser
                325                 330                 335

Thr Thr Lys Val Ser Ala Thr Ala Thr Thr Leu Val Thr Ser Ser Lys
            340                 345                 350

Thr Ala Ser Ala Ser Cys Thr Pro Ala Ala Thr Val Ala Val Thr Phe
        355                 360                 365

Asn His Leu Ala Ser Thr Ser Tyr Gly Glu Ser Ile Lys Ile Val Gly
    370                 375                 380

Ser Ile Ser Gln Leu Gly Ser Trp Ser Ala Ser Ser Gly Val Ala Leu
385                 390                 395                 400

Ser Ala Ser Gln Tyr Thr Thr Ser Asn Pro Leu Trp Thr Ala Thr Val
                405                 410                 415

Ser Leu Pro Ala Gly Thr Lys Phe Glu Tyr Lys Phe Val Lys Val Ser
            420                 425                 430

Ser Glu Gly Ser Ala Val Thr Trp Glu Ser Asp Pro Asn Arg Ser Tyr
        435                 440                 445

Thr Val Pro Gln Ser Cys Ala Glu Ser Val Ala Val Glu Ser Ser Trp
    450                 455                 460

Lys
465

<210> 20
<211> 379
<212> PRT
<213> Colletotrichum gloeosporioides

<400> 20

Met Lys Phe Ser Thr Thr Leu Ala Val Ser Ser Leu Leu Gly Ser Val
1               5                   10                  15

Tyr Gly His Gly Tyr Leu Thr Ile Pro Ser Ser Arg Thr Arg Leu Gly
            20                  25                  30

His Glu Ala Gly Ile Asp Thr Cys Pro Glu Cys Thr Ile Arg Glu Pro
        35                  40                  45

Val Ser Ala Trp Pro Asp Leu Thr Ala Ala Thr Val Gly Arg Cys Gly
    50                  55                  60

Tyr Asn Ala Arg Val Ser Val Asp Tyr Asn Gln Pro Ser Thr Asn Trp
65                  70                  75                  80

Gly Asn Ser Pro Val Ala Thr Tyr Thr Arg Gly Gln Val Val Asp Val
                85                  90                  95

Gln Trp Cys Val Asp Asn Asn Gly Asp His Gly Gly Met Phe Ser Tyr
            100                 105                 110

Arg Ile Cys Gln Asp Gln Ala Leu Val Asp Lys Phe Leu Thr Pro Gly
        115                 120                 125

Tyr Ile Pro Thr Asp Asp Glu Lys Gln Ala Ala Glu Tyr Cys Phe Gln
    130                 135                 140

Ala Gly Thr Leu Pro Cys Thr Asp Val Ser Gly Gln Thr Cys Asn Tyr
145                 150                 155                 160

Ser Pro Asp Cys Thr Ser Gly Gln Ala Cys Trp Arg Asn Asp Trp Phe
                165                 170                 175

Thr Cys Asn Ala Phe Gln Ala Asp Ser Arg Arg Ala Cys Gln Gly Val
            180                 185                 190

Asp Asn Ser Ala Leu Gly Ser Cys Tyr Thr Ser Ile Ala Gly Gly Tyr
        195                 200                 205

Thr Val Thr Lys Lys Ile Lys Ile Pro Asp Tyr Val Ser Asn His Thr
    210                 215                 220

Leu Leu Ser Leu Arg Trp Asn Ser Phe Gln Thr Gly Gln Val Tyr Leu
225                 230                 235                 240

Thr Cys Ala Asp Ile Ala Ile Ser Gly Ser Gly Gly Ser Asn Pro Val
                245                 250                 255

Thr Ser Thr Arg Thr Thr Ser Ala Ala Ala Thr Ala Thr Ser Gly Ser
            260                 265                 270

Cys Thr Ala Ala Ser Ser Val Ala Val Thr Phe Asn Glu Leu Val Thr
        275                 280                 285

Thr Val Pro Gly Gln Thr Ile Lys Val Ala Gly Ser Ile Ala Ala Leu
    290                 295                 300

Gly Asn Trp Asp Ala Ala Ser Ala Pro Ala Leu Ser Ala Ser Gln Tyr
305                 310                 315                 320

Thr Ser Ser Asn Pro Leu Trp Thr Tyr Thr Val Thr Leu Pro Ala Gly
                325                 330                 335

Thr Thr Phe Gln Tyr Lys Phe Ile Asn Val Ala Ser Ser Glu Thr Val
            340                 345                 350

Thr Trp Glu Ser Asp Pro Asn Arg Ser Tyr Thr Val Pro Thr Gly Cys

355                 360                 365

Ala Thr Ala Val Thr Val Ser Ser Asn Trp Lys
    370                 375

<210>  21
<211>  400
<212>  PRT
<213>  Verticillium albo-atrum

<400>  21

Met Lys Thr Thr Thr Ala Leu Thr Leu Leu Ser Leu Ala Ser Ser Val
1               5                   10                  15

Leu Gly His Gly Tyr Leu Thr Ile Pro Lys Ser Arg Thr Arg Leu Gly
            20                  25                  30

Ala Glu Phe Ser Arg Val Asp Thr Asn Ser Phe Leu Val Gln Ala Gly
        35                  40                  45

Leu Asp Ser Cys Pro Glu Cys Asn Ile Arg Glu Pro Val Glu Ala Trp
    50                  55                  60

Pro Asn Val Thr Gln Ala Thr Val Gly Arg Ser Gly Pro Cys Gly Tyr
65                  70                  75                  80

Asn Ala Arg Val Ser Ile Asp Tyr Asn Gln Pro Lys Thr Asn Trp Gly
                85                  90                  95

Asn Ser Pro Val Val Thr Tyr Thr Ala Gly Gln Thr Val Asp Val Glu
            100                 105                 110

Trp Cys Val Asp Ala Asn Gly Asp His Gly Gly Met Phe Ser Tyr Arg
        115                 120                 125

Ile Cys Gln Asp Gln Ala Leu Val Asn Lys Phe Ile Thr Pro Gly Tyr
    130                 135                 140

Leu Pro Thr Glu Ala Glu Lys Glu Ala Ala Glu Gln Cys Phe Gln Arg
145                 150                 155                 160

Gly Thr Leu Pro Cys Thr Asp Val Thr Gly Gln Ser Cys Gly Phe Asn
                165                 170                 175

Pro Asp Cys Gln Pro Gly Gln Pro Cys Trp Arg Asn Asp Trp Phe Thr
            180                 185                 190

Cys Asn Ala Phe Gln Ala Ser Asn Arg Arg Ala Cys Gln Gly Val Asp

195                 200                 205

Gly Ala Ala Leu Gly Ser Cys Tyr Thr Ser Ile Ala Gly Gly Tyr Lys
    210                 215                 220

Val Ser Lys Lys Ile Lys Ile Pro Asn Tyr Asn Ser Ala His Thr Leu
225                 230                 235                 240

Leu Ser Phe Arg Trp Asn Ser Phe Gln Thr Gly Gln Val Tyr Leu Thr
                245                 250                 255

Cys Ala Asp Ile Ala Ile Asn Gly Gly Ser Asn Asn Asn Pro Thr Thr
            260                 265                 270

Ala Val Ala Pro Thr Thr Leu Arg Thr Thr Thr Thr Ala Ala Ala Ala
        275                 280                 285

Thr Thr Thr Gly Ala Cys Thr Pro Ala Ala Asn Val Ala Val Thr Phe
    290                 295                 300

Asn Thr Leu Ala Thr Thr Val Val Gly Gln Thr Ile Lys Leu Ala Gly
305                 310                 315                 320

Ser Ile Ser Gln Leu Gly Ser Trp Thr Pro Ala Ser Ala Pro Ala Leu
                325                 330                 335

Ser Ala Ser Gly Tyr Thr Thr Ser Arg Pro Leu Trp Thr Tyr Thr Val
            340                 345                 350

Thr Leu Pro Ala Gly Thr Thr Phe Gln Tyr Lys Phe Ile Asn Val Gln
        355                 360                 365

Ser Asp Gly Ser Val Arg Trp Glu Ser Asp Pro Asn Arg Ser Tyr Thr
    370                 375                 380

Val Pro Thr Gly Cys Ala Ser Ala Val Thr Val Asp Ser Thr Trp Arg
385                 390                 395                 400

<210>  22
<211>  390
<212>  PRT
<213>  Arthrobotrys oligospora

<400>  22

Met Lys Val Ser His Tyr Phe Asn Thr Leu Ala Leu Ala Thr Leu Val
1               5                   10                  15

Ser Gly His Gly Tyr Leu Thr Thr Pro Lys Ser Arg Thr Arg Leu Gly

20                  25                  30

Ser Glu Ala Gly Ile Asp Thr Cys Pro Glu Cys Ser Ile Leu Glu Pro
        35                  40                  45

Val Thr Ala Trp Pro Asp Leu Tyr Ala Ala Lys Val Gly Arg Ser Gly
    50                  55                  60

Pro Cys Gly Tyr Asn Ala Arg Val Ser Val Asp Tyr Asn Lys Pro Gly
65                  70                  75                  80

Ser Ala Trp Gly Asn Ala Pro Val Ala Thr Tyr Thr Ala Gly Asp Val
                85                  90                  95

Ile Thr Val Gln Trp Cys Val Asp Asn Asn Gly Asp His Gly Gly Met
            100                 105                 110

Phe Thr Tyr Arg Ile Cys Gln Asp Gln Ala Leu Val Asp Lys Phe Leu
        115                 120                 125

Asp Pro Asn Tyr Thr Pro Thr Ile Ala Glu Lys Glu Ala Ala Glu Ala
    130                 135                 140

Cys Phe Glu Gln Gly Ile Leu Ser Cys Lys Asp Val Ser Gly Gln Ser
145                 150                 155                 160

Cys Asp Tyr Ser Pro Asp Cys Gln Gln Gly Gln Ala Cys Tyr Arg Asn
                165                 170                 175

Asp Trp Phe Thr Cys Lys Gly Trp Glu Ser Gly Arg Cys Gln Ala Val
            180                 185                 190

Asp Gly Ala Gly Leu Asn Ser Cys Tyr Thr Ser Ile Ala Gly Gly Tyr
        195                 200                 205

Thr Val Thr Lys Lys Ile Lys Ile Pro Asn Tyr Val Ser Ser His Thr
    210                 215                 220

Leu Leu Ser Phe Lys Trp Asn Ser Phe Gln Thr Ala Gln Val Tyr Leu
225                 230                 235                 240

His Cys Ala Asp Ile Ala Ile Leu Pro Ser Gly Gly Thr Thr Thr Ser
                245                 250                 255

Arg Ala Ser Thr Thr Phe Ser Thr Ser Ser Thr Thr Ser Leu Thr Ala
            260                 265                 270

Cys Ser Gly Ala Glu Thr Val Thr Lys Thr Val Thr Ala Ala Ala Cys
        275                 280                 285

Pro Ser Ser Val Ala Val Thr Phe Lys Glu Leu Val Thr Thr Val Thr
    290                 295                 300

Gly Gln Asp Ile Tyr Val Val Gly Ser Ile Ser Gln Leu Gly Ser Trp
305                 310                 315                 320

Asn Ala Ala Asn Ala Ile Leu Leu Ser Ala Ser Gln Tyr Thr Ser Ser
                325                 330                 335

Asn Pro Leu Trp Phe Gly Thr Val Asn Ile Pro Ala Gly Thr Ser Phe
            340                 345                 350

Thr Tyr Lys Tyr Ile Arg Lys Ser Gly Ser Thr Val Thr Trp Glu Ser
        355                 360                 365

Asp Pro Asn Arg Ser Tyr Ser Val Pro Ser Cys Ala Ser Thr Ala Thr
    370                 375                 380

Val Ser Asp Thr Trp Arg
385                 390

<210>  23
<211>  395
<212>  PRT
<213>  Bipolaris maydis

<400>  23

Met Leu Ser Lys Ile Leu Leu Pro Val Val Ala Leu Ala Ala Ser Ala
1               5                   10                  15

Asn Ala His Gly Tyr Leu Thr Ser Pro Met Ser Arg Thr Gly Leu Asn
            20                  25                  30

Ala Gln Ser Gly Ala Asp Thr Cys Pro Glu Cys Thr Ile Leu Glu Pro
        35                  40                  45

Val Thr Ala Trp Pro Asp Leu Asp Ala Ala Ala Val Gly Arg Ser Gly
    50                  55                  60

Pro Cys Gly Tyr Asn Ala Arg Val Ser Val Asp Tyr Asn Gln Pro Gly
65                  70                  75                  80

Pro Arg Trp Gly Ser Glu Pro Val Ile Thr Tyr Lys Ala Gly Asp Val
                85                  90                  95

Val Asp Val Gln Trp Cys Val Asp Ala Asn Gly Asp His Gly Gly Met
            100                 105                 110

Phe Thr Tyr Arg Ile Cys Gln Asn Gln Ala Leu Val Asp Lys Leu Leu
        115                 120                 125

Thr Pro Gly Tyr Leu Pro Thr Glu Ala Glu Lys Gln Ala Ala Glu Asp
    130                 135                 140

Cys Phe Gln Ala Gly Glu Leu Lys Cys Thr Asp Val Pro Gly Gln Thr
145                 150                 155                 160

Cys Gly Phe Asn Ser Asp Cys Gln Gln Gly Gln Ala Cys Trp Arg Asn
                165                 170                 175

Asp Trp Phe Thr Cys Gly Gly Phe Asn Asp Asn Leu Lys Cys Arg Ser
            180                 185                 190

Val Asp Asn Ala Pro Leu Asn Ser Cys Tyr Thr Ser Ile Ala Gly Gly
        195                 200                 205

Tyr Thr Val Ser Ser Lys Ile Lys Ile Pro Asn Tyr Thr Ser Asn His
    210                 215                 220

Thr Leu Leu Ser Phe Lys Trp Asn Ser Phe Gln Thr Pro Gln Val Tyr
225                 230                 235                 240

Leu Thr Cys Ala Asp Ile Lys Ile Thr Gly Ser Ser Ser Gly Thr Ser
                245                 250                 255

Pro Pro Pro Thr Ser Ser Lys Pro Pro Thr Ser Ser Ser Lys Pro Thr
            260                 265                 270

Ser Thr Ser Thr Ser Thr Ser Ala Pro Ser Ala Thr Pro Thr Ala Cys
        275                 280                 285

Ala Thr Pro Val Ser Thr Val Ala Val Thr Phe Asn Ser Lys Thr Thr
    290                 295                 300

Thr Ser Phe Gly Gln Thr Val Lys Leu Ala Gly Ser Ile Ser Gln Leu
305                 310                 315                 320

Gly Ser Trp Asn Thr Ala Asn Ala Pro Ala Leu Ser Ala Ala Gln Tyr
                325                 330                 335

Thr Ser Ser Asn Pro Leu Trp Thr Thr Thr Leu Asn Leu Pro Ala Gly
            340                 345                 350

Thr Ser Phe Glu Tyr Lys Tyr Ile Lys Val Asp Ser Ser Gly Ala Val
        355                 360                 365

Thr Tyr Glu Ser Gly Ala Asn Arg Gln Tyr Thr Val Pro Asn Gly Cys
    370                 375                 380

Ala Asn Thr Val Thr Val Glu Gly Thr Trp Lys
385                 390                 395

<210>  24
<211>  393
<212>  PRT
<213>  Pyrenophora teres

<400>  24

Met Leu Ser Lys Leu Ile Pro Val Leu Ala Leu Ala Thr Ser Ala Tyr
1               5                   10                  15

Ala His Gly Tyr Leu Ser Ser Pro Met Ser Arg Thr Gly Leu Asn Ala
            20                  25                  30

Gln Ser Gly Ala Asp Thr Cys Pro Glu Cys Thr Ile Leu Glu Pro Val
        35                  40                  45

Thr Ala Trp Pro Asp Leu Asp Val Ala Ala Val Gly Arg Ser Gly Pro
    50                  55                  60

Cys Gly Tyr Asn Ala Arg Val Ser Val Asp Tyr Asn Gln Pro Gly Pro
65                  70                  75                  80

Arg Trp Gly Ser Gln Pro Val Ile Thr Tyr Lys Thr Gly Asp Val Val
                85                  90                  95

Asp Val Gln Trp Cys Leu Asp Ala Asn Gly Asp His Gly Gly Met Phe
            100                 105                 110

Ser Tyr Arg Ile Cys Gln Asn Gln Ala Ile Val Asp Lys Leu Leu Thr
        115                 120                 125

Pro Gly Tyr Leu Pro Thr Glu Ala Glu Lys Gln Ala Ala Glu Lys Cys
    130                 135                 140

Phe Glu Ala Gly Glu Leu Lys Cys Thr Asp Val Pro Gly Gln Thr Cys
145                 150                 155                 160

Gly Tyr Asn Pro Asp Cys Gln Ala Gly Gln Ala Cys Tyr Arg Asn Asp
                165                 170                 175

Trp Phe Thr Cys Gly Gly Phe Asn Asp Gly Thr Arg Cys Arg Gly Val
            180                 185                 190

Asp Asn Ala Pro Leu Asn Ser Cys Tyr Thr Ser Ile Ala Gly Gly Tyr
        195                 200                 205

Thr Val Ser Ser Lys Ile Lys Ile Pro Asn Tyr Thr Ser Asn His Thr
    210                 215                 220

Leu Leu Ser Phe Lys Trp Asn Ser Phe Gln Thr Pro Gln Ile Tyr Leu
225                 230                 235                 240

Thr Cys Ala Asp Ile Gln Ile Thr Gly Thr Gly Ser Gly Gly Ser Thr
                245                 250                 255

Pro Pro Thr Ser Ser Lys Ala Pro Thr Ser Ser Ser Glu Pro Thr Ser
            260                 265                 270

Thr Gln Thr Thr Ser Thr Ser Thr Pro Val Ala Thr Gly Cys Ala Thr
        275                 280                 285

Pro Val Ala Thr Val Ala Val Thr Phe Asn Ser Lys Thr Thr Thr Ser
    290                 295                 300

Val Gly Gln Thr Val Lys Ile Ala Gly Ser Ile Ala Gln Leu Gly Ser
305                 310                 315                 320

Trp Asn Thr Ala Ser Ala Pro Ala Leu Ser Ala Ser Gln Tyr Thr Ser
                325                 330                 335

Ser Asn Pro Leu Trp Thr Thr Thr Ile Lys Leu Pro Ala Gly Thr Ser
            340                 345                 350

Phe Asp Tyr Lys Phe Ile Arg Val Glu Ser Ser Gly Ser Val Thr Tyr
        355                 360                 365

Glu Ser Gly Ala Asn Arg Ala Tyr Thr Val Pro Lys Gly Cys Ala Ser
    370                 375                 380

Val Val Thr Val Asp Ser Ile Trp Lys
385                 390

<210> 25
<211> 395
<212> PRT
<213> Bipolaris sorokiniana

<400> 25

Met Leu Ser Lys Ile Leu Leu Pro Val Leu Ala Leu Ala Ala Ser Ala
1               5                   10                  15

Asn Ala His Gly Tyr Leu Thr Ser Pro Met Ser Arg Thr Gly Leu Asn
            20                  25                  30

Ala Gln Ser Gly Ala Asp Thr Cys Pro Glu Cys Thr Ile Leu Glu Pro
        35                  40                  45

Val Thr Ala Trp Pro Asp Leu Asp Ala Ala Ala Val Gly Arg Ser Gly
    50                  55                  60

Pro Cys Gly Tyr Asn Ala Arg Val Ser Val Asp Tyr Asn Gln Pro Gly
65                  70                  75                  80

Pro Arg Trp Gly Ser Glu Pro Val Ile Thr Tyr Lys Ala Gly Asp Val
                85                  90                  95

Val Asp Val Gln Trp Cys Val Asp Ala Asn Gly Asp His Gly Gly Met
            100                 105                 110

Phe Thr Tyr Arg Ile Cys Gln Asn Gln Ala Leu Val Asp Lys Leu Leu
        115                 120                 125

Thr Pro Gly Tyr Leu Pro Thr Glu Ala Glu Lys Gln Ala Ala Glu Asp
    130                 135                 140

Cys Phe Glu Ala Gly Glu Leu Lys Cys Thr Asp Val Pro Gly Gln Thr
145                 150                 155                 160

Cys Gly Phe Asn Ser Asp Cys Gln Gln Gly Gln Ala Cys Trp Arg Asn
                165                 170                 175

Asp Trp Phe Thr Cys Gly Gly Phe Asn Asp Asn Thr Lys Cys Arg Ser
            180                 185                 190

Val Asp Asn Ala Pro Leu Asn Ser Cys Tyr Thr Ser Ile Ala Gly Gly
        195                 200                 205

Tyr Thr Val Ser Ser Lys Ile Lys Ile Pro Asn Tyr Thr Ser Asn His
    210                 215                 220

Thr Leu Leu Ser Phe Lys Trp Asn Ser Phe Gln Thr Pro Gln Val Tyr
225                 230                 235                 240

Leu Thr Cys Ala Asp Ile Lys Ile Thr Gly Ser Gly Ser Gly Thr Ser
                245                 250                 255

Gln Pro Pro Thr Ser Ser Lys Pro Pro Thr Ser Ser Ser Lys Pro Thr
            260                 265                 270

Ser Thr Ser Thr Ser Thr Ser Ala Pro Ala Ala Thr Pro Thr Ala Cys
        275                 280                 285

Ala Thr Pro Val Ser Thr Val Ala Val Thr Phe Asn Ser Lys Thr Thr
    290                 295                 300

Thr Ser Phe Gly Gln Thr Val Lys Leu Ala Gly Ser Ile Ser Gln Leu
305                 310                 315                 320

Gly Ala Trp Asn Thr Ala Asn Ala Pro Ala Leu Ser Ala Ala Gln Tyr
                325                 330                 335

Thr Ser Ser Asn Pro Leu Trp Thr Thr Thr Ile Asn Leu Pro Ala Gly
            340                 345                 350

Thr Ser Phe Glu Tyr Lys Tyr Ile Lys Val Glu Ser Ser Gly Thr Val
        355                 360                 365

Thr Tyr Glu Ser Gly Ala Asn Arg Gln Tyr Thr Val Pro Lys Gly Cys
    370                 375                 380

Ala Arg Ala Val Thr Val Glu Gly Ala Trp Lys
385                 390                 395

<210> 26
<211> 388
<212> PRT
<213> Verticillium dahliae

<400> 26

Met Lys Thr Thr Thr Ala Ile Thr Leu Leu Ser Phe Ala Ser Ser Val
1               5                   10                  15

Leu Gly His Gly Tyr Leu Thr Ile Pro Lys Ser Arg Thr Arg Leu Gly
            20                  25                  30

Ala Glu Ala Gly Leu Asp Ser Cys Pro Glu Cys Asn Ile Arg Glu Pro
        35                  40                  45

Val Glu Ala Trp Pro Asn Val Thr Gln Ala Thr Val Gly Arg Ser Gly
    50                  55                  60

Pro Cys Gly Tyr Asn Ala Arg Val Ser Ile Asp Tyr Asn Gln Pro Lys
65                  70                  75                  80

Thr Asn Trp Gly Asn Ser Pro Val Val Thr Tyr Thr Ala Gly Gln Thr
                85                  90                  95

Val Asp Val Glu Trp Cys Val Asp Ala Asn Gly Asp His Gly Gly Met
            100                 105                 110

Phe Ser Tyr Arg Ile Cys Gln Asp Gln Ala Leu Val Asn Lys Phe Ile
        115                 120                 125

Thr Pro Gly Tyr Leu Pro Thr Glu Ala Glu Lys Glu Ala Ala Glu Gln
    130                 135                 140

Cys Phe Gln Arg Gly Thr Leu Pro Cys Thr Asp Val Thr Gly Gln Ser
145                 150                 155                 160

Cys Gly Phe Asn Pro Asp Cys Gln Pro Gly Gln Pro Cys Trp Arg Asn
                165                 170                 175

Asp Trp Phe Thr Cys Asn Ala Phe Gln Ala Ser Asn Arg Arg Ala Cys
            180                 185                 190

Gln Gly Val Asp Gly Ala Ala Leu Gly Ser Cys Tyr Thr Ser Ile Ala
        195                 200                 205

Gly Gly Tyr Lys Val Ser Lys Lys Ile Lys Ile Pro Asn Tyr Asn Ser
    210                 215                 220

Ala His Thr Leu Leu Ser Phe Arg Trp Asn Ser Phe Gln Thr Gly Gln
225                 230                 235                 240

Val Tyr Leu Thr Cys Ala Asp Ile Ala Ile Asn Gly Gly Ser Asn Asn
                245                 250                 255

Asn Pro Thr Thr Ala Val Ala Pro Thr Thr Leu Arg Thr Thr Thr Thr
            260                 265                 270

Ala Ala Ala Ala Thr Thr Thr Gly Ala Cys Thr Pro Ala Ala Asn Val
        275                 280                 285

Ala Val Thr Phe Asn Thr Leu Ala Thr Thr Val Val Gly Gln Thr Ile
    290                 295                 300

Lys Leu Ala Gly Ser Ile Ser Gln Leu Gly Ser Trp Thr Pro Ala Ser
305                 310                 315                 320

Ala Pro Ser Leu Ser Ala Ser Gly Tyr Thr Thr Ser Arg Pro Leu Trp

                325                 330                 335

Thr Tyr Thr Val Thr Leu Pro Ala Gly Thr Thr Phe Gln Tyr Lys Phe
            340                 345                 350

Ile Asn Val Gln Ser Asp Gly Ser Val Ala Gly Arg Ala Ile Arg Thr
        355                 360                 365

Ala Arg Thr Pro Phe Pro Thr Gly Cys Ala Ser Ala Val Thr Val Asp
    370                 375                 380

Ser Thr Trp Arg
385

<210> 27
<211> 371
<212> PRT
<213> Nectria haematococca

<400> 27

Met Lys Tyr Ser Gln Leu Leu Ala Ile Gly Phe Ala Ala Ser Val Glu
1               5                   10                  15

Ala His Gly Tyr Leu Thr Ile Pro Lys Ser Arg Thr Arg Leu Gly Ala
            20                  25                  30

Glu Ala Gly Ile Asp Ser Cys Pro Glu Cys Thr Ile Arg Glu Pro Val
        35                  40                  45

Ser Ala Trp Pro Asp Val Asp Ala Ala Thr Val Gly Arg Ser Gly Pro
    50                  55                  60

Cys Gly Phe Asn Ala Arg Val Asn Val Asp Tyr Asn Val Pro Ser Ser
65                  70                  75                  80

Asn Trp Gly Asn Thr Pro Val Ala Thr Tyr Lys Pro Gly Asp Val Val
                85                  90                  95

Asp Val Gln Trp Cys Val Asp Asn Asn Gly Asp His Gly Gly Met Phe
            100                 105                 110

Ser Tyr Arg Ile Cys Gln Asp Gln Ala Ile Val Asp Lys Leu Leu Thr
        115                 120                 125

Pro Gly Tyr Thr Pro Thr Asp Glu Glu Lys Gln Ala Ala Glu Asp Cys
    130                 135                 140

Phe Gln Asp Gly Leu Leu Pro Cys Thr Asp Val Ser Gly Gln Thr Cys

145                 150                 155                 160

Gly Phe Asn Pro Asp Cys Ser Ser Gly Gln Pro Cys Trp Arg Asn Asp
                165                 170                 175

Trp Phe Thr Cys Asn Ala Phe Gly Ala Ser Ser Arg Arg Gly Cys Gln
            180                 185                 190

Gly Val Asp Asn Ala Pro Leu Gly Ser Cys Tyr Thr Ser Ile Ala Gly
        195                 200                 205

Gly Tyr Pro Val Ser Lys Lys Ile Lys Ile Pro Asn Tyr Val Ser Asn
    210                 215                 220

His Thr Leu Leu Ser Phe Arg Trp Asn Ser Phe Gln Thr Gly Gln Val
225                 230                 235                 240

Tyr Leu Thr Cys Ala Asp Ile Ala Ile Thr Gly Ser Gly Thr Gly Thr
                245                 250                 255

Pro Pro Pro Ser Thr Thr Thr Ser Pro Gly Thr Gly Cys Ser Thr Val
            260                 265                 270

Pro Val Thr Phe Asp His Val Val Thr Thr Ser Val Gly Gln Thr Val
        275                 280                 285

Lys Val Val Gly Ser Ile Pro Ala Leu Gly Asn Trp Ser Pro Ser Ser
    290                 295                 300

Ala Pro Ala Leu Ser Ala Ser Gly Tyr Thr Ser Ser Asn His Leu Trp
305                 310                 315                 320

Thr Thr Thr Leu Asn Leu Pro Ala Gly Gln Ser Phe Gln Tyr Lys Phe
                325                 330                 335

Ala Ile Val Glu Ser Ser Gly Gly Val Lys Trp Glu Ser Asp Pro Asn
            340                 345                 350

Arg Ala Tyr Ala Val Pro Ser Ser Cys Gln Ala Val Thr Val Ser Gly
        355                 360                 365

Thr Trp Arg
    370

<210>  28
<211>  369
<212>  PRT
<213>  Pyrenophora tritici-repentis

<400>  28

Met Ser Arg Thr Gly Leu Asn Ala Gln Ser Gly Ala Asp Thr Cys Pro
1               5                   10                  15

Glu Cys Thr Ile Leu Glu Pro Val Thr Ala Trp Pro Asp Leu Asp Ala
            20                  25                  30

Ala Ala Val Gly Arg Ser Gly Pro Cys Gly Tyr Asn Ala Arg Val Ser
        35                  40                  45

Val Asp Tyr Asn Gln Pro Gly Pro Arg Trp Gly Ser Gln Pro Val Ile
    50                  55                  60

Thr Tyr Lys Ala Gly Asp Val Val Asp Val Gln Trp Cys Leu Asp Ala
65                  70                  75                  80

Asn Gly Asp His Gly Gly Met Phe Ser Tyr Arg Ile Cys Gln Asn Gln
                85                  90                  95

Ala Ile Val Asp Lys Phe Leu Thr Pro Gly Tyr Leu Pro Thr Glu Ala
            100                 105                 110

Glu Lys Gln Ala Ala Glu Lys Cys Phe Glu Ala Gly Glu Leu Lys Cys
        115                 120                 125

Thr Asp Val Pro Gly Gln Thr Cys Gly Tyr Asn Pro Asp Cys Gln Val
    130                 135                 140

Gly Gln Ala Cys Tyr Arg Asn Asp Trp Phe Thr Cys Gly Gly Phe Asn
145                 150                 155                 160

Asp Gly Ser Arg Cys Arg Gly Val Asp Asn Ala Pro Leu Asn Ser Cys
                165                 170                 175

Tyr Thr Ser Ile Ala Gly Gly Tyr Thr Val Ser Ser Lys Ile Lys Ile
            180                 185                 190

Pro Asn Tyr Thr Ser Asn His Thr Leu Leu Ser Phe Lys Trp Asn Ser
        195                 200                 205

Phe Gln Thr Pro Gln Val Tyr Leu Thr Cys Ala Asp Ile Gln Ile Thr
    210                 215                 220

Gly Thr Gly Ser Gly Gly Ser Thr Pro Pro Thr Ser Ser Lys Ala Pro
225                 230                 235                 240

Thr Ser Ser Ser Lys Pro Thr Thr Thr Ala Thr Thr Ser Thr Ser Thr
                245                 250                 255

Ala Val Ala Ser Gly Cys Ala Thr Pro Phe Ala Thr Val Ala Val Thr
            260                 265                 270

Phe Asn Ser Lys Ala Thr Thr Ser Phe Gly Gln Thr Val Lys Ile Ala
        275                 280                 285

Gly Ser Ile Ser Gln Leu Gly Ser Trp Asn Thr Ala Ser Ala Pro Ala
    290                 295                 300

Leu Phe Ala Ser Gln Tyr Thr Ser Ser Asn Pro Leu Trp Thr Thr Thr
305                 310                 315                 320

Ile Lys Leu Pro Ala Gly Thr Ser Leu Glu Tyr Lys Phe Ile Lys Val
                325                 330                 335

Glu Ser Ser Gly Ser Val Thr Tyr Glu Ser Gly Ala Asn Arg Ala Tyr
            340                 345                 350

Thr Val Pro Lys Gly Cys Ala Ser Ala Val Ser Val Asp Ser Thr Trp
        355                 360                 365

Lys

<210>  29
<211>  399
<212>  PRT
<213>  Glomerella graminicola

<400>  29

Met Lys Ser Thr Thr Asn Leu Ala Ile Ala Ser Leu Phe Ile Gly Phe
1               5                   10                  15

Val Tyr Gly His Gly Tyr Leu Ala Val Pro Ser Ser Arg Thr Arg Leu
            20                  25                  30

Gly Ala Glu Ala Gly Ser Pro Arg Cys Pro Trp Pro Ser Tyr Leu Thr
        35                  40                  45

Tyr Lys Lys Lys Val Gly Ile Asp Ser Cys Pro Glu Cys Thr Ile Arg
    50                  55                  60

Glu Pro Val Ser Ala Trp Pro Asp Val Thr Ala Ala Thr Val Gly Arg
65                  70                  75                  80

Ser Gly Pro Cys Gly Tyr Asn Ala Arg Val Gly Val Asp Tyr Asn Pro
                85                  90                  95

Pro Ser Asn Asn Trp Gly Ser Thr Pro Ile Ala Thr Tyr Thr Ser Gly
            100                 105                 110

Gln Thr Val Asp Val Gln Trp Cys Val Asp Asn Asn Gly Asp His Gly
        115                 120                 125

Gly Met Phe Ser Tyr Arg Ile Cys Gln Asp Gln Ala Leu Val Asp Gln
    130                 135                 140

Phe Ile Thr Pro Asn Tyr Leu Pro Thr Glu Ala Glu Lys Leu Ala Ala
145                 150                 155                 160

Glu Asp Cys Phe Gln Arg Gly Thr Leu Lys Cys Thr Asp Val Ser Gly
                165                 170                 175

Gln Ser Cys Gly Tyr Asn Pro Asp Cys Ser Ser Glu Glu Ala Cys Trp
            180                 185                 190

Arg Asn Asp Trp Phe Thr Cys Asn Ala Phe Gln Ala Ser Ser Arg Arg
        195                 200                 205

Ala Cys Gln Gly Val Asp Asn Ala Ser Leu Gly Ser Cys Tyr Thr Ser
    210                 215                 220

Ile Ala Gly Gly Tyr Thr Val Thr Lys Lys Ile Lys Ile Pro Asn Tyr
225                 230                 235                 240

Leu Ser Asn His Thr Leu Leu Ser Phe Arg Trp Asn Ser Phe Gln Thr
                245                 250                 255

Gly Gln Val Tyr Leu Thr Cys Ala Asp Ile Ala Ile Gly Gly Gly Gly
            260                 265                 270

Ser Ser Pro Pro Val Ser Thr Arg Thr Ser Thr Ala Ala Ala Thr Ala
        275                 280                 285

Thr Ser Gly Thr Cys Thr Ile Ala Ser Asn Val Ala Val Thr Phe Asp
    290                 295                 300

Glu Ile Val Lys Thr Asn Pro Gly Gln Thr Ile Lys Val Ala Gly Ser
305                 310                 315                 320

Ile Thr Ala Leu Gly Asn Trp Asn Val Ala Ser Ala Pro Ala Leu Ser
                325                 330                 335

Ser Ala Gln Tyr Thr Ser Thr Asn Asn Leu Trp Thr Tyr Thr Val Ser
        340                 345                 350

Leu Pro Ala Gly Thr Ala Phe Gln Tyr Lys Phe Ile Asn Leu Ala Ser
        355                 360                 365

Asp Gly Thr Val Thr Trp Glu Ser Asp Pro Asn Arg Ser Tyr Thr Val
    370                 375                 380

Pro Arg Ser Cys Leu Thr Ala Val Thr Ile Gly Ser Ser Trp Lys
385                 390                 395

<210>  30
<211>  404
<212>  PRT
<213>  Leptosphaeria maculans

<400>  30

Met Arg Ala Ser Leu Ala Lys Val Leu Ala Tyr Ala Thr Thr Val Ser
1               5                   10                  15

Ala His Gly Tyr Leu Ser Ser Pro Met Ser Arg Thr Gly Leu Asn Ala
            20                  25                  30

Gln Asp Leu Asp Arg Asn Leu Ser Asn Ser Lys Cys Ser Gln Ala Gly
        35                  40                  45

Ala Asp Thr Cys Pro Glu Cys Thr Ile Leu Glu Pro Val Pro Ala Trp
    50                  55                  60

Pro Asp Leu Asp Ala Ala Ala Val Gly Arg Ser Gly Pro Cys Gly Phe
65                  70                  75                  80

Asn Ala Arg Val Ser Val Asp Tyr Asn Gln Pro Gly Pro Arg Trp Gly
                85                  90                  95

Ser Gln Pro Val Ile Thr Tyr Lys Gly Gly Glu Thr Val Asp Val Gln
            100                 105                 110

Trp Cys Val Asp Asn Asn Gly Asp His Gly Gly Met Phe Ser Tyr Arg
        115                 120                 125

Ile Cys Gln Asp Gln Ser Ile Val Asp Lys Leu Ile Thr Pro Gly Tyr
    130                 135                 140

Leu Pro Thr Glu Ala Glu Lys Ala Ala Ala Glu Asp Cys Phe Asn Lys
145                 150                 155                 160

Gly Leu Leu Lys Cys Thr Asp Val Ala Gly Gln Ser Cys Gly Phe Asn
                165                 170                 175

Pro Asp Cys Arg Gln Gly Gln Pro Cys Trp Arg Asn Asp Trp Phe Thr
            180                 185                 190

Cys Lys Gly Phe Gln Asp Gly Pro Arg Cys Lys Gly Val Asp Asn Ala
        195                 200                 205

Pro Ile Asn Ser Cys Tyr Thr Ser Ile Ala Gly Gly Tyr Thr Val Ser
    210                 215                 220

Ser Lys Ile Lys Ile Pro Asn Tyr Val Ser Asn His Thr Leu Leu Ser
225                 230                 235                 240

Leu Lys Trp Asn Ala Phe Glu Thr Pro Gln Ile Tyr Ser Thr Cys Ala
                245                 250                 255

Asp Ile Lys Ile Thr Gly Pro Gly Ser Gly Thr Ala Pro Asn Pro Pro
            260                 265                 270

Pro Ala Ser Ser Arg Pro Thr Ser Ala Val Ala Thr Ala Thr Thr Ala
        275                 280                 285

Thr Ser Thr Ala Ala Ala Thr Gly Cys Ala Thr Pro Val Ala Ser Val
    290                 295                 300

Ala Val Thr Phe Asn Ser Arg Thr Thr Thr Ser Val Gly Gln Asn Val
305                 310                 315                 320

Lys Ile Ala Gly Ser Ile Ser Gln Leu Gly Ser Trp Asn Thr Gly Asn
                325                 330                 335

Ala Phe Ala Leu Ser Ala Ser Gln Tyr Thr Ser Ala Asn Pro Leu Trp
            340                 345                 350

Thr Gly Thr Val Asn Leu Pro Ala Gly Thr Ser Phe Glu Tyr Lys Val
        355                 360                 365

Ile Arg Val Glu Ser Gly Gly Ala Val Thr Tyr Gln Ser Gly Ala Asn
    370                 375                 380

Arg Gln Tyr Thr Val Pro Arg Gly Cys Ala Gly Thr Ala Thr Val Gln
385                 390                 395                 400

Ala Ala Trp Thr

<210>  31
<211>  426
<212>  PRT
<213>  Podospora anserina

<400>  31

Met Met Leu Gln Asn Thr Thr Ser Gln Pro His Arg Leu Arg Arg Ala
1               5                   10                  15

His Ser Val Thr Thr Ser Gln Asn Glu Ala Val Ile Asp Leu Phe Arg
            20                  25                  30

Pro His Gly Gly His Ser Gly Ser Arg Pro Trp Leu Pro Asp His Pro
        35                  40                  45

Val Gln Ser Asp Thr Ser Trp Leu Arg Gly Lys Ser Cys Lys Leu Arg
    50                  55                  60

Val Leu Trp Asn Leu Ser Thr Asp Thr Trp Ala Gln Gln Ala Gly Leu
65                  70                  75                  80

Asp Ser Cys Pro Glu Cys Ser Ile Leu Glu Pro Val Ser Ala Trp Pro
                85                  90                  95

Asp Leu Asp Val Ala Pro Val Gly Arg Ser Gly Pro Cys Gly Tyr Asn
            100                 105                 110

Ala Arg Val Ser Ile Asp Tyr Asn Gln Pro Arg Ala Gly Leu Trp Gly
        115                 120                 125

Asn Ser Pro Val Ala Arg Tyr Ser Pro Gly Gln Thr Ile Asp Val Gln
    130                 135                 140

Trp Cys Val Asp Asn Asn Gly Asp His Gly Gly Met Phe Ala Tyr Arg
145                 150                 155                 160

Ile Cys Gln Asp His Ala Leu Val Lys Lys Phe Leu Thr Pro Gly Tyr
                165                 170                 175

Leu Pro Thr Asp Glu Glu Lys Gln Ala Ala Glu Asp Cys Phe Glu Arg
            180                 185                 190

Gly Thr Leu Pro Cys Thr Asp Val Ser Gly Gln Asn Cys Gly Phe Ser
        195                 200                 205

Pro Asp Cys Ser Pro Gly Gln Pro Cys Trp Arg Asn Asp Trp Phe Thr
    210                 215                 220

Cys Asn Ala Phe Asn Ala Gly Asp Arg Arg Ala Cys Gln Gly Val Asp
225                 230                 235                 240

Asp Ala Pro Arg Gly Ser Cys Tyr Thr Ser Ile Ala Gly Gly Phe Pro
                245                 250                 255

Val Thr Lys Lys Ile Lys Leu Pro Asn Ile Asn Val Gly His Thr Leu
            260                 265                 270

Leu Ser Phe Lys Trp Asn Ser Phe Gln Thr Gly Gln Ile Tyr Leu Ser
        275                 280                 285

Cys Ala Asp Ile Ala Ile Gly Glu Gly Ser Gly Thr Val Asp Pro Pro
    290                 295                 300

Ala Ser Thr Thr Phe Ser Thr Val Val Thr Pro Gly Ala Ser Cys Ala
305                 310                 315                 320

Ala Ala Pro Ser Val Pro Val Val Phe Asn Glu Lys Ala Thr Thr Ala
                325                 330                 335

Tyr Gly Gln Asn Ile Lys Val Val Gly Ser Ile Ala Ala Leu Gly Ser
            340                 345                 350

Trp Asn Pro Ala Asn Ala Val Pro Leu Ser Ala Ala Gly Tyr Thr Asn
        355                 360                 365

Ser Asn Pro Leu Trp Ser Thr Thr Leu Asn Leu Ala Pro Gly Thr Ser
    370                 375                 380

Phe Thr Tyr Lys Phe Ile Arg Val Asp Ser Asn Gly Ala Val Thr Trp
385                 390                 395                 400

Glu Ser Asp Pro Asn Arg Ser Tyr Thr Val Pro Ala Ala Cys Gln Gly
                405                 410                 415

Gln Ser Val Ala Val Asp Ser Thr Trp Arg
            420                 425

<210>  32
<211>  401
<212>  PRT
<213>  Botryotinia fuckeliana

<400>  32

Met Ser Arg Ser Ser Thr Ile Leu Phe Val Leu Ala Ala Ala Asn Leu
1               5                   10                  15

Ala Ser Leu Val Asp Ala His Gly Tyr Met Asn Ile Pro Ser Ser Arg
            20                  25                  30

Thr Ser Ile Gly His Ser Val Gly Ile Asp Thr Cys Pro Glu Cys Thr
        35                  40                  45

Ile Leu Glu Pro Val Ser Ser Trp Pro Asp Leu Asp Val Ala Pro Val
    50                  55                  60

Gly Arg Ser Gly Pro Cys Gly Tyr Asn Ala Arg Val Ser Val Asp Tyr
65                  70                  75                  80

Asn Tyr Pro Ser Thr Asn Trp Gly Asn Thr Pro Val Thr Thr Tyr Thr
                85                  90                  95

Ala Gly Gln Thr Val Asp Val Gln Trp Cys Val Asp Asn Asn Gly Asp
            100                 105                 110

His Gly Gly Met Phe Ser Trp Arg Ile Cys Gln Asp Gln Ser Ile Val
        115                 120                 125

Asp Lys Leu Ile Thr Pro Gly Tyr Ile Pro Thr Asp Ala Glu Lys Gln
    130                 135                 140

Ala Ala Glu Asp Cys Phe Asp Ala Gly Leu Leu Pro Cys Thr Asp Val
145                 150                 155                 160

Thr Gly Ala Thr Cys Asp Tyr Asn Ala Asp Cys Thr Pro Gly Gln Ala
                165                 170                 175

Cys Tyr Arg Asn Asp Trp Phe Thr Cys Lys Gly Phe Asn Ser Gly Thr
            180                 185                 190

Arg Cys Gln Gly Val Asp Gly Ala Ala Leu Gly Ser Cys Tyr Thr Ser
        195                 200                 205

Ile Ala Gly Gly Tyr Thr Val Thr Lys Lys Val Lys Ile Pro Asn Tyr
    210                 215                 220

Ala Ser Asn His Thr Leu Ile Ser Leu Lys Trp Asn Ser Phe Gln Thr
225                 230                 235                 240

Gly Gln Ile Tyr Leu Thr Cys Ala Asp Ile Ala Ile Val Gly Ser Gly
                245                 250                 255

Ser Gly Gly Gly Ser Ser Ser Thr Thr Ser Lys Thr Ser Ser Thr Ser

260                 265                 270

Thr Thr Leu Ser Thr Ser Thr Lys Ser Ser Thr Thr Thr Ser Ala Thr
        275                 280                 285

Thr Ala Thr Thr Ser Cys Ala Ser Thr Pro Ser Leu Ile Pro Val Thr
    290                 295                 300

Phe Asn Glu Leu Val Thr Thr Thr Tyr Gly Gln Thr Ile Lys Val Ala
305                 310                 315                 320

Gly Ser Val Ala Ala Leu Gly Asn Trp Asp Val Ser Ala Ala Val Ala
                325                 330                 335

Leu Ser Ala Val Asn Tyr Thr Ser Ala Asn Pro Leu Trp Thr Gly Thr
            340                 345                 350

Val Met Leu Pro Pro Gly Gln Val Val Gln Tyr Lys Tyr Val Asn Val
        355                 360                 365

Ala Ser Ser Gly Thr Pro Thr Trp Glu Lys Asp Pro Asn Arg Val Phe
    370                 375                 380

Thr Val Pro Ala Gly Cys Ala Thr Lys Gly Thr Val Gly Asp Thr Trp
385                 390                 395                 400

Arg

<210>  33
<211>  328
<212>  PRT
<213>  Emericella nidulans

<400>  33

Met Lys Ala Phe Phe Ala Ile Ser Ala Ser Thr Leu Leu Ala Thr Val
1               5                   10                  15

His Gly His Gly Tyr Leu Thr Val Pro Ala Ser Arg Thr Arg Leu Gly
            20                  25                  30

Phe Glu Ala Gly Ile Asp Thr Cys Pro Glu Cys Ser Ile Leu Glu Pro
        35                  40                  45

Val Asp Ala Trp Pro Asn Val Thr Glu Ala Gln Val Gly Arg Ser Gly
    50                  55                  60

Pro Cys Gly Tyr Asn Ala Arg Val Ser Val Asp Tyr Asn Gln Pro Gly

65                  70                  75                  80

Asp Asn Trp Gly Asn Glu Pro Val Val Thr Tyr Lys Ala Gly Asp Ile
                85                  90                  95

Val Glu Val Gln Trp Cys Val Asp Asn Asn Gly Asp His Gly Gly Met
            100                 105                 110

Phe Thr Tyr Gly Ile Cys Gln Asp Gln Glu Leu Val Asp Lys Phe Leu
        115                 120                 125

Asp Pro Asp Tyr Leu Pro Thr Glu Glu Glu Lys Gln Ala Ala Glu Asp
    130                 135                 140

Cys Phe Leu Gln Gly Glu Leu Lys Cys Gly Asp Val Asp Gly Gln Glu
145                 150                 155                 160

Cys Glu Tyr Ser Pro Asp Cys Gly Glu Gly Glu Ala Cys Tyr Arg Asn
                165                 170                 175

Asp Trp Phe Thr Cys Asn Ala Phe Glu Ala Asp Ser Asp Arg Gly Cys
            180                 185                 190

Gln Gly Val Asp Gly Ala Glu Leu Asn Ser Cys Lys Thr Thr Ile Ala
        195                 200                 205

Gly Gly Tyr Thr Val Thr Lys Lys Ile Lys Ile Pro Asp Tyr Thr Ser
    210                 215                 220

Glu His Thr Leu Leu Arg Phe Arg Trp Asn Ser Phe Gln Thr Pro Gln
225                 230                 235                 240

Ile Tyr Leu His Cys Ala Asp Pro Thr Ile Glu Gly Gly Met Glu Val
                245                 250                 255

Arg Met Arg Met Ile Val Met His Gly Ser Phe Gly Val Asp Thr Gln
            260                 265                 270

His Ser Phe Gly His Ser Phe Gly Phe Gln Gly Glu Gly Val Tyr Arg
        275                 280                 285

Ala Tyr Arg Tyr Ile Arg Gly Val Ala Ile Ile Gln Met Asn Leu Asn
    290                 295                 300

Ile Asn Ala Ser Leu Leu Pro Gln Pro Thr Leu Pro Ile Arg Gly Trp
305                 310                 315                 320

Ser Thr Arg Asn Ile Gln His Thr
                325

<210>  34
<211>  398
<212>  PRT
<213>  Sclerotinia sclerotiorum

<400>  34

Met Thr Ile Ser Lys Ser Val Gly Val Leu Leu Ala Leu Ala Ala Asn
1               5                   10                  15

Leu Leu Pro Val Val Asp Cys His Gly Tyr Met Val Ile Pro Ser Ser
            20                  25                  30

Arg Thr Ser Leu Gly His Leu Ala Gly Ile Asp Thr Cys Pro Glu Cys
        35                  40                  45

Thr Ile Leu Glu Pro Val Ser Ser Trp Pro Asp Leu Asp Ala Ala Pro
    50                  55                  60

Val Gly Arg Ser Gly Pro Cys Gly Tyr Asn Ala Arg Val Ser Val Asp
65                  70                  75                  80

Tyr Asn Phe Pro Ala Thr Asn Trp Gly Asn Ser Pro Val Ala Thr Tyr
                85                  90                  95

Thr Pro Gly Gln Val Val Asp Val Gln Trp Cys Val Asp His Asn Gly
            100                 105                 110

Asp His Gly Gly Met Phe Ser Trp Arg Ile Cys Glu Asp Gln Ser Ile
        115                 120                 125

Ile Asp Lys Leu Ile Thr Pro Gly Tyr Thr Pro Thr Asn Ala Glu Lys
    130                 135                 140

Gln Ala Ala Glu Asp Cys Phe Glu Ala Gly Leu Leu Pro Cys Thr Asp
145                 150                 155                 160

Val Thr Gly Ala Thr Cys Ala Tyr Asn Gly Asp Cys Ser Pro Gly Gln
                165                 170                 175

Pro Cys Tyr Arg Asn Asp Trp Phe Thr Cys Asn Glu Phe Asn Ser Gly
            180                 185                 190

Thr Lys Cys Gln Gly Val Asp Asn Ser Ala Leu Gly Ser Cys Tyr Thr
        195                 200                 205

Ser Ile Ala Gly Gly Tyr Thr Val Thr Lys Lys Val Lys Ile Pro Asn
    210                 215                 220

Phe Thr Ser Asn His Thr Leu Ile Ser Leu Lys Trp Asn Ser Tyr Gln
225                 230                 235                 240

Thr Gly Gln Ile Tyr Leu Thr Cys Ala Asp Ile Ala Ile Ala Gly Ser
                245                 250                 255

Gly Thr Gly Ser Gly Gly Gly Ser Ser Ser Thr Thr Thr Lys Thr Ser
            260                 265                 270

Ser Thr Thr Ser Thr Leu Lys Thr Ser Thr Thr Thr Ala Thr Thr Ser
        275                 280                 285

Ser Cys Thr Ala Asn Pro Thr Leu Ile Pro Val Ser Phe Asn Glu Leu
    290                 295                 300

Ala Thr Thr Thr Tyr Gly Gln Thr Ile Lys Ile Ala Gly Ser Val Ala
305                 310                 315                 320

Ala Leu Gly Asn Trp Asp Val Ser Ser Ala Pro Ala Leu Ser Ala Ser
                325                 330                 335

Ala Tyr Thr Ser Thr Asn Pro Leu Trp Ser Ala Thr Ile Met Leu Ala
            340                 345                 350

Pro Gly Gln Val Val Gln Tyr Lys Phe Val Asn Val Ala Ser Ser Gly
        355                 360                 365

Thr Pro Thr Trp Glu Ser Asp Pro Asn Arg Val Tyr Thr Val Pro Gly
    370                 375                 380

Gly Cys Val Thr Thr Ala Thr Val Gly Ser Thr Trp Lys Lys
385                 390                 395

<210>  35
<211>  254
<212>  PRT
<213>  Myceliophthora thermophila

<400>  35

Met Lys Ser Thr Tyr Gly Ala Ala Ile Leu Ala Thr Ala Thr Leu Val
1               5                   10                  15

Gln Gly His Gly Tyr Leu Thr Ile Pro Ser Ser Arg Thr Arg Leu Gly
            20                  25                  30

Ala Glu Ala Gly Leu Asp Ser Cys Pro Glu Cys Ser Ile Leu Glu Pro
        35                  40                  45

Val Gln Ser Trp Pro Asp Leu Asp Ala Ala Leu Val Gly Arg Ser Gly
    50                  55                  60

Pro Cys Gly Tyr Asn Ala Arg Val Ser Ile Asp Tyr Asn Gln Pro Gly
65                  70                  75                  80

Glu His Trp Gly Asn Glu Pro Val Ala Thr Tyr Ser Pro Gly Gln Val
                85                  90                  95

Val Glu Val Gln Trp Cys Val Asp His Asn Gly Asp His Gly Gly Met
            100                 105                 110

Phe Ser Tyr Arg Ile Cys Gln Asn Gln Thr Leu Val Asp Lys Phe Leu
        115                 120                 125

Thr Pro Gly Tyr Leu Pro Thr Ala Asp Glu Lys Gln Ala Ala Glu Asp
    130                 135                 140

Cys Phe Gln Ala Gly Thr Leu Ser Cys Lys Asp Val Thr Gly Gln Asp
145                 150                 155                 160

Cys Gly Tyr Asn Pro Asp Cys Ser Glu Gly Gln Pro Cys Trp Arg Asn
                165                 170                 175

Asp Trp Phe Thr Cys Asn Ala Phe Gln Ala Asp Ser Lys Arg Ala Cys
            180                 185                 190

Gln Gly Val Asp Asn Ala Pro Gln Gly Ser Cys Tyr Thr Ser Ile Ser
        195                 200                 205

Gly Gly Phe Pro Val Thr Lys Lys Ile Lys Ile Pro Asp Tyr Gln Ser
    210                 215                 220

Ser His Thr Leu Leu Ser Phe Lys Trp Asn Ser Phe Gln Thr Gly Gln
225                 230                 235                 240

Ile Tyr Leu Ser Cys Ala Asp Ile Ala Ile Thr Ala Ser Asn
                245                 250

<210>  36
<211>  353
<212>  PRT
<213>  Magnaporthe oryzae

<400>  36

Met Lys Trp Ser Val Ile Gln Ala Leu Ala Leu Ala Ser Gly Val Gln
1               5                   10                  15

Ala His Gly Tyr Leu Thr Phe Pro Met Ser Arg Thr Gly Leu Asn Ala
            20                  25                  30

Gln Ala Gly Pro Asp Thr Cys Pro Glu Cys Thr Ile Leu Glu Pro Val
        35                  40                  45

Thr Ala Trp Pro Asp Leu Asp Ser Ala Gln Val Gly Arg Ser Gly Pro
    50                  55                  60

Cys Gly Tyr Asn Ala Arg Val Ser Val Asp Tyr Asn Gln Pro Gly Pro
65                  70                  75                  80

Arg Trp Gly Ser Ala Pro Val Val Thr Tyr Lys Gly Gly Asp Val Ala
                85                  90                  95

Asp Val Gln Trp Cys Val Asp Asn Asn Gly Asp His Gly Gly Met Phe
            100                 105                 110

Thr Tyr Arg Ile Cys Gln Asp Gln Ala Leu Val Asp Lys Leu Leu Thr
        115                 120                 125

Pro Gly Tyr Leu Pro Ser Glu Ala Glu Lys Gln Ala Ala Glu Asn Cys
    130                 135                 140

Phe Arg Ala Gly Thr Leu Pro Cys Thr Asp Val Asn Gly Gln Ser Cys
145                 150                 155                 160

Gly Tyr Ser Pro Asp Cys Ser Pro Gly Gln Ala Cys Trp Arg Asn Asp
                165                 170                 175

Trp Phe Thr Cys Lys Gly Phe Gln Asp Thr Lys Cys Arg Gly Val Asp
            180                 185                 190

Asn Ala Pro Leu Asn Ser Cys Tyr Thr Ser Ile Ala Gly Gly Tyr Thr
        195                 200                 205

Thr Pro Gln Ile Tyr Leu Thr Cys Ala Asp Ile Lys Ile Thr Ala Pro
    210                 215                 220

Asp Ser Gln Ser Pro Pro Thr Thr Thr Thr Thr Ser Thr Pro Ala Ser
225                 230                 235                 240

Pro Pro Pro Thr Ser Cys Ala Thr Pro Ala Ala Ser Val Ala Val Thr
                245                 250                 255

Phe Arg Ser Lys Thr Thr Thr Ser Val Gly Gln Thr Val Lys Ile Ala
            260                 265                 270

Gly Ser Ile Ala Gln Leu Gly Gly Trp Asp Ala Ser Lys Ala Pro Ala
        275                 280                 285

Leu Ser Ala Ser Gln Tyr Thr Ser Ser Asn Pro Leu Trp Thr Thr Thr
    290                 295                 300

Ile Ser Leu Pro Ala Gly Ala Thr Phe Glu Tyr Lys Phe Ile Arg Val
305                 310                 315                 320

Glu Ser Ser Gly Ala Val Thr Tyr Glu Ser Gly Ala Asn Arg Val Tyr
                325                 330                 335

Thr Val Pro Arg Asp Cys Ala Gly Thr Ala Thr Val Asp Thr Ala Trp
            340                 345                 350

Lys

<210>  37
<211>  261
<212>  PRT
<213>  Chaetomium globosum

<400>  37

Met Lys Gln Phe Thr Ala Leu Ser Leu Ile Ser Leu Ala Thr Ala Val
1               5                   10                  15

Lys Gly His Gly Tyr Met Val Ser Pro Ala Ser Arg Thr Arg Gln Gly
            20                  25                  30

Phe Glu Asp Gly Thr Asp Thr Cys Pro Glu Cys Thr Ile Leu Glu Pro
        35                  40                  45

Val Gln Ser Trp Pro Asp Leu Asp Ala Ala Thr Val Gly Arg Ser Gly
    50                  55                  60

Pro Cys Gly Tyr Asn Ala Arg Val Ser Val Asp Tyr Asn Gln Pro Gly
65                  70                  75                  80

Lys Ala Trp Gly Asn Glu Val Val Glu Thr Tyr Ala Ala Gly Asp Val
                85                  90                  95

Ile Thr Val Gln Trp Cys Val Asp His Asn Gly Asp His Gly Gly Met
            100                 105                 110

Phe Thr Tyr Arg Ile Cys Gln Asp Gln Ala Leu Val Asp Lys Phe Thr
        115                 120                 125

Asp Pro Ser Tyr Leu Pro Thr Glu Ala Glu Lys Gln Ala Ala Glu Asp
    130                 135                 140

Cys Phe Asn Glu Gly Val Leu Ser Cys Gly Asp Val Asp Gly Gln Asp
145                 150                 155                 160

Cys Ser Tyr Ser Pro Asp Cys Ser Glu Gly Ala Ala Cys Trp Arg Asn
                165                 170                 175

Asp Trp Phe Thr Cys Glu Lys Phe Gly Glu Gly Ser Cys Lys Gly Val
            180                 185                 190

Asp Gly Ala Ala Leu Asn Ser Cys Ala Thr Thr Ile Ala Gly Gly Tyr
        195                 200                 205

Thr Val Thr Lys Gln Val Lys Leu Pro Asp Tyr Ser Ser Lys His Thr
    210                 215                 220

Leu Leu Ser Phe Lys Trp Asn Ser Phe Gln Thr Gly Gln Ile Tyr Leu
225                 230                 235                 240

Ser Cys Ala Asp Ile Ala Ile Thr Gly Gly Ser Gly Gly Gly Ala Ser
                245                 250                 255

Ser Asn Ser Thr Arg
            260

<210>  38
<211>  248
<212>  PRT
<213>  Penicillium chrysogenum

<400>  38

Met Lys Ser Ala Ala Val Val Leu Val Gly Ser Leu Ser Ala Ile Val
1               5                   10                  15

Ala Gly His Gly Tyr Leu Val Thr Pro Asp Ser Arg Thr Lys Leu Gly
            20                  25                  30

Phe Gln Ala Gly Ile Asp Thr Cys Pro Glu Cys Thr Ile Leu Glu Pro
        35                  40                  45

Val Ser Ser Trp Pro Asp Leu Asp Val Ala Pro Val Gly Arg Ser Gly
    50                  55                  60

Pro Cys Gly Tyr Asn Ala Arg Val Ser Val Asp Tyr Asn Gln Pro Gly
65                  70                  75                  80

Ser Asp Trp Gly Lys Glu Val Val Lys Thr Tyr Lys Ala Gly Asp Val
                85                  90                  95

Ile Asp Val Val Trp Cys Val Asp His Asn Gly Asp His Gly Gly Met
            100                 105                 110

Phe Thr Tyr Arg Ile Cys Gln Asp Gln Ala Leu Val Asp Lys Phe Leu
        115                 120                 125

Asp Pro Asp Tyr Leu Pro Thr Asp Glu Glu Lys Gln Ala Ala Glu Asp
    130                 135                 140

Cys Phe Asn Lys Gly Leu Leu Asp Cys Thr Gly Val Asp Gly Gln Ala
145                 150                 155                 160

Cys Asp Tyr Asn Ala Asp Cys Gly Glu Gly Glu Ala Cys His Arg Asn
                165                 170                 175

Asp Trp Phe Thr Cys Asn Gly Phe Asp Asp Thr Lys Cys Gln Gly Val
            180                 185                 190

Asp Ser Ala Glu Leu Gly Ser Cys Lys Thr Thr Ile Ala Gly Gly Tyr
        195                 200                 205

Thr Val Ser Lys Lys Ile Lys Leu Pro Asp Tyr Val Ser Asn His Thr
    210                 215                 220

Leu Leu Ser Trp Lys Trp Asn Ser Phe Gln Thr Gly Gln Ile Tyr Leu
225                 230                 235                 240

Ser Cys Ser Asp Ile Ala Ile Gln
                245

<210>  39
<211>  251
<212>  PRT
<213>  Aspergillus oryzae

<400>  39

Met Lys Val Phe Ala Pro Leu Leu Ser Leu Ser Leu Ala Thr Ser Val
1               5                   10                  15

Ala Gly His Gly Tyr Met Tyr Ile Pro Ser Ser Arg Thr Arg Leu Gly
            20                  25                  30

His Glu Ala Gly Ile Asp Ser Cys Pro Glu Cys Ala Ile Leu Glu Pro
        35                  40                  45

Val Ser Ser Trp Pro Asp Leu Asp Ala Ala Pro Val Gly Arg Ser Gly
    50                  55                  60

Pro Cys Gly Tyr Asn Ala Arg Asp Ser Ile Asp Tyr Asn Gln Pro Thr
65                  70                  75                  80

Thr Asn Trp Gly Ser Asp Ala Val Gln Ser Tyr Ser Pro Gly Glu Glu
                85                  90                  95

Ile Glu Val Gln Trp Cys Val Asp His Asn Gly Asp His Gly Gly Met
            100                 105                 110

Phe Thr Tyr Arg Ile Cys Gln Asp Gln Ser Ile Val Asp Lys Phe Leu
        115                 120                 125

Asp Pro Ser Tyr Leu Pro Thr Asn Asp Glu Lys Gln Ala Ala Glu Asp
    130                 135                 140

Cys Phe Asp Ala Gly Leu Leu Pro Cys Thr Asp Val Ser Gly Gln Glu
145                 150                 155                 160

Cys Gly Tyr Ser Ala Asp Cys Thr Glu Gly Glu Ala Cys Trp Arg Asn
                165                 170                 175

Asp Trp Phe Thr Cys Asn Gly Phe Glu Ala Ser Asp Arg Pro Lys Cys
            180                 185                 190

Gln Gly Val Asp Asn Ala Glu Leu Asn Ser Cys Tyr Thr Ser Ile Ala
        195                 200                 205

Gly Gly Tyr Thr Val Thr Lys Lys Val Lys Leu Pro Glu Tyr Thr Ser
    210                 215                 220

Asn His Thr Leu Ile Ser Phe Lys Trp Asn Ser Phe Gln Thr Gly Gln
225                 230                 235                 240

Ile Tyr Leu Ser Cys Ala Asp Ile Ala Ile Gln
                245                 250

<210>  40
<211>  251
<212>  PRT
<213>  Aspergillus flavus

<400>  40

Met Lys Val Phe Ala Pro Leu Leu Ser Leu Gly Leu Ala Thr Ser Val
1               5                   10                  15

Ala Gly His Gly Tyr Met Tyr Ile Pro Ser Ser Arg Thr Arg Leu Gly
            20                  25                  30

His Glu Ala Gly Val Asp Ser Cys Pro Glu Cys Ala Ile Leu Glu Pro
        35                  40                  45

Val Ser Ser Trp Pro Asp Leu Asp Ala Ala Pro Val Gly Arg Ser Gly
    50                  55                  60

Pro Cys Gly Tyr Asn Ala Arg Asp Ser Ile Asp Tyr Asn Gln Pro Thr
65                  70                  75                  80

Thr Asn Trp Gly Ser Asp Ala Val Gln Ser Tyr Ser Pro Gly Glu Glu
                85                  90                  95

Ile Glu Val Gln Trp Cys Val Asp His Asn Gly Asp His Gly Gly Met
            100                 105                 110

Phe Thr Tyr Arg Ile Cys Gln Asp Gln Ser Ile Val Asp Lys Phe Leu
        115                 120                 125

Asp Pro Ser Tyr Leu Pro Thr Asn Asp Glu Lys Gln Ala Ala Glu Asp
    130                 135                 140

Cys Phe Asp Ala Gly Leu Leu Pro Cys Thr Asp Val Ser Gly Gln Glu
145                 150                 155                 160

Cys Gly Tyr Ser Ala Asp Cys Thr Glu Gly Glu Ala Cys Trp Arg Asn
                165                 170                 175

Asp Trp Phe Thr Cys Asn Gly Phe Glu Ala Ser Asp Arg Pro Lys Cys
            180                 185                 190

Gln Gly Val Asp Asn Ala Glu Leu Asn Ser Cys Tyr Thr Ser Ile Ala
        195                 200                 205

Gly Gly Tyr Thr Val Thr Lys Lys Val Lys Leu Pro Glu Tyr Thr Ser
    210                 215                 220

Asn His Thr Leu Ile Ser Phe Lys Trp Asn Ser Phe Gln Thr Gly Gln
225                 230                 235                 240

Ile Tyr Leu Ser Cys Ala Asp Ile Ala Ile Gln
                245                 250

<210>  41
<211>  279
<212>  PRT
<213>  Aspergillus oryzae

<400>  41

Met Ser Ala Pro Cys Ser Gly Met Phe Gln Val Arg Trp Arg Trp Tyr
1               5                   10                  15

Asn Lys Asp Val Asp Arg Ile Leu Ser His Val Tyr Ile Met Val Phe
            20                  25                  30

Ala Pro Leu Leu Ser Leu Ser Leu Ala Thr Ser Val Ala Gly His Gly
        35                  40                  45

Tyr Met Tyr Ile Pro Ser Ser Arg Thr Arg Leu Gly His Glu Ala Gly
    50                  55                  60

Ile Asp Ser Cys Pro Glu Cys Ala Ile Leu Glu Pro Val Ser Ser Trp
65                  70                  75                  80

Pro Asp Leu Asp Ala Ala Pro Val Gly Arg Ser Gly Pro Cys Gly Tyr
                85                  90                  95

Asn Ala Arg Asp Ser Ile Asp Tyr Asn Gln Pro Thr Thr Asn Trp Gly
            100                 105                 110

Ser Asp Ala Val Gln Ser Tyr Ser Pro Gly Glu Glu Ile Glu Val Gln
        115                 120                 125

Trp Cys Val Asp His Asn Gly Asp His Gly Gly Met Phe Thr Tyr Arg
    130                 135                 140

Ile Cys Gln Asp Gln Ser Ile Val Asp Lys Phe Leu Asp Pro Ser Tyr
145                 150                 155                 160

Leu Pro Thr Asn Asp Glu Lys Gln Ala Ala Glu Asp Cys Phe Asp Ala
                165                 170                 175

Gly Leu Leu Pro Cys Thr Asp Val Ser Gly Gln Glu Cys Gly Tyr Ser
            180                 185                 190

Ala Asp Cys Thr Glu Gly Glu Ala Cys Trp Arg Asn Asp Trp Phe Thr
        195                 200                 205

Cys Asn Gly Phe Glu Ala Ser Asp Arg Pro Lys Cys Gln Gly Val Asp
    210                 215                 220

Asn Ala Glu Leu Asn Ser Cys Tyr Thr Ser Ile Ala Gly Gly Tyr Thr
225                 230                 235                 240

Val Thr Lys Lys Val Lys Leu Pro Glu Tyr Thr Ser Asn His Thr Leu
                245                 250                 255

Ile Ser Phe Lys Trp Asn Ser Phe Gln Thr Gly Gln Ile Tyr Leu Ser
            260                 265                 270

Cys Ala Asp Ile Ala Ile Gln
        275

<210>  42
<211>  279
<212>  PRT
<213>  Aspergillus oryzae

<400>  42

Met Ser Ala Pro Cys Ser Gly Met Phe Arg Val Arg Trp Arg Trp Tyr
1               5                   10                  15

Asn Lys Asp Val Asp Arg Ile Leu Ser His Val Tyr Ile Met Val Phe
            20                  25                  30

Ala Pro Leu Leu Ser Leu Gly Leu Ala Thr Ser Val Ala Gly His Gly
        35                  40                  45

Tyr Met Tyr Ile Pro Ser Ser Arg Thr Arg Leu Gly His Glu Ala Gly
    50                  55                  60

Val Asp Ser Cys Pro Glu Cys Ala Ile Leu Glu Pro Val Ser Ser Trp
65                  70                  75                  80

Pro Asp Leu Asp Ala Ala Pro Val Gly Arg Ser Gly Pro Cys Gly Tyr
                85                  90                  95

Asn Ala Arg Asp Ser Ile Asp Tyr Asn Gln Pro Thr Thr Asn Trp Gly
            100                 105                 110

Ser Asp Ala Val Gln Ser Tyr Ser Pro Gly Glu Glu Ile Glu Val Gln
        115                 120                 125

Trp Cys Val Asp His Asn Gly Asp His Gly Gly Met Phe Thr Tyr Arg
    130                 135                 140

Ile Cys Gln Asp Gln Ser Ile Val Asp Lys Phe Leu Asp Pro Ser Tyr
145                 150                 155                 160

Leu Pro Thr Asn Asp Glu Lys Gln Ala Ala Glu Asp Cys Phe Asp Ala
                165                 170                 175

Gly Leu Leu Pro Cys Thr Asp Val Ser Gly Gln Glu Cys Gly Tyr Ser
            180                 185                 190

Ala Asp Cys Thr Glu Gly Glu Ala Cys Trp Arg Asn Asp Trp Phe Thr
        195                 200                 205

Cys Asn Gly Phe Glu Ala Ser Asp Arg Pro Lys Cys Gln Gly Val Asp
    210                 215                 220

Asn Ala Glu Leu Asn Ser Cys Tyr Thr Ser Ile Ala Gly Gly Tyr Thr
225                 230                 235                 240

Val Thr Lys Lys Val Lys Leu Pro Glu Tyr Thr Ser Asn His Thr Leu
                245                 250                 255

Ile Ser Phe Lys Trp Asn Ser Phe Gln Thr Gly Gln Ile Tyr Leu Ser
            260                 265                 270

Cys Ala Asp Ile Ala Ile Gln
        275

<210>  43
<211>  248
<212>  PRT
<213>  Aspergillus clavatus

<400>  43

Met Lys Pro Phe Ala Thr Leu Val Ala Phe Ala Leu Ala Thr Cys Val
1               5                   10                  15

Ala Gly His Gly Tyr Met Tyr Ile Pro Thr Ser Arg Thr Arg Leu Gly
            20                  25                  30

His Glu Ala Gly Val Asp Thr Cys Pro Glu Cys Ala Ile Leu Glu Pro
        35                  40                  45

Val Gln Ala Trp Pro Ser Leu Asp Ser Ala Lys Val Gly Arg Ser Gly
    50                  55                  60

Pro Cys Gly Tyr Asn Ala Arg Asp Ser Ile Asp Tyr Asn Gln Pro Thr
65                  70                  75                  80

Lys Asn Trp Gly Ser Lys Pro Val Ser Ala Phe Lys Ala Gly Gln Glu
                85                  90                  95

Ile Glu Val Gln Trp Cys Val Asp His Asn Gly Asp His Gly Gly Met
            100                 105                 110

Phe Thr Tyr Arg Ile Cys Gln Asp Gln Lys Leu Val Ser Lys Phe Leu
        115                 120                 125

Asp Pro Ser Tyr Met Pro Thr Glu Ala Glu Lys Gln Ala Ala Glu Asp
    130                 135                 140

Cys Phe Glu Lys Gly Ile Leu Pro Cys Thr Asp Val Asn Gly Gln Lys
145                 150                 155                 160

Cys Ser Tyr Ser Pro Asp Cys Lys Gln Gly Glu Pro Cys Trp Arg Asn
                165                 170                 175

Asp Trp Phe Thr Cys Asp Ala Phe Asp Gly Ala Lys Cys Lys Gly Val
            180                 185                 190

Asp Asn Ala Ala Leu Asn Ser Cys Lys Thr Thr Ile Ala Gly Gly Tyr
        195                 200                 205

Thr Val Thr Lys Lys Val Lys Leu Pro Asn Tyr Val Ser Asn His Thr
    210                 215                 220

Leu Leu Ser Trp Lys Trp Asn Ser Phe Gln Thr Pro Gln Ile Tyr Leu
225                 230                 235                 240

Ser Cys Ala Asp Ile Ser Ile Ser
                245

<210> 44
<211> 253
<212> PRT
<213> Fusarium oxysporum

<400> 44

Met Phe Thr Leu Gln Ala Leu Gln Leu Ala Ser Met Cys Thr Leu Val
1               5                   10                  15

Tyr Gly His Gly Tyr Leu Ser Lys Pro Met Ser Arg Thr Gly Leu Asn
            20                  25                  30

Ala Glu Ala Gly Pro Asp Thr Cys Pro Glu Cys Thr Ile Leu Glu Pro
        35                  40                  45

Val Thr Ala Trp Pro Asp Leu Asp Ser Ala Lys Val Gly Arg Ser Gly
    50                  55                  60

Pro Cys Gly Tyr Asn Ala Arg Val Ser Val Asp Tyr Asn Gln Pro Gly
65                  70                  75                  80

Ala Asn Trp Gly Lys Glu Pro Ile Ala Thr Tyr Lys Pro Gly Gln Val
                85                  90                  95

Val Asp Val Gln Trp Cys Val Asp Asn Asn Gly Asp His Gly Gly Met
            100                 105                 110

Tyr Ala Tyr Arg Ile Cys Gln Asp Gln Asp Ile Val Asp Lys Phe Leu
        115                 120                 125

Asp Pro Lys Tyr Ile Pro Thr Glu Ala Glu Lys Gln Ala Ala Glu Asp
    130                 135                 140

Cys Phe Glu Glu Gly Leu Leu Pro Cys Thr Asp Val Asn Gly Gln Glu
145                 150                 155                 160

Cys Gly Tyr Ser Pro Asp Cys Ser Ala Asp Gln Pro Cys His Arg Asn
                165                 170                 175

Asp Trp Phe Thr Cys Lys Ser Phe Asp Gly Asn Ser Asp Gly Lys Gly
            180                 185                 190

Cys Arg Gly Val Asp Asn Ala Pro Ile Asn Ser Cys Tyr Thr Ser Ile
        195                 200                 205

Ala Gly Gly Tyr Thr Val Ser Gly Lys Ile Lys Ile Pro Asp Tyr Val
    210                 215                 220

Ser Asn His Thr Leu Leu Ser Phe Lys Trp Asn Ala Phe Gln Thr Pro
225                 230                 235                 240

Gln Val Tyr Leu Thr Cys Ala Asp Ile Ala Ile Ser Ala
                245                 250

<210>  45
<211>  266
<212>  PRT
<213>  Neosartorya fischeri

<400>  45

Met Lys Ala Phe Thr Ile Leu Thr Ala Ser Val Leu Thr Thr Cys Val
1               5                   10                  15

Ala Gly His Gly Tyr Leu Tyr Ile Pro Asn Ser Arg Thr Arg Leu Gly
            20                  25                  30

Asn Glu Val Cys His His Ser Thr Leu Ser Leu Pro Gln Leu Thr Ser
        35                  40                  45

Gln Ala Gly Val Asp Ser Cys Pro Glu Cys Ala Ile Leu Glu Pro Val
    50                  55                  60

Ser Ser Trp Pro Asn Leu Asp Ser Ala Pro Val Ser Arg Ser Gly Pro
65                  70                  75                  80

Cys Gly Tyr Asn Ala Arg Asp Ser Ile Asp Tyr Asn Gln Pro Thr Ser
                85                  90                  95

Asn Trp Gly Thr Lys Pro Val Ala Ser Tyr Thr Ala Gly Gln Glu Ile
            100                 105                 110

Glu Val Gln Trp Cys Val Asp His Asn Gly Asp His Gly Gly Met Phe
        115                 120                 125

Ser Tyr Arg Ile Cys Gln Asp Gln Ser Ile Val Asp Lys Leu Leu Asp
    130                 135                 140

Ala Ser Tyr Leu Pro Thr Gln Ala Glu Lys Gln Ala Ala Glu Asp Cys
145                 150                 155                 160

Phe Glu Ala Gly Leu Leu Pro Cys Thr Asp Val Asn Gly Gln Glu Cys
                165                 170                 175

Gly Tyr Ser Ala Asp Cys Val Glu Gly Gln Ala Cys Trp Arg Asn Asp
            180                 185                 190

Trp Phe Thr Cys Asn Gly Phe Gln Ala Ser Glu Arg Pro Lys Cys Gln
        195                 200                 205

Gly Val Asp Asn Ala Pro Leu Asn Ser Cys Tyr Thr Ser Ile Ala Gly
    210                 215                 220

Gly Tyr Thr Val Thr Lys Lys Val Lys Ile Pro Asp Tyr Val Ser Asn
225                 230                 235                 240

His Thr Leu Leu Ser Phe Lys Trp Asn Ser Phe Gln Thr Gly Gln Ile
                245                 250                 255

Tyr Leu Ser Cys Ala Asp Ile Ser Ile Ser
            260                 265

<210>  46

<211>  248
<212>  PRT
<213>  Aspergillus terreus

<400>  46

Met Lys Gly Val Ala Ala Leu Ile Ser Leu Ser Met Val Thr Cys Val
1               5                   10                  15

Ala Gly His Gly Tyr Met Trp Ser Pro Met Ser Arg Thr Arg Ile Gly
            20                  25                  30

Ala Glu Ser Gly Asn Asp Thr Cys Pro Glu Cys Thr Ile Leu Glu Pro
        35                  40                  45

Val Thr Ala Trp Pro Thr Leu Asp Glu Ala Lys Val Gly Arg Ser Gly
    50                  55                  60

Val Cys Gly Tyr Asn Ala Arg Val Ser Thr Asp Tyr Asn Val Pro Ala
65                  70                  75                  80

Gly Asp Trp Gly Ala Asp Val Val Lys Thr Tyr Ser Pro Gly Glu Glu
                85                  90                  95

Ile Glu Val Val Trp Cys Val Asp His Asn Gly Asp His Gly Gly Met
            100                 105                 110

Phe Thr Tyr Arg Ile Cys Gln Asp Gln Thr Ile Val Asp Lys Phe Leu
        115                 120                 125

Asp Pro Ser His Thr Pro Thr Glu Glu Glu Lys Gln Ala Ala Glu Asp
    130                 135                 140

Cys Phe Gln Lys Gly Val Leu Pro Cys Thr Asp Val Asn Gly Gln Glu
145                 150                 155                 160

Cys Asn Tyr Ser Pro Asp Cys Ser Glu Gly Glu Ala Cys Trp Arg Lys
                165                 170                 175

Asp Trp Phe Thr Cys Asn Gly Phe Asp Asp Thr Lys Cys Gln Gly Val
            180                 185                 190

Asp Asn Ala Glu Leu Asn Ser Cys Tyr Thr Ser Ile Ala Gly Gly Tyr
        195                 200                 205

Thr Val Thr Lys Lys Val Lys Leu Pro Asp Tyr Thr Ser Lys His Thr
    210                 215                 220

Leu Leu Ser Phe Lys Trp Asn Ser Tyr Gln Thr Pro Gln Ile Tyr Leu

225                 230                 235                 240

Ser Cys Ala Asp Ile Ala Ile Gln
                245

<210>  47
<211>  246
<212>  PRT
<213>  Fusarium graminearum

<400>  47

Met Phe Thr Leu Gln Ala Leu Gln Leu Val Ser Met Cys Thr Leu Val
1               5                   10                  15

Tyr Gly His Gly Tyr Leu Thr Asp Lys Arg Lys Ala Gly Pro Asp Thr
            20                  25                  30

Cys Pro Glu Cys Thr Ile Leu Glu Pro Val Thr Ala Trp Pro Asp Leu
        35                  40                  45

Asp Ala Ala Lys Val Gly Arg Thr Gly Pro Cys Gly Tyr Asn Ala Arg
    50                  55                  60

Val Ser Val Asp Tyr Asn Gln Pro Gly Ala Asn Trp Gly Lys Glu Pro
65                  70                  75                  80

Ile Ala Thr Tyr Lys Pro Gly Gln Val Val Asp Val Gln Trp Cys Val
                85                  90                  95

Asp Asn Asn Gly Asp His Gly Gly Met Tyr Ala Tyr Arg Ile Cys Gln
            100                 105                 110

Asp Gln Asp Ile Val Asp Asn Phe Leu Asp Pro Asn Tyr Val Pro Thr
        115                 120                 125

Glu Ser Glu Lys Gln Ala Ala Glu Asp Cys Phe Glu Glu Gly Leu Leu
    130                 135                 140

Pro Cys Thr Glu Val Asp Gly Gln Glu Cys Gly Tyr Ser Pro Asp Cys
145                 150                 155                 160

Ser Thr Asp Gln Pro Cys His Arg Asn Asp Trp Phe Ala Cys Arg Ser
                165                 170                 175

Phe Gln Gly Asp Ser Asp Gly Lys Gly Cys Arg Gly Val Asp Asn Ala
            180                 185                 190

Pro Ile Asn Ser Cys Tyr Thr Ser Ile Ala Gly Gly Tyr Thr Val Ser

195                 200                 205

Gly Lys Ile Lys Ile Pro Asp Tyr Ile Ser Asn His Thr Leu Leu Ser
    210                 215                 220

Phe Lys Trp Asn Ser Phe Gln Thr Pro Gln Val Tyr Leu Thr Cys Ala
225                 230                 235                 240

Asp Ile Ala Ile Ser Ala
                245

<210>  48
<211>  338
<212>  PRT
<213>  Chaetomium globosum

<400>  48

Met Cys Trp Thr Arg Gly Gly Ala Gly Val Ala Glu Gly Gln Arg Thr
1               5                   10                  15

Ala Leu Cys Asp Asp Asn Thr Arg Thr Ser Arg Arg Ala Asp Phe Gly
            20                  25                  30

Gly Thr Thr Leu Glu Pro Glu Gln Arg Pro Asn Arg Met Asp Ala Gly
        35                  40                  45

Leu Asp Ser Cys Pro Glu Cys Ser Ile Leu Glu Pro Val Val Ala Trp
    50                  55                  60

Pro Asp Leu Thr Ala Ala Pro Val Gly Arg Ser Gly Pro Cys Gly Tyr
65                  70                  75                  80

Asn Ala Arg Val Ser Ile Asp Tyr Asn Gln Pro Thr Ser Asn Trp Gly
                85                  90                  95

Asn Ser Pro Val Ala Ser Tyr Ser Pro Gly Gln Val Val Asp Val Gln
            100                 105                 110

Trp Cys Val Asp His Asn Gly Asp His Gly Gly Met Phe Ser Tyr Arg
        115                 120                 125

Ile Cys Gln Asp Gln Ser Leu Val Asp Lys Phe Leu Asp Pro Asp Tyr
    130                 135                 140

Leu Pro Thr Glu Ala Glu Lys Gln Ala Ala Glu Asp Cys Phe Glu Ala
145                 150                 155                 160

Gly Gly Tyr Ile Ser Gly Gly Phe Pro Val Thr Lys Lys Ile Arg Ile

                165                 170                 175

Pro Asn Tyr Gln Ser Ser His Thr Leu Leu Ser Phe Lys Trp Asn Ser
            180                 185                 190

Tyr Gln Thr Gly Gln Ile Tyr Leu Ser Cys Ala Asp Ile Ala Ile Gly
        195                 200                 205

Gly Gly Ser Asn Ser Ala Thr Thr Leu Ser Thr Ser Thr Arg Ser Ala
    210                 215                 220

Thr Ala Thr Pro Gly Ala Ser Cys Ala Ala Ala Ala Ser Val Ala Val
225                 230                 235                 240

Ala Phe Arg Thr Arg Val Thr Thr Ser Trp Gly Gln Thr Val Lys Ile
                245                 250                 255

Val Gly Ser Ile Pro Glu Leu Gly Asn Trp Asp Pro Ser Leu Ala Pro
            260                 265                 270

Thr Leu Ser Ala Ala Glu Tyr Thr Thr Ser Asn Pro Val Trp Thr Ser
        275                 280                 285

Thr Ile Asn Leu Ala Ala Gly Thr Lys Phe Glu Tyr Lys Phe Val Lys
    290                 295                 300

Val Thr Gly Asn Glu Ala Val Thr Trp Glu Ser Asp Pro Asn Arg Ser
305                 310                 315                 320

Tyr Thr Val Ala Ser Gly Cys Asp Thr Thr Ala Val Ser Glu Ser Ser
                325                 330                 335

Trp Arg

<210> 49
<211> 279
<212> PRT
<213> Phaeosphaeria nodorum

<400> 49

Met Phe Ala Tyr Arg Ile Cys Gln Asp Gln Ala Leu Val Asp Lys Phe
1               5                   10                  15

Leu Gln Pro Gly Tyr Leu Pro Thr Glu Ala Glu Lys Gln Ala Ala Glu
            20                  25                  30

Asp Cys Phe Glu Ala Gly Thr Leu Lys Cys Thr Asp Val Ala Gly Gln

35                  40                  45

Asn Cys Gly Phe Asn Pro Asp Cys Gln Gln Gly Gln Pro Cys Trp Arg
    50                  55                  60

Asn Asp Trp Phe Thr Cys Lys Gly Phe Gln Glu Gly Ser Lys Cys Arg
65                  70                  75                  80

Gly Val Asp Asn Ala Pro Ile Asn Ser Cys Phe Thr Ser Ile Ala Gly
                85                  90                  95

Gly Tyr Thr Val Ser Ser Lys Ile Lys Ile Pro Asn Tyr Ala Ser Ser
            100                 105                 110

His Thr Leu Leu Ser Phe Lys Trp Asn Ser Phe Gln Thr Pro Gln Ile
        115                 120                 125

Tyr Leu Thr Cys Ala Asp Ile Lys Ile Ala Gly Ala Gly Thr Gly Thr
    130                 135                 140

Ser Pro Lys Pro Ser Thr Ser Ser Lys Pro Ala Thr Ser Thr Ser Ser
145                 150                 155                 160

Thr Lys Ala Val Pro Ser Ala Ser Ala Thr Gly Cys Ala Thr Pro Val
                165                 170                 175

Ser Asn Val Ala Val Thr Phe Asn Ser Lys Ser Thr Thr Ser Val Gly
            180                 185                 190

Gln Thr Ile Lys Ile Val Gly Ser Ile Ser Gln Leu Gly Ser Trp Asn
        195                 200                 205

Thr Ala Asn Ala Pro Ala Leu Ser Ala Glu Lys Tyr Thr Ser Ser Asn
    210                 215                 220

Thr Leu Trp Thr Thr Thr Ile Arg Leu Gln Ala Gly Val Ser Phe Glu
225                 230                 235                 240

Tyr Lys Phe Ile Arg Val Ala Ser Ser Gly Ala Val Ala Tyr Glu Ser
                245                 250                 255

Gly Ala Asn Arg Ala Tyr Thr Val Pro Arg Gly Cys Glu Gly Asn Gln
            260                 265                 270

Thr Val Asp Thr Gln Trp Lys
        275

<210>  50
<211>  219
<212>  PRT
<213>  Geomyces destructans

<400>  50

Met Val Ala Phe Ile Thr Leu Thr Ala Leu Ser Leu Ala Thr Ser Val
1               5                   10                  15

Tyr Gly His Gly Tyr Leu Val Val Pro Glu Ser Arg Thr Gly Leu Gly
            20                  25                  30

His Glu Ala Asn Leu Asp Ser Tyr Thr Glu Cys Thr Ile Leu Glu Pro
        35                  40                  45

Val Ser Ala Trp Pro Asp Leu Thr Thr Ala Ser Gly Lys Ser Pro Val
    50                  55                  60

Ala Thr Tyr Ala Pro Gly Asp Ile Val Asn Val Gln Trp Cys Val Asp
65                  70                  75                  80

Ala Asn Gly Asp His Gly Gly Met Phe Ser Tyr Arg Ile Cys Gln Asp
                85                  90                  95

Gln Thr Ile Val Asp Lys Phe Leu Asp Ala Ser Tyr Leu Pro Thr His
            100                 105                 110

Glu Glu Lys Gln Ala Ala Glu Val Cys Phe Gln Ala Gly Thr Leu Asp
        115                 120                 125

Cys Thr Asp Val Ser Gly Gln Asp Cys Gly Ala Gly Asp Gln Thr Asn
    130                 135                 140

Cys Lys Gly Val Asp Asn Val Ala Leu Asn Ser Cys Tyr Thr Ser Ile
145                 150                 155                 160

Ala Gly Gly Tyr Thr Val Pro Lys Lys Ile Lys Ile Pro Asp Tyr Ser
                165                 170                 175

Ser Glu His Thr Leu Leu Ser Leu Arg Trp Asn Ser Phe Gln Thr Gly
            180                 185                 190

Gln Val Tyr Leu Thr Cys Ser Asn Ile Ala Ile Ser Gly Ser Ser Pro
        195                 200                 205

Gly Asn Gly Thr Ser Asn Ser Thr Thr Gly Ala
    210                 215

<210>  51
<211>  251
<212>  PRT
<213>  Emericella nidulans

<400>  51

Met Lys Ala Phe Phe Ala Ile Ser Ala Ser Thr Leu Leu Ala Thr Val
1               5                   10                  15

His Gly His Gly Tyr Leu Thr Val Pro Ala Ser Arg Thr Arg Leu Gly
            20                  25                  30

Phe Glu Ala Gly Ile Asp Thr Cys Pro Glu Cys Ser Ile Leu Glu Pro
        35                  40                  45

Val Asp Ala Trp Pro Asn Val Thr Glu Ala Gln Val Gly Arg Ser Gly
    50                  55                  60

Pro Cys Gly Tyr Asn Ala Arg Val Ser Val Asp Tyr Asn Gln Pro Gly
65                  70                  75                  80

Asp Asn Trp Gly Asn Glu Pro Val Val Thr Tyr Lys Ala Gly Asp Ile
                85                  90                  95

Val Glu Val Gln Trp Cys Val Asp Asn Asn Gly Asp His Gly Gly Met
            100                 105                 110

Phe Thr Tyr Gly Ile Cys Gln Asp Gln Glu Leu Val Asp Lys Phe Leu
        115                 120                 125

Asp Pro Asp Tyr Leu Pro Thr Glu Glu Glu Lys Gln Ala Ala Glu Asp
    130                 135                 140

Cys Phe Leu Gln Gly Glu Leu Lys Cys Gly Asp Val Asp Gly Gln Glu
145                 150                 155                 160

Cys Glu Tyr Ser Pro Asp Cys Gly Glu Gly Glu Ala Cys Tyr Arg Asn
                165                 170                 175

Asp Trp Phe Thr Cys Asn Ala Phe Glu Ala Asp Ser Asp Arg Gly Cys
            180                 185                 190

Gln Gly Val Asp Gly Ala Glu Leu Asn Ser Cys Lys Thr Thr Ile Ala
        195                 200                 205

Gly Gly Tyr Thr Val Thr Lys Lys Ile Lys Ile Pro Asp Tyr Thr Ser
    210                 215                 220

Glu His Thr Leu Leu Arg Phe Arg Trp Asn Ser Phe Gln Thr Pro Gln
225                 230                 235                 240

Ile Tyr Leu His Cys Ala Asp Pro Thr Ile Glu
                245                 250

<210>  52
<211>  326
<212>  PRT
<213>  Ustilago maydis

<400>  52

Met Val Phe Ser Asn Ser Asn Ala Ser Val Ser Leu Phe Arg Leu Val
1               5                   10                  15

Ala Leu Val Ala Thr Leu Ser His Leu Val Phe Thr Phe Val Asp Ala
            20                  25                  30

His Gly Tyr Val Thr Phe Pro Ala Ser Arg Ala Tyr Met Cys Lys Gln
        35                  40                  45

Gly Gln Ala Lys Asn Cys Gly Glu Ile Gln Tyr Glu Pro Gln Ser Val
    50                  55                  60

Glu Ala Pro Lys Gly Leu Pro Phe Ala Arg Lys Gly Asp Gly Gln Leu
65                  70                  75                  80

Cys Ser Ala Gly Leu Gly Gln Phe Ser Gln Leu Asp Arg Gln Gly Pro
                85                  90                  95

Ser Ala Trp Pro Thr Thr Lys Ala Ser Gly Val His Ser Phe Ser Trp
            100                 105                 110

Thr Phe Thr Ala Gln His Ala Thr Thr Asp Phe Lys Tyr Phe Ile Thr
        115                 120                 125

Lys Ala Asn Trp Asp Ser Ser Lys Thr Ser Gly Leu Ser Ala Ser Asp
    130                 135                 140

Leu Glu Ser Asp Pro Phe Leu Thr Val Ser Met Asn Gly Lys Ala Pro
145                 150                 155                 160

Pro Arg Thr Met Asn His Asp Leu Ser Lys Ala Met Pro Ser Arg Ser
                165                 170                 175

Gly Tyr His Val Val Tyr Ala Val Trp Thr Val Asp Asn Thr Ala Asn
            180                 185                 190

Ala Phe Tyr Gln Cys Leu Asp Leu Asp Phe Gly Gly Gly Asn Ser Ser
        195                 200                 205

Ser Ser Ser Ser Ser Ser Asn Ser Ser Ala Thr Ser Thr Thr Gly Ser
    210                 215                 220

Ser Ser Ala Ala Ser Ala Ala Thr Ser Ser Thr Thr Ser Ser Ser Val
225                 230                 235                 240

Ser Glu Ser Gly Thr Ala Ser Thr Ala Ser Gly Ser Gly Ser Asp Asp
                245                 250                 255

Asp Ser Ser Ser Ser Gly Asp Ser Ser Asn Gly Ser Ser Ser Asp Asn
            260                 265                 270

Asn Gly Gly Ser Ser Gly Ser Thr Thr Met Pro Lys Ser Gln Ile Ala
        275                 280                 285

Gln Ser Gly Ala Cys Arg Met Lys Lys Arg Arg Arg Ser Pro Asn Ala
    290                 295                 300

Ser Val Leu Ala Ala Arg Gly Asp Tyr Arg Arg His Lys Ser Gln Met
305                 310                 315                 320

Arg Arg Asp Arg Gln Gly
                325

<210>  53
<211>  385
<212>  PRT
<213>  Emericella nidulans

<400>  53

Met Lys Ser Leu Leu Ala Leu Val Ala Gly Asn Leu Val Thr Ala Val
1               5                   10                  15

Ser Gly His Gly Tyr Leu Thr Val Pro Ala Ser Arg Thr Arg Leu Gly
            20                  25                  30

Phe Glu Ala Gly Ile Asp Thr Cys Pro Glu Cys Ser Ile Leu Glu Pro
        35                  40                  45

Val Ser Ala Trp Pro Asp Leu Thr Ala Ala Gln Val Gly Arg Ser Gly
    50                  55                  60

Pro Cys Gly Tyr Asn Ala Arg Val Ser Val Asp Tyr Asn Gln Pro Gly
65                  70                  75                  80

Asp Tyr Trp Gly Asn Glu Pro Val Val Ser Tyr Thr Ala Gly Asp Val
                85                  90                  95

Val Glu Val Gln Trp Cys Val Asp His Asn Gly Asp His Gly Gly Met
            100                 105                 110

Phe Thr Tyr Gly Ile Cys Gln Asn Gln Thr Leu Val Asp Leu Phe Leu
        115                 120                 125

Thr Pro Gly Tyr Leu Pro Thr Asn Glu Glu Lys Gln Ala Ala Glu Asp
    130                 135                 140

Cys Phe Leu Glu Gly Glu Leu Ser Cys Leu His Val Pro Gly Gln Thr
145                 150                 155                 160

Cys Asn Tyr Asn Pro Asp Cys Ser Ala Gly Glu Pro Cys Tyr Gln Asn
                165                 170                 175

Asp Trp Phe Thr Cys Asn Ala Phe Gln Ala Asp Asn Asn Arg Ala Cys
            180                 185                 190

Gln Gly Val Asp Gly Ala Ala Leu Asn Ser Cys Met Thr Thr Ile Ala
        195                 200                 205

Gly Gly Tyr Thr Val Thr Lys Lys Ile Lys Ile Pro Asp Tyr Ser Ser
    210                 215                 220

Ser His Thr Leu Leu Arg Phe Arg Trp Asn Ser Phe Gln Thr Ala Gln
225                 230                 235                 240

Val Tyr Leu His Cys Ala Asp Ile Ala Ile Val Gly Gly Ser Gly Ser
                245                 250                 255

Ser Pro Ser Pro Thr Ser Thr Thr Ser Thr Ala Thr Ser Thr Thr Thr
            260                 265                 270

Pro Ser Ser Thr Ser Cys Ala Ser Ala Ile Ser Ile Pro Val Thr Phe
        275                 280                 285

Asn Ala Leu Val Thr Thr Thr Tyr Gly Glu Asn Val Tyr Leu Ala Gly
    290                 295                 300

Ser Ile Ser Gln Leu Gly Ser Trp Ser Thr Ser Ser Ala Val Ala Leu
305                 310                 315                 320

Ser Ala Ser Lys Tyr Ser Ser Ser Ser Pro Leu Trp Thr Val Thr Val
                325                 330                 335

Asp Leu Pro Val Gly Ala Thr Phe Glu Tyr Lys Tyr Ile Lys Lys Glu
        340                 345                 350

Ser Asp Gly Ser Ile Val Trp Glu Ser Gly Pro Asn Arg Ser Tyr Thr
    355                 360                 365

Val Pro Thr Gly Cys Ser Gly Thr Thr Ala Thr Glu Ser Gly Ala Trp
    370                 375                 380

Arg
385

<210>  54
<211>  251
<212>  PRT
<213>  Aspergillus oryzae

<400>  54

Met Lys Val Phe Ala Pro Leu Leu Ser Leu Ser Leu Ala Thr Ser Val
1               5                   10                  15

Ala Gly His Gly Tyr Met Tyr Ile Pro Ser Ser Arg Thr Arg Leu Gly
            20                  25                  30

His Glu Ala Gly Ile Asp Ser Cys Pro Glu Cys Ala Ile Leu Glu Pro
        35                  40                  45

Val Ser Ser Trp Pro Asp Leu Asp Ala Ala Pro Val Gly Arg Ser Gly
    50                  55                  60

Pro Cys Gly Tyr Asn Ala Arg Asp Ser Ile Asp Tyr Asn Gln Pro Thr
65                  70                  75                  80

Thr Asn Trp Gly Ser Asp Ala Val Gln Ser Tyr Ser Pro Gly Glu Glu
                85                  90                  95

Ile Glu Val Gln Trp Cys Val Asp His Asn Gly Asp His Gly Gly Met
            100                 105                 110

Phe Thr Tyr Arg Ile Cys Gln Asp Gln Ser Ile Val Asp Lys Phe Leu
        115                 120                 125

Asp Pro Ser Tyr Leu Pro Thr Asn Asp Glu Lys Gln Ala Ala Glu Asp
    130                 135                 140

Cys Phe Asp Ala Gly Leu Leu Pro Cys Thr Asp Val Ser Gly Gln Glu
145                 150                 155                 160

Cys Gly Tyr Ser Ala Asp Cys Thr Glu Gly Glu Ala Cys Trp Arg Asn
                165                 170                 175

Asp Trp Phe Thr Cys Asn Gly Phe Glu Ala Ser Asp Arg Pro Lys Cys
            180                 185                 190

Gln Gly Val Asp Asn Ala Glu Leu Asn Ser Cys Tyr Thr Ser Ile Ala
        195                 200                 205

Gly Gly Tyr Thr Val Thr Lys Lys Val Lys Leu Pro Glu Tyr Thr Ser
    210                 215                 220

Asn His Thr Leu Ile Ser Phe Lys Trp Asn Ser Phe Gln Thr Gly Gln
225                 230                 235                 240

Ile Tyr Leu Ser Cys Ala Asp Ile Ala Ile Gln
                245                 250

<210>  55
<211>  251
<212>  PRT
<213>  Emericella nidulans

<400>  55

Met Lys Val Phe Ala Pro Leu Leu Ser Leu Ser Leu Ala Thr Ser Val
1               5                   10                  15

Ala Gly His Gly Tyr Met Tyr Ile Pro Ser Ser Arg Thr Arg Leu Gly
            20                  25                  30

His Glu Ala Gly Ile Asp Ser Cys Pro Glu Cys Ala Ile Leu Glu Pro
        35                  40                  45

Val Ser Ser Trp Pro Asp Leu Asp Ala Ala Pro Val Gly Arg Ser Gly
    50                  55                  60

Pro Cys Gly Tyr Asn Ala Arg Asp Ser Ile Asp Tyr Asn Gln Pro Thr
65                  70                  75                  80

Thr Asn Trp Gly Ser Asp Ala Val Gln Ser Tyr Ser Pro Gly Glu Glu
                85                  90                  95

Ile Glu Val Gln Trp Cys Val Asp His Asn Gly Asp His Gly Gly Met
            100                 105                 110

Phe Thr Tyr Arg Ile Cys Gln Asp Gln Ser Ile Val Asp Lys Phe Leu
        115                 120                 125

Asp Pro Ser Tyr Leu Pro Thr Asn Asp Glu Lys Gln Ala Ala Glu Asp
    130                 135                 140

Cys Phe Asp Ala Gly Leu Leu Pro Cys Thr Asp Val Ser Gly Gln Glu
145                 150                 155                 160

Cys Gly Tyr Ser Ala Asp Cys Thr Glu Gly Glu Ala Cys Trp Arg Asn
                165                 170                 175

Asp Trp Phe Thr Cys Asn Gly Phe Glu Ala Ser Asp Arg Pro Lys Cys
            180                 185                 190

Gln Gly Val Asp Asn Ala Glu Leu Asn Ser Cys Tyr Thr Ser Ile Ala
        195                 200                 205

Gly Gly Tyr Thr Val Thr Lys Lys Val Lys Leu Pro Glu Tyr Thr Ser
    210                 215                 220

Asn His Thr Leu Ile Ser Phe Lys Trp Asn Ser Phe Gln Thr Gly Gln
225                 230                 235                 240

Ile Tyr Leu Ser Cys Ala Asp Ile Ala Ile Gln
                245                 250

<210>  56
<211>  197
<212>  PRT
<213>  Artificial Sequence

<220>
<223>  polysaccharide monooxygenase CBP21

<400>  56

Met Asn Lys Thr Ser Arg Thr Leu Leu Ser Leu Gly Leu Leu Ser Ala
1               5                   10                  15

Ala Met Phe Gly Val Ser Gln Gln Ala Asn Ala His Gly Tyr Val Glu
            20                  25                  30

Ser Pro Ala Ser Arg Ala Tyr Gln Cys Lys Leu Gln Leu Asn Thr Gln
        35                  40                  45

Cys Gly Ser Val Gln Tyr Glu Pro Gln Ser Val Glu Gly Leu Lys Gly
    50                  55                  60

Phe Pro Gln Ala Gly Pro Ala Asp Gly His Ile Ala Ser Ala Asp Lys
65                  70                  75                  80

Ser Thr Phe Phe Glu Leu Asp Gln Gln Thr Pro Thr Arg Trp Asn Lys
                85                  90                  95

Leu Asn Leu Lys Thr Gly Pro Asn Ser Phe Thr Trp Lys Leu Thr Ala
            100                 105                 110

Arg His Ser Thr Thr Ser Trp Arg Tyr Phe Ile Thr Lys Pro Asn Trp
        115                 120                 125

Asp Ala Ser Gln Pro Leu Thr Arg Ala Ser Phe Asp Leu Thr Pro Phe
    130                 135                 140

Cys Gln Phe Asn Asp Gly Gly Ala Ile Pro Ala Ala Gln Val Thr His
145                 150                 155                 160

Gln Cys Asn Ile Pro Ala Asp Arg Ser Gly Ser His Val Ile Leu Ala
                165                 170                 175

Val Trp Asp Ile Ala Asp Thr Ala Asn Ala Phe Tyr Gln Ala Ile Asp
            180                 185                 190

Val Asn Leu Ser Lys
        195